# EUROPEAN PATENT SPECIFICATION

(11) **EP 1 778 282 B1**
(45) Date of publication and mention of the grant of the patent: **30.03.2016**
(21) Application number: 05774364.3
(22) Date of filing: 17.08.2005
(51) Int. Cl.: C07K 14/005, C07K 14/16, A61K 39/21, A61K 38/16

(54) **MUTATED HIV NEF FOR MODULATING IMMUNITY**
MUTIERTES HIV NEF ZUR MODULATION DER IMMUNITÄT
NEF MUTANT DU VIH POUR MODULER L'IMMUNITE

(30) Priority: 17.08.2004 EP 04292056
(43) Date of publication of application: 02.05.2007
(60) Divisional application: 10187179.6
(73) Proprietor: Institut Gustave Roussy, 94805 Villejuif Cédex (FR); Centre National de la Recherche Scientifique, 75794 Paris Cedex 16 (FR); Université de Paris-Sud XI, 91405 Orsay Cedex (FR)
(72) Inventor: RENARD, Martial, F-75012 Paris (FR); MANGENEY, Marianne, F-75014 Paris (FR); HEIDMANN, Thierry, F-75017 Paris (FR)
(74) Representative: Grosset-Fournier, Chantal Catherine
(86) International application number: PCT/EP2005/008907
(87) International publication number: WO 2006/018289

(56) References cited:
- WO-A-98/26075
- WO-A-02/069691
- US-A- 5 962 635
- US-A- 5 968 514
- US-A1- 2004 037 825
- ALI AYUB ET AL: "Broadly increased sensitivity to cytotoxic T lymphocytes resulting from Nef epitope escape mutations." JOURNAL OF IMMUNOLOGY, vol. 171, no. 8, 15 October 2003 (2003-10-15), pages 3999-4005, XP002311186 ISSN: 0022-1767
- HAHN TOBIAS ET AL: "Evaluation of genetic diversity of human immunodeficiency virus type 1 nef gene associated with vertical transmission." JOURNAL OF BIOMEDICAL SCIENCE, vol. 10, no. 4, 2003, pages 436-450, XP002348903 ISSN: 1021-7770
- PADUA ELIZABETH ET AL: "Natural variation of the nef gene in human immunodeficiency virus type 2 infections in Portugal." JOURNAL OF GENERAL VIROLOGY, vol. 84, no. 5, May 2003 (2003-05), pages 1287-1299, XP002348904 ISSN: 0022-1317
- ASAMITSU AK ET AL: "Conservation of the central proline-rich (PxxP) motifs of human immunodeficiency virus type 1 Nef protein during the disease progression in two hemophiliac patients" FEBS LETTERS, ELSEVIER, AMSTERDAM, NL, vol. 459, no. 3, 15 October 1999 (1999-10-15), pages 399-404, XP004375868 ISSN: 0014-5793
- FUJINO MASAYUKI ET AL: "Prolonged survival of rat liver allograft with adenoviral gene transfection of human immunodeficiency virus type 1 nef." LIVER TRANSPLANTATION : OFFICIAL PUBLICATION OF THE AMERICAN ASSOCIATION FOR THE STUDY OF LIVER DISEASES AND THE INTERNATIONAL LIVER TRANSPLANTATION SOCIETY. AUG 2003, vol. 9, no. 8, August 2003 (2003-08), pages 805-813, XP009041657 ISSN: 1527-6465
- LIU LANG XIA ET AL: "Mutation of a conserved residue (D123) required for oligomerization of human immunodeficiency virus type 1 Nef protein abolishes interaction with human thioesterase and results in impairment of Nef biological functions" JOURNAL OF VIROLOGY, vol. 74, no. 11, June 2000 (2000-06), pages 5310-5319, XP002348905 ISSN: 0022-538X
- COHEN GEORGE B ET AL: "The human thioesterase II protein binds to a site on HIV-1 Nef critical for CD4 down-regulation" JOURNAL OF BIOLOGICAL CHEMISTRY, vol. 275, no. 30, 28 July 2000 (2000-07-28), pages 23097-23105, XP002348906 ISSN: 0021-9258
- HANNA ZAHER ET AL: "In vivo mutational analysis of the N-terminal region of HIV-1 Nef reveals critical motifs for the development of an AIDS-like disease in CD4C/HIV transgenic mice" VIROLOGY, vol. 327, no. 2, 1 October 2004 (2004-10-01), pages 273-286, XP002311184 ISSN: 0042-6822
- YAMADA TAKESHI ET AL: "Antibody-dependent cellular cytotoxicity via humoral immune epitope of Nef protein expressed on cell surface." JOURNAL OF IMMUNOLOGY (BALTIMORE, MD. : 1950) 15 FEB 2004, vol. 172, no. 4, 15 February 2004 (2004-02-15), pages 2401-2406, XP002311185 ISSN: 0022-1767
- KANG MI RAN ET AL: "Phylogenetic analysis of the nef gene reveals a distinctive monophyletic Clade in Korean HIV-1 cases" JOURNAL OF ACQUIRED IMMUNE DEFICIENCY SYNDROMES AND HUMAN RETROVIROLOGY, vol. 17, no. 1, 1 January 1998 (1998-01-01), pages 58-68, XP009041552 ISSN: 1077-9450 -& DATABASE NCBI NIH; AAC17893 1998, KANG, MR ET AL.: "Nef protein (HIV-1)" XP002311359
- COUILLIN ISABELLE ET AL: "HLA-dependent variations in human immunodeficiency virus Nef protein alter peptide/HLA binding" EUROPEAN JOURNAL OF IMMUNOLOGY, vol. 25, no. 3, 1995, pages 728-732, XP009041575 ISSN: 0014-2980
- COUILLIN ISABELLE ET AL: "Impaired cytotoxic T lymphocytes recognition due to genetic variations in the main immunogenic region of the human immunodeficiency virus 1 NEF protein" JOURNAL OF EXPERIMENTAL MEDICINE, vol. 180, no. 3, 1994, pages 1129-1134, XP000572554 ISSN: 0022-1007
- CRAIG H M ET AL: "Analysis of the SH3-Binding Region of HIV-1 Nef: Partial Functional Defects Introduced by Mutations in the Polyproline Helix and the Hydrophobic Pocket" VIROLOGY, ACADEMIC PRESS,ORLANDO, US, vol. 262, no. 1, 15 September 1999 (1999-09-15), pages 55-63, XP004439819 ISSN: 0042-6822
- BUMPERS-HL: "Abstract 52: Antiangiogenic effects of a novel HIV-1 nef cytotoxic peptide on the growth of primary and metastatic colorectal cancer" PROCEEDINGS OF THE AACR, vol. 45, March 2004 (2004-03), XP002311241
- ABHAY JERE ET AL: 'Genetic analysis of Indian HIV-1 nef: subtyping, variability and implications' MICROBES AND INFECTION vol. 6, no. 3, 01 March 2004, pages 279 - 289, XP055013179 DOI: 10.1016/j.micinf.2003.11.012 ISSN: 1286-4579

## Description

The present invention relates to the use of the immunosuppressive function of an accessory protein of the human or simian immunodeficiency virus for the preparation of a vaccine. In particular, the present invention relates to vaccine compositions comprising a Nef protein.

Although more than 20 years of scientific research have been devoted to finding a vaccine against HIV (Human Immunodeficiency Virus), a convincing prophylactic means to fight HIV is still awaiting to be discovered. Thus, more than 20 clinical trials of anti-HIV vaccines have been launched, and as of today, none of them has shown sufficient efficacy in preventing infections.

Nef (negative regulatory factor), is a 27 to 35 kDa regulatory protein of HIV or SIV. Among its various functions, Nef is in particular involved in the down-regulation of the expression of Class I MHC (Major Histocompatibility Complex) molecules (MHC-I) of the A and B types in humans (HLA-A and HLA-B). This property of Nef has been shown to be sensitive to mutations, however, mutations of HIV-1 Nef at the amino acid position 93 have proved inefficient at modulating its MHC-I down-regulation properties (Ali et al. (2003) J. Immunol. 171:3999-4005). Nef is also involved in the down-regulation of CD4 molecules normally expressed at the surface of T helper cells. Furthermore, Nef also down-regulates the expression of mature Class II MHC molecules and up-regulates the expression of immature Class II MHC molecules. All these regulations are a consequence of Nef interference with normal cellular trafficking and in particular with the endocytosis-degradation pathway (Le Gall et al. (1998) Immunity 8:483-495).

Nef, as one of the antigens of HIV or SIV, has been included in several vaccine compositions, alone or in combination with other antigens, such as described, for example, in WO 98/26075, WO 01/00232 or in WO 03/011334. However, these approaches have not been demonstrated to be effective either. The lack of an effective immune response against HIV or SIV, as a result of Nef administration, might relate to an as yet unidentified function of Nef, whereas generation of an active vaccine against HIV or SIV most probably requires an effective immune response to be raised against Nef.

Thus, an object of the present invention is to relate immunosuppressive properties of HIV or SIV to the Nef protein.

Another object of the invention relates to the identification of an immunosuppressive domain in the Nef protein.

A further object of the invention is to provide pharmaceutical or vaccine compositions comprising a modified Nef protein.

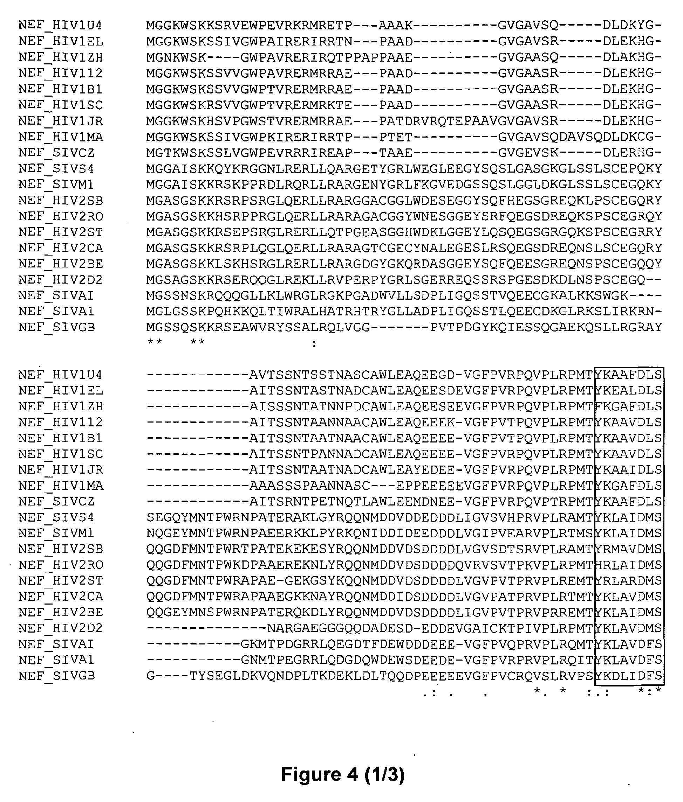

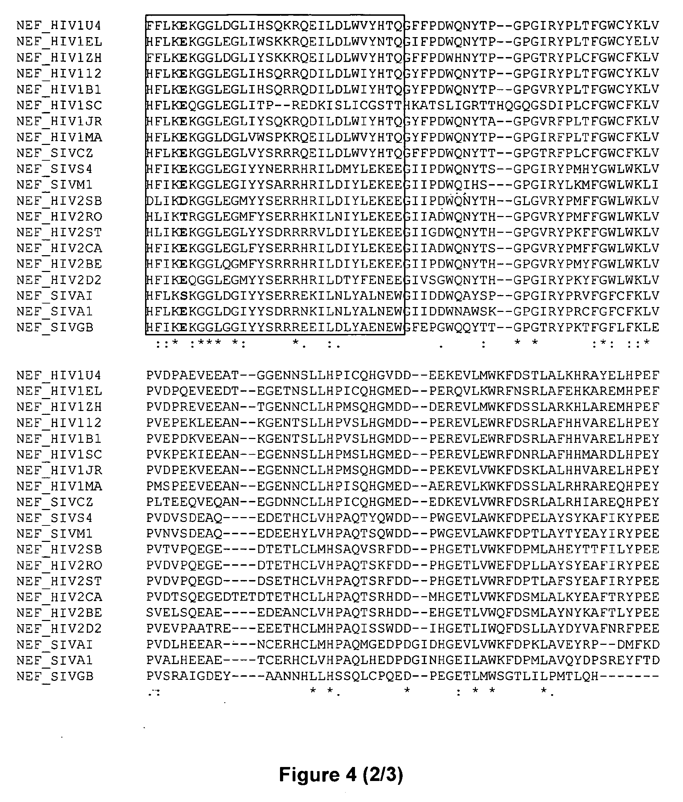

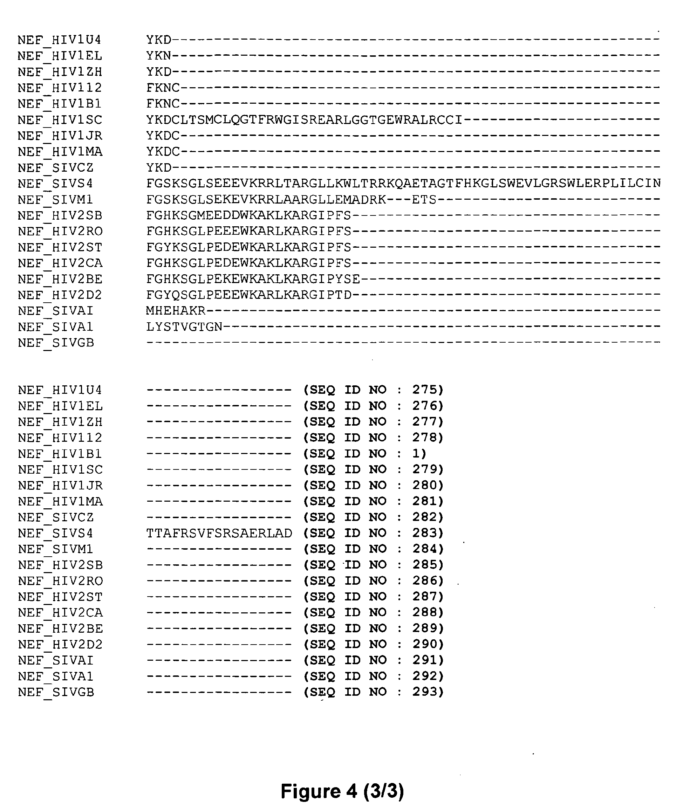

The present invention relates to a pharmaceutical or vaccine composition, comprising as active substance, a mutated Nef protein obtained by the substitution of one amino acid within the immunosuppressive domain of a Nef protein, said Nef protein being constituted by one of the amino acid sequences of the group consisting of SEQ ID NO: 1, SEQ ID NO: 275, SEQ ID NO: 276, SEQ ID NO: 277, SEQ ID NO: 278, SEQ ID NO: 279, SEQ ID NO: 280, SEQ ID NO: 281, SEQ ID NO: 282, SEQ ID NO: 283, SEQ ID NO: 284, SEQ ID NO : 285, SEQ ID NO: 286, SEQ ID NO: 287, SEQ ID NO: 288, SEQ ID NO: 289, SEQ ID NO: 290, SEQ ID NO: 291, SEQ ID NO: 292 and SEQ IDNO: 293, as depicted in Figure 4, said mutated Nef protein having substantially no immunosuppressive activity, wherein
- the amino acid at position 93 of one of SEQ ID NO: 1, SEQ ID NO: 275, SEQ ID NO: 277, SEQ ID NO: 278, SEQ ID NO: 279, SEQ ID NO: 282or
- the amino acid at position 94 of SEQ ID NO: 276, or
- the amino acid at position 96 of SEQ ID NO: 281, or
- the amino acid at position 103 of SEQ ID NO: 280, or
- the amino acid at position 109 of SEQ ID NO: 291, or
- the amino acid at position 112 of SEQ ID NO: 292, or
- the amino acid at position 114 of SEQ ID NO: 293, or
- the amino acid at position 124 of SEQ ID NO: 287, or
- the amino acid at position 125 of one of SEQ ID NO: 283, SEQ ID NO: 284, SEQ ID NO : 285, SEQ ID NO: 286, SEQ ID NO: 288, SEQ ID NO: 289, and SEQ ID NO: 290,
is replaced by R, H or K,
in association with a pharmaceutically acceptable carrier.

The present invention is illustrated by the use, in particular the *in vitro* or to the *ex vivo* use, of a mutation of at least one amino acid in the immunosuppressive domain of a Nef protein, for modulating the immunosuppressive property of said protein.

*In vivo,* the Nef protein is in particular found in HIV (such as HIV-1 or HIV-2) infected individuals or in SIV infected apes.

As intended herein, a mutation either relates to the substitution, the insertion or the deletion of at least one amino acid purposely brought to a Nef protein, or to the naturally occurring substitution, insertion or deletion of at least one amino acid in a given Nef protein with respect to the majority of Nef proteins (*i.e.* at least about 80% of the identified Nef proteins).

According to the invention, a given protein is said to hold an immunosuppressive property, if it is liable to inhibit the immune system of an organism in which it is present. In particular, the immunosuppressive property of said given protein can be measured by following the general procedure described in Mangeney & Heidmann (1998) Proc. Natl. Acad. Sci. U.S.A. 95:14920-5 and Mangeney et al. (2001) J. Gen. Virol. 82:2515-8. That is, stable tumor cell lines expressing, or in particular excreting, said given protein in the intra- or extracellular space are established and engrafted onto mice, and the size of the tumors (Aₚᵣₒₜₑᵢₙ) is compared, after several days, to the size of tumors (Aₙₒₙₑ) obtained from mice engrafted with tumor cell lines which do not express, or in particular excrete, said given protein. If the size of the tumors which express, or in particular excrete, the given protein is significantly greater than the size of the non-expressing, or in particular the non-excreting tumors, the given protein is said to be immunosuppressive. The immunosuppressive property of a given protein can also be characterized by its immunosuppression index [(Aₚᵣₒₜₑᵢₙ-Aₙₒₙₑ)/Aₙₒₙₑ]. If the immunosuppression index of a given protein is positive then the given protein is said to be immunosuppressive, and if its immunosuppression index is equal to zero or negative, the given protein is said to have essentially no immunosuppressive activity.

The present invention results from the relation which has been established by the Inventors between the immunosuppressive properties of HIV or SIV and the Nef protein. In other terms, the present invention results from the identification of the immunosuppressive function of the Nef protein, which is furthermore shown to be both an intra and an extracellular function. Further, the Inventors have shown that the immunosuppressive function of Nef is independent from the Nef-induced downregulation of CD4 or MHC-I.

Thus, the invention is illustrated by the use of a mutation of at least one amino acid in the immunosuppressive domain of a Nef protein, for modulating the immunosuppressive property of said protein, provided that the resulting mutated protein presents substantially preserved CD4 and/or MHC-I down-regulation functions with respect to the non-mutated Nef protein.

As intended herein the expression "substantially preserved CD4 and/or MHC-I down-regulation functions" means that at least 60%, in particular at least 80% of the CD4 and/or MHC-I downregulation functions of a given Nef protein is preserved in the corresponding Nef protein carrying a mutation according to the invention.

The donwregulation of CD4 and MHC-I can be determined by measuring the fluorescence of CD4 or MHC-I expressing cells transformed with increasing amounts of nucleic acids encoding said given protein and contacted with fluorescent anti-CD4 or anti-MHC-I antibodies. Such methods are well known to the man skilled in the art and are in particular described in the following examples.

The immunosuppressive domain of an immunosuppressive protein is defined as being the region of said protein which is responsible for conferring its immunosuppressive activity to said protein and, in particular, it is constituted of all the amino acids, the mutation of which is liable to modulate the immunosuppressive property of said protein.

As intended herein, the expression "modulating the immunosuppressive property" of a given protein relates to an increase or a decrease in the immunosuppressive property of said protein.

The invention is illustrated by the above defined use of a mutation of at least one amino acid in the immunosuppressive domain of a Nef protein, for inhibiting the immunosuppressive property of said protein. According to this embodiment the Nef protein presents an imunosuppressive property.

As intended in the present invention, the inhibiting of the immunosuppressive property of a given protein, yields a protein with substantially no immunosuppressive activity, that is having an immunosuppression index equal to zero or negative.

Nef proteins devoid of immunosuppressivity are particularly advantageous for the manufacture of anti-HIV or anti-SIV vaccines. Indeed, vaccine compositions containing Nef proteins devoid of immunosuppressivity according to the present invention are particularly effective at preventing HIV or SIV infections since they potently stimulate the immune response and in particular the production of antibodies directed against the Nef protein and the elicitation of a cellular immune response against infected cells which express the Nef protein. This stimulation of the immune response therefore prevents the subsequent immunosuppressive action of Nef when it is liberated in the organism or expressed by infected cells during the initial steps of HIV or SIV infection. Thus, the absence of immunosuppression conveyed by the Nef protein, which results from the immune response elicited against Nef, prevents the HIV or SIV precocious infectious cycles from being effective and favour the elimination of the virus by the immune system.

In particular, vaccine compositions according to the invention are more effective than Nef-containing compositions of the prior art to induce an anti-HIV or anti-SIV response from the immune system, since it is herein disclosed that non-mutated Nef is in itself an inhibitor of the immune system.

The invention is illustrated by the above defined use, to obtain a Nef protein mutated in its immunosuppressive domain, or a fragment thereof, provided said fragment comprises the mutated immunosuppressive domain of said Nef protein, for the manufacture of a medicament or a vaccine intended for the prevention and/or the treatment of viral diseases.

As intended herein, viral diseases encompass all diseases or syndromes resulting from a viral infection, such as AIDS for instance. Besides, vaccines according to the invention are meant to be used prophylactically or therapeutically.

The invention is illustrated by the above defined use, wherein the structure of the Nef protein is substantially preserved.

The substantial preservation of the structure of a Nef protein mutated in its immunosuppressive domain with respect to its natural counterpart can be for instance determined by comparing the circular dichroism spectra, the RMN spectra, the X-ray diffraction pattern, or any other physicochemical property of said mutated Nef protein with that of the natural Nef protein from which it derives, according to methods well known to the man skilled in the art. It is to be noted that, as intended herein, the natural Nef protein from which the mutated Nef protein is deriving presents an immunosuppressive activity.

The invention is illustrated by the above defined use, wherein the epitopes, in particular the conformational epitopes, of the Nef protein are substantially preserved. In particular, B-cell epitopes as well as T-cell epitopes are preserved.

More particularly, the invention is illustrated by the above defined use, wherein the epitopes, in particular the conformational epitopes, located outside of the immunosuppressive domain of the Nef protein are substantially preserved.

The substantial preservation of the epitopes for a Nef protein mutated in its immunosuppressive domain, with respect to its natural counterpart, can be for instance determined by checking that antibodies known to bind to a natural Nef protein, also bind to the corresponding Nef mutant.

The invention is illustrated by the above defined use, wherein the intracellular functional properties of the Nef protein other than its immunosuppressive properties are substantially preserved.

The invention is illustrated by the above defined use, wherein the CD4 and/or MHC-I down-regulation functions of the Nef protein are substantially preserved.

The intracellular functional properties of the Nef protein other than its immunosuppressive properties relate to the non-immunosuppressive functions of the protein which are only operative when said protein is hosted inside a cell. Such functions notably comprise the down-regulation of CD4 and MHC-I expression.

The down-regulation of CD4 and MHC-I expression by a given protein can be determined by measuring the fluorescence of CD4 or MHC-I expressing cells transformed with increasing amounts of nucleic acids encoding said given protein and contacted with fluorescent anti-CD4 or anti-MHC-I antibodies. Such methods are well known to the man skilled in the art and are in particular described in the following examples.

The present invention is also illustrated by a process for cancelling the immunosuppressive property of a Nef protein, comprising:
- mutating the immunosuppressive domain of said Nef protein by deletion, substitution or insertion of at least one amino acid,
- checking the cancelling of said immunosuppressive activity by an *in vivo* immunosupressivity assay,

The *in vivo* immunosuppressivity assay corresponds to the above described assay.

A preferred illustration of the above mentioned process comprises a further step of checking that the structure and/or the epitopes, in particular the epitopes located outside the immunosuppressive domain, of the Nef protein are substantially preserved.

Another preferred illustration of the above mentioned process comprises a further step of checking that the structure and/or the epitopes, in particular the epitopes located outside the immunosuppressive domain, and/or the CD4 and/or MHC-I down-regulation functions, of the Nef protein are substantially preserved.

The substantial preservation of the structure and/or the epitopes, and/or the CD4 and/or MHC-I down-regulation functions, of the Nef protein can be determined as described above.

The present invention is illustrated in particular to a pharmaceutical or vaccine composition, comprising as active substance, a protein or a polypeptide comprising or being constituted of a Nef protein or a fragment thereof, wherein
- the immunosuppressive domain of said Nef protein is mutated by deletion, substitution and/or insertion of at least one amino acid, provided that said Nef protein has substantially no immunosuppressive activity, and
- said fragment comprises the mutated immunosuppressive domain of said Nef protein and has substantially no immunosuppressive activity,
in association with a pharmaceutically acceptable carrier.

In particular, the sequences adjacent to the respective N-terminal and C-terminal ends of said fragment can be identical to the sequences adjacent to the respective N-terminal end and C-terminal end of said fragment in the Nef protein from which it derives.

In a particular illustration of the above mentioned pharmaceutical or vaccine composition the protein or polypeptide comprising a fragment of Nef protein is such that the sequences adjacent to the respective N-terminal and/or C-terminal end of said fragment are different from the sequences adjacent to the respective N-terminal end and/or C-terminal end of said fragment in the Nef protein from which it derives.

More particularly, in another illustration of the above mentioned pharmaceutical or vaccine composition, the protein or polypeptide comprising a fragment of Nef protein is such that:
- the sequence adjacent to the N-terminal end of said fragment is different from the sequences adjacent to the N-terminal end of said fragment in the Nef protein from which it derives, or
- the sequence adjacent to the C-terminal end of said fragment is different from the sequences adjacent to the C-terminal end of said fragment in the Nef protein from which it derives, or
- the sequence adjacent to the respective N-terminal and C-terminal ends of said fragment are different from the sequences adjacent to the respective N-terminal and C-terminal ends of said fragment in the Nef protein from which it derives.

The mutated Nef protein or fragment thereof according to the invention is said to be immunosuppressive deficient.

The present invention is also illustrated by a pharmaceutical or vaccine composition, comprising as active substance, a protein or a polypeptide comprising or being constituted of a Nef protein or a fragment thereof, wherein
- the immunosuppressive domain of said Nef protein is mutated by deletion, substitution and/or insertion of at least one amino acid, provided that said Nef protein has substantially no immunosuppressive activity and that the CD4 and/or MHC-I down-regulation functions, of the Nef protein are substantially preserved, and
- said fragment comprises the mutated immunosuppressive domain of said Nef protein and has substantially no immunosuppressive activity,
in association with a pharmaceutically acceptable carrier.

In a preferred illustration of the above defined pharmaceutical or vaccine composition, the sequence of the mutated immunosuppressive domain of the Nef protein is comprised in the amino acid sequence extending from the N-terminus of the first α helix to the C-terminus of the second α helix of the Nef protein.

The structure of the Nef protein is in particular described in Arold et al. (1997) Structure 5:1361-72 and in Grzesiek et al. (1997) Protein Science 6:1248-63. The nomenclature of the secondary structure elements of the Nef protein, and in particular of its α helices, is based on the structural description of the core domain of the Nef protein, according to Arold *et al.* (1997) and Grzesiek *et al.* (1997).

In another preferred illustration of the above defined pharmaceutical or vaccine composition, the sequence of the mutated immunosuppressive domain of the Nef protein is comprised in a sequence ranging from the amino acid at position 80 to the amino acid at position 150, particularly from the amino acid at position 81 to the amino acid at position 140, of the sequence of said Nef protein, and in particular:
- in a sequence ranging from the amino acid at position 80 to the amino acid at position 120, more particularly from the amino acid at position 81 to the amino acid at position 118, of the sequence of a HIV-1 Nef protein, or
- in a sequence ranging from the amino acid at position 104 to the amino acid at position 150, in particular from the amino acid at position 104 to the amino acid at position 140, of the sequence of a HIV-2 Nef protein.

Nef protein sequences can be easily accessed by the man skilled in the art. By way of example, several HIV-1, HIV-2 or SIV Nef protein sequences are presented in **Figure 4****.**

More preferably, in the above defined pharmaceutical or vaccine composition, the sequence of the mutated immunosuppressive domain of the HIV-1 Nef protein is comprised in a sequence ranging from the amino acid at position 90 to the amino acid at position 113, in particular from the amino acid at position 90 to the amino acid at position 112, of the sequence of said Nef protein.

In a particular illustration of the above defined pharmaceutical or vaccine composition, the sequence of the mutated immunosuppressive domain of the Nef protein is comprised in a sequence which is homologous to the amino acid sequence ranging from the amino acid at position 80 to the amino acid at position 120 of SEQ ID NO: 1, in particular from the amino acid at position 81 to the amino acid at position 117 of SEQ ID NO: 1, more particularly from the amino acid at position 90 to the amino acid at position 112 of SEQ ID NO: 1.

SEQ ID NO: 1 corresponds to the amino acid sequence of the Nef protein described by Wain-Hobson et al. (1985) Cell 40:9-17 (HIV-1 strain LAI).

According to the invention, two sequences are said to be homologous if they can be aligned by using an algorithm such as defined in Altschul et al., Nucleic Acids Res. (1997) 25:3389 or by using the Clustal W software, well known from the man skilled in the art and described in Thompson et al., Nucleic Acids Res. (1994) 22:4673-4680, for instance.

In particular, two sequences are said to be homologous if the amino acid identity percentage between said two sequences is equal to or larger than about 35%.

By way of example, **Figure 4** represents a sequence alignment of several Nef proteins originating from HIV-1, HIV-2 or SIV, as obtained with the Clustal W software. Sequences homologous to the amino acid sequence ranging from the amino acid at position 81 to the amino acid at position 118 of SEQ ID NO: 1 are boxed.

In an embodiment, the invention relates to a vaccine composition as defined above, wherein
- the amino acid at position 93 of one of SEQ ID NO: 1, SEQ ID NO: 275, SEQ ID NO: 277, SEQ ID NO: 278, SEQ ID NO: 279, SEQ ID NO: 282or
- the amino acid at position 94 of SEQ ID NO: 276, or
- the amino acid at position 96 of SEQ ID NO: 281, or
- the amino acid at position 103 of SEQ ID NO: 280, or
- the amino acid at position 109 of SEQ ID NO: 291, or
- the amino acid at position 112 of SEQ ID NO: 292, or
- the amino acid at position 114 of SEQ ID NO: 293, or
- the amino acid at position 124 of SEQ ID NO: 287, or
- the amino acid at position 125 of one of SEQ ID NO: 283, SEQ ID NO: 284, SEQ ID NO : 285, SEQ ID NO: 286, SEQ ID NO: 288, SEQ ID NO: 289, and SEQ ID NO: 290,
is replaced by R.

In another particular illustration of the above defined pharmaceutical or vaccine composition, the sequence of the mutated immunosuppressive domain of the Nef protein is comprised in a 26 or 27 amino acid-long sequence of said Nef protein, the N-terminal end of said 26 or 27 amino acid-long sequence being the pentapeptide AX₁DX₂S and the C-terminal end of said 26 or 27 amino acid-long sequence being the amino acid L, in which X₁ represents any amino acid, and in particular I, V, L, F, or R, and X₂ represents any amino acid, and in particular M, L, or F.

Examples of such sequences are presented in **Figure 4****.**

In yet another particular illustration of the invention, the above defined pharmaceutical or vaccine composition comprises as active substance a protein or polypeptide comprising or being constituted of a Nef protein or a fragment thereof comprising the following sequence:
AX₁DX₂SX₃X₄X₅KX₆X₇GX₈LX₉G (SEQ ID NO: 3)
wherein
X₁ represents I, L, V, F, or R,
X₂ represents M, L, or F,
X₃ represents H, D, or F,
X₄ represents F or L,
X₅ represents I or L,
X₆ represents any amino acid different from E, in particular R,
X₇ represents K, Q, or R,
X₈ represents G or no amino acid,
X₉ represents E, D, or R.

SEQ ID NO: 3 comprises the immunosuppressive domain of Nef.

Examples of such sequences are presented in **Figure 4****.**

In a particularly preferred illustration of the invention, the above defined pharmaceutical or vaccine composition comprises as active substance a protein or polypeptide comprising or being constituted of a Nef protein or a fragment thereof, wherein the amino acid homologous to the amino acid at position 93 of SEQ ID NO: 1 is replaced by any amino acid different from E, in particular by W, F, M, Y, R, H or K, more particularly by R, H, or K, and preferably by R.

The amino acid homologous to the amino acid at position 93 of SEQ ID NO: 1 can be determined by aligning the sequence of the above mentioned protein or polypeptide with SEQ ID NO: 1 (for instance using the Clutal W software) and by selecting the amino acid which is aligned with the amino acid at position 93 of SEQ ID NO: 1. By way of example **Figure 4** represents the amino acid homologous to the amino acid at position 93 of SEQ ID NO: 1 for several Nef proteins originating from HIV-1, HIV-2 or SIV. Advantageously, the single substitution of the amino acid homologous to the amino acid at position 93 of SEQ ID NO: 1 amino acid yields Nef mutants substantially devoid of immunosuppressivity.

In a particularly preferred illustration of the invention, the above defined pharmaceutical or vaccine composition comprises as active substance, a protein or polypeptide comprising or being constituted of a HIV-1 Nef protein or a fragment thereof, wherein the amino acid at position 93 of the sequence of said HIV-1 Nef protein is replaced by any amino acid different from E, in particular by W, F, M, Y, R, H or K, more particularly by R, H, or K, and preferably by R.

In another particularly preferred illustration of the invention, the above defined pharmaceutical or vaccine composition comprises as active substance a Nef protein, wherein the amino acid homologous to the amino acid at position 93 of SEQ ID NO: 1 is replaced by any amino acid different from E, in particular by W, F, M, Y, R, H or K, more particularly by R, H, or K, and preferably by R.

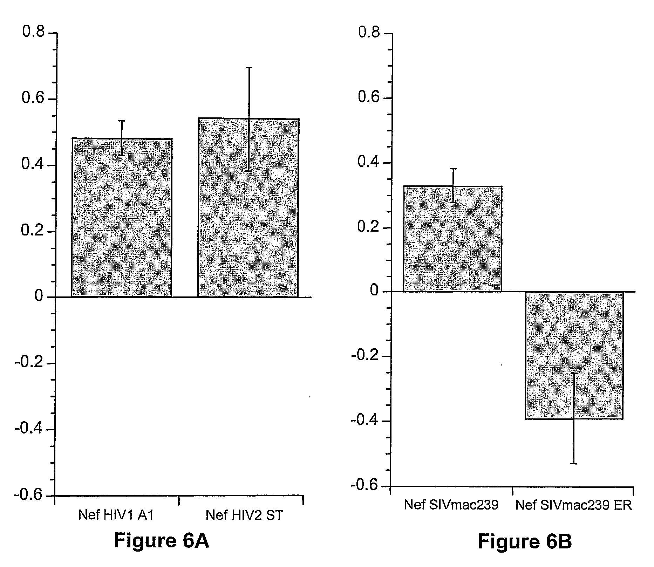

By way of example, as depicted in **Figure 6B****,** the position homologous to the position 93 of HIV-1 Nef corresponds to position 125 in SIV strain mac239 Nef (SEQ ID NO: 22) and the substitution of the E at position 125 in SIV strain mac239 Nef by R yields an immunosuppressive-deficient Nef mutant (SEQ ID NO: 23).

According to a most preferred embodiment of the invention, the above defined pharmaceutical or vaccine composition comprises as active substance a mutated Nef protein corresponding to SEQ ID NO: 2.

In yet another preferred illustration of the invention, the above defined pharmaceutical or vaccine composition is characterized in that when a Nef protein is comprised in said pharmaceutical or vaccine composition, the structure of the Nef protein is substantially preserved.

In a further preferred illustration of the invention, the above defined pharmaceutical or vaccine composition is characterized in that when a Nef protein is comprised in said pharmaceutical or vaccine composition, the epitopes, such as B cell or T cell epitopes, in particular the conformational epitopes, of the Nef protein are substantially preserved.

More particularly, the invention relates to the above defined pharmaceutical or vaccine composition, wherein the epitopes, in particular the conformational epitopes, located outside of the immunosuppressive domain of the Nef protein are substantially preserved.

In another further preferred embodiment of the invention, the above defined pharmaceutical or vaccine composition is characterized in that when a Nef protein is comprised in said pharmaceutical or vaccine composition, the intracellular functional properties other than the immunosuppressive properties of the Nef protein are substantially preserved.

More preferably, the invention relates to the above defined pharmaceutical or vaccine composition, wherein the CD4 and/or MHC-I down-regulation functions of the Nef protein are substantially preserved.

The invention relates to the above defined pharmaceutical or vaccine composition, wherein at least 60% of the CD4 and/or MHC-I downregulation functions are preserved in the mutated Nef protein with respect to the non mutated Nef protein.

The present invention is also illustrated by the protein or polypeptide as defined in the above mentioned pharmaceutical or vaccine composition.

In particular, the present invention relates to a protein represented by SEQ ID NO: 2 or SEQ ID NO: 31.

The present invention also relates to a pharmaceutical or vaccine composition, comprising as active substance a nucleic acid encoding a protein or polypeptide such as defined above.

The present invention is also illustrated by the nucleic acid sequences coding for the protein or the polypeptide as defined in the above mentioned pharmaceutical or vaccine composition.

In particular, the present invention relates to a nucleic acid sequence coding for a protein represented by SEQ ID NO: 2 or SEQ ID NO: 31.

The present invention also relates to the use of a protein as defined above, or of a nucleic acid as defined above, for the manufacture of a medicament or a vaccine intended for the prevention and/or the treatment of viral diseases, such as HIV infections.

The present invention is also illustrated by the use of a protein or a polypeptide as defined above, or of a nucleic acid as defined above, for the manufacture of a medicament or a vaccine intended for the prevention and/or the treatment of viral diseases, such as HIV infections.

In a preferred illustration of the invention, the above defined medicament, or the above defined pharmaceutical or vaccine composition comprising a protein or a polypeptide as defined above as active substance, also comprise at least one HIV protein or lipopeptide, or a fragment thereof, in particular selected from gp41, gp120, gp140, gp160, Env, Gag, Pol, Rev, RT, Vpu or Tat.

In a preferred illustration of the invention, the above defined medicament, or the above defined pharmaceutical or vaccine composition comprising as active substance a nucleic acid encoding a protein or polypeptide such as defined above, also comprise at least one nucleic acid encoding a HIV protein, or a fragment thereof, in particular selected from a nucleic acid encoding gp120, gp140, gp160, Env, Gag, Pol, Rev, RT, Vpu or Tat.

In another preferred illustration of the above defined medicament, or the above defined pharmaceutical or vaccine composition comprising as active substance a nucleic acid encoding a protein or polypeptide such as defined above, the nucleic acid is naked or comprised in a vector, in particular selected from a canarypox viral vector, an adenoviral vector, or a measles viral vector.

The present invention is also illustrated by the use of a protein or a polypeptide as defined above, for the preparation of:
- polyclonal or monoclonal antibodies, or fragments thereof, such as Fab or F(ab)'₂ fragments, directed against said protein or polypeptide as defined above,
- scFv polypeptides directed against said protein or polypeptide as defined above,
- aptamers directed against said protein or polypeptide as defined above,
- binding peptides directed against said protein or polypeptide as defined above.

The procedures for the preparation of the above mentioned antibodies or fragments of antibodies, scFv polypeptides, aptamers, or binding peptides, are particularly well known to the man skilled in the art. As regards binding peptides, they can also be prepared according to methods well known to the man skilled in the art, such as ribosome or phage display methods.

The present invention is also illustrated by antibodies or fragments thereof, scFv polypeptides, aptamers, or binding peptides, directed against the above defined proteins or polypeptides involved in the invention, provided that said antibodies or fragments thereof, scFv polypeptides, or aptamers do not bind to proteins or polypeptides different from the above defined proteins or polypeptides involved in the invention.

As intended herein, the above defined antibodies or fragments thereof, scFv polypeptides, aptamers, or binding peptides, bind specifically to the proteins or the polypeptides according to the invention, in other words they are specific ligands for the proteins or the polypeptides according to the invention. In particular, the specificity of these ligands is such that they bind to the proteins or polypeptides according to the invention, but not to the proteins from which said proteins or polypeptides according to the invention are derived by mutation.

The present invention is also illustrated by a method for preparing mutants of a Nef protein, wherein:
in a first step, the sequence ranging from the amino acid at position 80 to the amino acid at position 150 of the sequence said Nef protein is mutated by deletion, insertion or substitution of at least one amino acid,
- in a second step, the immunosuppressive properties of the mutated Nef protein obtained in the first step are checked and mutants lacking immunosuppressive properties are selected.

The mutants obtained according to this method are immunosuppressive-deficient mutants.

In a preferred illustration of the above defined method for preparing mutants of a Nef protein, in a third step the CD4 and/or MHC-I downregulation functions of the mutated Nef protein obtained in the second step are checked and mutated Nef proteins having substantially preserved CD4 and/or MHC-I downregulation functions with respect to said Nef protein are selected.

In another preferred illustration of the above defined method for preparing mutants of a Nef protein, the mutated sequence ranges:
- from the amino acid at position 80 to the amino acid at position 120, more particularly from the amino acid at position 90 to the amino acid at position 112, of the sequence of a HIV-1 protein, or
- from the amino acid at position 104 to the amino acid at position 150 of the sequence of a HIV-2 Nef protein.

In another preferred illustration of the above defined method for preparing mutants of a Nef protein, the sequence of the Nef protein is mutated by directed mutagenesis of the nucleic acid sequence coding for said Nef protein.

In another preferred illustration of the above defined method for preparing mutants of a Nef protein, the immunosuppressive properties of the mutated Nef protein are checked according to the general procedure described in Mangeney & Heidmann (1998) Proc. Natl. Acad. Sci. U.S.A. 95:14920-5 and Mangeney et al. (2001) J. Gen.Virol. 82:2515-8 as defined above, in particular the above-defined immunosuppression index is measured and mutated Nef protein having immunosuppression indexes equal to zero or negative are selected.

The down-regulation of CD4 and MHC-I expression by a given protein can be determined by measuring the fluorescence of CD4 or MHC-I expressing cells transformed with increasing amounts of nucleic acids encoding said given protein and contacted with fluorescent anti-CD4 or anti-MHC-I antibodies. Such methods are well known to the man skilled in the art and are in particular described in the following examples.

The present invention is also illustrated by the mutants of a Nef protein liable to be prepared by the above defined method and to pharmaceutical compositions comprising said mutants of a Nef protein in association with a pharmaceutically acceptable carrier.

The present invention is also illustrated by a new protein or polypeptide comprising or being constituted by the immunosuppressive domain of a Nef protein, provided that if present, the sequences adjacent to the respective N-terminal end and/or C-terminal end of the immunosuppressive domain in said protein or polypeptide are different from the sequences adjacent to the respective N-terminal end and/or C-terminal end of the immunosuppressive domain in the Nef protein from which it derives.

More particularly, in an illustration of the above defined new protein or polypeptide:
- the sequence adjacent to the N-terminal end of the immunosuppressive domain is different from the sequences adjacent to the N-terminal end of the immunosuppressive domain in the Nef protein from which it derives, or
- the sequence adjacent to the C-terminal end of the immunosuppressive domain is different from the sequences adjacent to the C-terminal end of the immunosuppressive domain in the Nef protein from which it derives, or
- the sequence adjacent to the respective N-terminal and C-terminal ends of the immunosuppressive domain are different from the sequences adjacent to the respective N-terminal and C-terminal ends of the immunosuppressive domain in the Nef protein from which it derives.

In a particular illustration of the present invention, the new protein or polypeptide as defined above presents CD4 and/or MHC-I down-regulation functions.

The Nef immunosuppressive domain which constitutes or is comprised in the above defined new protein or polypeptide can be either mutated or not with respect to the immunosuppressive domain of naturally occurring Nef proteins.

Thus, the new protein or polypeptide as defined above can be immunosuppressive, in the general case, if the Nef immunosuppressive domain which it comprises or which it is constituted of, derives without mutations from a naturally occuring Nef protein, which generally presents immunosuppressive properties.

The new protein or polypeptide can also comprise or be constituted of a Nef immunosuppressive domain which is mutated with respect to its natural form. This mutation can either be silent as concerns the immunosuppressive properties of the Nef immunosuppressive domain, which means, in the general case, that it does not affect the immunosuppressive properties of the Nef immunosuppressive domain, or the mutation can render the immunosuppressive domain immunosuppressive-deficient, as is the case for the above-mentioned mutations affecting Nef immunosuppressive domain.

Further, in certain particular cases, the immunosuppressive domain can also derive from naturally occuring Nef variants devoid of immunosuppressive properties.

In a preferred illustration of the above defined new protein or polypeptide, the sequence of the immunosuppressive domain of the Nef protein is comprised in the amino acid sequence extending from the N-terminus of the first α helix to the C-terminus of the second α helix of the Nef protein.

In another preferred illustration of the above defined new protein or polypeptide, the sequence of the immunosuppressive domain of the Nef protein is comprised in a sequence ranging from the amino acid at position 80 to the amino acid at position 150, particularly from the amino acid at position 81 to the amino acid at position 140, of the sequence of said Nef protein, and in particular:
- in a sequence ranging from the amino acid at position 80 to the amino acid at position 120, more particularly from the amino acid at position 81 to the amino acid at position 118, of the sequence of a HIV-1 Nef protein
- in a sequence ranging from the amino acid at position 104 to the amino acid at position 150, in particular from the amino acid at position 104 to the amino acid at position 140, of the sequence of a HIV-2 Nef protein.

More preferably, in the above defined new protein or polypeptide, the sequence of the immunosuppressive domain of the HIV-1 Nef protein is comprised in a sequence ranging from the amino acid at position 90 to the amino acid at position 113, in particular from the amino acid at position 90 to the amino acid at position 112, of the sequence of said Nef protein.

In another preferred illustration of the above defined new protein or polypeptide, the sequence of the immunosuppressive domain of the Nef protein is comprised in a sequence which is homologous to the amino acid sequence ranging from the amino acid at position 80 to the amino acid at position 120 of SEQ ID NO: 1, in particular from the amino acid at position 81 to the amino acid at position 117 of SEQ ID NO: 1, more particularly from the amino acid at position 90 to the amino acid at position 112 of SEQ ID NO: 1.

In a particularly preferred illustration of the above defined new protein or polypeptide, the sequence of the immunosuppressive domain of the Nef protein is comprised in a 26 or 27 amino acid-long sequence, the N-terminal end of said Nef protein, the N-terminal end of said 26 or 27 amino acid-long sequence being the pentapeptide AX₁DX₂S and the C-terminal end of said 26 or 27 amino acid-long sequence being the amino acid L, in which X₁ represents any amino acid, and in particular I, V, L, F, or R, and X₂ represents any amino acid, and in particular M, L, or F.

In a preferred illustration of the present invention, the Nef immunosuppressive domain which constitutes or is comprised in the above defined new protein or polypeptide is not mutated, such a domain is said to be non-mutated, and is derived from a naturally immunosuppressive Nef protein. Advantageously, the new protein or polypeptide which comprises or is constituted of such a non-mutated domain is immunosuppressive.

In another preferred illustration, the new protein or polypeptide is constituted of one of the following HIV-1 Nef fragments :
80-120 TYKAAVDLSHFLKEKGGLEGLIHSQRRQDILDLWIYHTQGY (SEQ ID NO: 32)
81-120 YKAAVDLSHFLKEKGGLEGLIHSQRRQDILDLWIYHTQGY (SEQ ID NO: 33)
82-120 KAAVDLSHFLKEKGGLEGLIHSQRRQDILDLWIYHTQGY (SEQ ID NO: 34)
83-120 AAVDLSHFLKEKGGLEGLIHSQRRQDILDLWIYHTQGY (SEQ ID NO: 35)
84-120 AVDLSHFLKEKGGLEGLIHSQRRQDILDLWIYHTQGY (SEQ ID NO: 36)
85-120 VDLSHFLKEKGGLEGLIHSQRRQDILDLWIYHTQGY (SEQ ID NO: 37)
86-120 DLSHFLKEKGGLEGLIHSQRRQDILDLWIYHTQGY (SEQ ID NO: 38)
87-120 LSHFLKEKGGLEGLIHSQRRQDILDLWIYHTQGY (SEQ ID NO: 39)
88-120 SHFLKEKGGLEGLIHSQRRQDILDLWIYHTQGY (SEQ ID NO: 40)
89-120 HFLKEKGGLEGLIHSQRRQDILDLWIYHTQGY (SEQ ID NO: 41)
90-120 FLKEKGGLEGLIHSQRRQDILDLWIYHTQGY (SEQ ID NO: 42)
80-119 TYKAAVDLSHFLKEKGGLEGLIHSQRRQDILDLWIYHTQG (SEQ ID NO: 43)
81-119 YKAAVDLSHFLKEKGGLEGLIHSQRRQDILDLWIYHTQG (SEQ ID NO: 44)
82-119 KAAVDLSHFLKEKGGLEGLIHSQRRQDILDLWIYHTQG (SEQ ID NO: 45)
83-119 AAVDLSHFLKEKGGLEGLIHSQRRQDILDLWIYHTQG (SEQ ID NO: 46)
84-119 AVDLSHFLKEKGGLEGLIHSQRRQDILDLWIYHTQG (SEQ ID NO: 47)
85-119 VDLSHFLKEKGGLEGLIHSQRRQDILDLWIYHTQG (SEQ ID NO: 48)
86-119 DLSHFLKEKGGLEGLIHSQRRQDILDLWIYHTQG (SEQ ID NO: 49)
87-119 LSHFLKEKGGLEGLIHSQRRQDILDLWIYHTQG (SEQ ID NO: 50)
88-119 SHFLKEKGGLEGLIHSQRRQDILDLWIYHTQG (SEQ ID NO: 51)
89-119 HFLKEKGGLEGLIHSQRRQDILDLWIYHTQG (SEQ ID NO: 52)
90-119 FLKEKGGLEGLIHSQRRQDILDLWIYHTQG (SEQ ID NO: 53)
80-118 TYKAAVDLSHFLKEKGGLEGLIHSQRRQDILDLWIYHTQ (SEQ ID NO: 54)
81-118 YKAAVDLSHFLKEKGGLEGLIHSQRRQDILDLWIYHTQ (SEQ ID NO: 55)
82-118 KAAVDLSHFLKEKGGLEGLIHSQRRQDILDLWIYHTQ (SEQ ID NO: 56)
83-118 AAVDLSHFLKEKGGLEGLIHSQRRQDILDLWIYHTQ (SEQ ID NO: 57)
84-118 AVDLSHFLKEKGGLEGLIHSQRRQDILDLWIYHTQ (SEQ ID NO: 58)
85-118 VDLSHFLKEKGGLEGLIHSQRRQDILDLWIYHTQ (SEQ ID NO: 59)
86-118 DLSHFLKEKGGLEGLIHSQRRQDILDLWIYHTQ (SEQ ID NO: 60)
87-118 LSHFLKEKGGLEGLIHSQRRQDILDLWIYHTQ (SEQ ID NO: 61)
88-118 SHFLKEKGGLEGLIHSQRRQDILDLWIYHTQ (SEQ ID NO: 62)
89-118 HFLKEKGGLEGLIHSQRRQDILDLWIYHTQ (SEQ ID NO: 63)
90-118 FLKEKGGLEGLIHSQRRQDILDLWIYHTQ (SEQ ID NO: 64)
80-117 TYKAAVDLSHFLKEKGGLEGLIHSQRRQDILDLWIYHT (SEQ ID NO: 65)
81-117 YKAAVDLSHFLKEKGGLEGLIHSQRRQDILDLWIYHT (SEQ ID NO: 66)
82-117 KAAVDLSHFLKEKGGLEGLIHSQRRQDILDLWIYHT (SEQ ID NO: 67)
83-117 AAVDLSHFLKEKGGLEGLIHSQRRQDILDLWIYHT (SEQ ID NO: 68)
84-117 AVDLSHFLKEKGGLEGLIHSQRRQDILDLWIYHT (SEQ ID NO: 69)
85-117 VDLSHFLKEKGGLEGLIHSQRRQDILDLWIYHT (SEQ ID NO: 70)
86-117 DLSHFLKEKGGLEGLIHSQRRQDILDLWIYHT (SEQ ID NO: 71)
87-117 LSHFLKEKGGLEGLIHSQRRQDILDLWIYHT (SEQ ID NO: 72)
88-117 SHFLKEKGGLEGLIHSQRRQDILDLWIYHT (SEQ ID NO: 73)
89-117 HFLKEKGGLEGLIHSQRRQDILDLWIYHT (SEQ ID NO: 74)
90-117 FLKEKGGLEGLIHSQRRQDILDLWIYHT (SEQ ID NO: 75)
80-116 TYKAAVDLSHFLKEKGGLEGLIHSQRRQDILDLWIYH (SEQ ID NO: 76)
81-116 YKAAVDLSHFLKEKGGLEGLIHSQRRQDILDLWIYH (SEQ ID NO: 77)
82-116 KAAVDLSHFLKEKGGLEGLIHSQRRQDILDLWIYH (SEQ ID NO: 78)
83-116 AAVDLSHFLKEKGGLEGLIHSQRRQDILDLWIYH (SEQ ID NO: 79)
84-116 AVDLSHFLKEKGGLEGLIHSQRRQDILDLWIYH (SEQ ID NO: 80)
85-116 VDLSHFLKEKGGLEGLIHSQRRQDILDLWIYH (SEQ ID NO: 81)
86-116 DLSHFLKEKGGLEGLIHSQRRQDILDLWIYH (SEQ ID NO: 82)
87-116 LSHFLKEKGGLEGLIHSQRRQDILDLWIYH (SEQ ID NO: 83)
88-116 SHFLKEKGGLEGLIHSQRRQDILDLWIYH (SEQ ID NO: 84)
89-116 HFLKEKGGLEGLIHSQRRQDILDLWIYH (SEQ ID NO: 85)
90-116 FLKEKGGLEGLIHSQRRQDILDLWIYH (SEQ ID NO: 86)
80-115 TYKAAVDLSHFLKEKGGLEGLIHSQRRQDILDLWIY (SEQ ID NO: 87)
81-115 YKAAVDLSHFLKEKGGLEGLIHSQRRQDILDLWIY (SEQ ID NO: 88)
82-115 KAAVDLSHFLKEKGGLEGLIHSQRRQDILDLWIY (SEQ ID NO: 89)
83-115 AAVDLSHFLKEKGGLEGLIHSQRRQDILDLWIY (SEQ ID NO: 90)
84-115 AVDLSHFLKEKGGLEGLIHSQRRQDILDLWIY (SEQ ID NO: 91)
85-115 VDLSHFLKEKGGLEGLIHSQRRQDILDLWIY (SEQ ID NO: 92)
86-115 DLSHFLKEKGGLEGLIHSQRRQDILDLWIY (SEQ ID NO: 93)
87-115 LSHFLKEKGGLEGLIHSQRRQDILDLWIY (SEQ ID NO: 94)
88-115 SHFLKEKGGLEGLIHSQRRQDILDLWIY (SEQ ID NO: 95)
89-115 HFLKEKGGLEGLIHSQRRQDILDLWIY (SEQ ID NO: 96)
90-115 FLKEKGGLEGLIHSQRRQDILDLWIY (SEQ ID NO: 97)
80-114 TYKAAVDLSHFLKEKGGLEGLIHSQRRQDILDLWI (SEQ ID NO: 98)
81-114 YKAAVDLSHFLKEKGGLEGLIHSQRRQDILDLWI (SEQ ID NO: 99)
82-114 KAAVDLSHFLKEKGGLEGLIHSQRRQDILDLWI (SEQ ID NO: 100)
83-114 AAVDLSHFLKEKGGLEGLIHSQRRQDILDLWI (SEQ ID NO: 101)
84-114 AVDLSHFLKEKGGLEGLIHSQRRQDILDLWI (SEQ ID NO: 102)
85-114 VDLSHFLKEKGGLEGLIHSQRRQDILDLWI (SEQ ID NO: 103)
86-114 DLSHFLKEKGGLEGLIHSQRRQDILDLWI (SEQ ID NO: 104)
87-114 LSHFLKEKGGLEGLIHSQRRQDILDLWI (SEQ ID NO: 105)
88-114 SHFLKEKGGLEGLIHSQRRQDILDLWI (SEQ ID NO: 106)
89-114 HFLKEKGGLEGLIHSQRRQDILDLWI (SEQ ID NO: 107)
90-114 FLKEKGGLEGLIHSQRRQDILDLWI (SEQ ID NO: 108)
80-113 TYKAAVDLSHFLKEKGGLEGLIHSQRRQDILDLW (SEQ ID NO: 109)
81-113 YKAAVDLSHFLKEKGGLEGLIHSQRRQDILDLW (SEQ ID NO: 110)
82-113 KAAVDLSHFLKEKGGLEGLIHSQRRQDILDLW (SEQ ID NO: 111)
83-113 AAVDLSHFLKEKGGLEGLIHSQRRQDILDLW (SEQ ID NO: 112)
84-113 AVDLSHFLKEKGGLEGLIHSQRRQDILDLW (SEQ ID NO: 113)
85-113 VDLSHFLKEKGGLEGLIHSQRRQDILDLW (SEQ ID NO: 114)
86-113 DLSHFLKEKGGLEGLIHSQRRQDILDLW (SEQ ID NO: 115)
87-113 LSHFLKEKGGLEGLIHSQRRQDILDLW (SEQ ID NO: 116)
88-113 SHFLKEKGGLEGLIHSQRRQDILDLW (SEQ ID NO: 117)
89-113 HFLKEKGGLEGLIHSQRRQDILDLW (SEQ ID NO: 118)
90-113 FLKEKGGLEGLIHSQRRQDILDLW (SEQ ID NO: 119)
80-112 TYKAAVDLSHFLKEKGGLEGLIHSQRRQDILDL (SEQ ID NO: 120)
81-112 YKAAVDLSHFLKEKGGLEGLIHSQRRQIDILDL (SEQ ID NO: 121)
82-112 KAAVDLSHFLKEKGGLEGLIHSQRRQDILDL (SEQ ID NO: 122)
83-112 AAVDLSHFLKEKGGLEGLIHSQRRQDILDL (SEQ ID NO: 123)
84-112 AVDLSHFLKEKGGLEGLIHSQRRQDILDL (SEQ ID NO: 124)
85-112 VDLSHFLKEKGGLEGLIHSQRRQDILDL (SEQ ID NO: 125)
86-112 DLSHFLKEKGGLEGLIHSQRRQDILDL (SEQ ID NO: 126)
87-112 LSHFLKEKGGLEGLIHSQRRQDILDL (SEQ ID NO: 127)
88-112 SHFLKEKGGLEGLIHSQRRQDILDL (SEQ ID NO: 128)
89-112 HFLKEKGGLEGLIHSQRRQDILDL (SEQ ID NO: 129)
90-112 FLKEKGGLEGLIHSQRRQDILDL (SEQ ID NO: 130)
or of homologous peptide sequences presenting at least 80% sequence identity, preferably 90% identity, with said HIV-1 Nef fragments.

The present invention is also illustrated by new proteins or polypeptides comprising said HIV-1 Nef fragments or homologous peptide sequences, provided that if present, the sequences adjacent to the respective N-terminal end and/or C-terminal end of the HIV-1 Nef fragments in said protein or polypeptide are different from the sequences adjacent to the respective N-terminal end and/or C-terminal end of the HIV-1 Nef fragments in the Nef proteins from which they derive.

In another preferred illustration, the new protein or polypeptide is constituted of one of the following HIV-2 Nef fragments :
104-150 RVPLREMTYRLARDMSHLIKEKGGLEGLYYSDRRRRVLDIYLEKEEG (SEQ ID NO: 131)
105-150 VPLREMTYRLARDMSHLIKEKGGLEGLYYSDRRRRVLDIYLEKEEG (SEQ ID NO: 132)
106-150 PLREMTYRLARDMSHLIKEKGGLEGLYYSDRRRRVLDIYLEKEEG (SEQ ID NO: 133)
107-150 LREMTYRLARDMSHLIKEKGGLEGLYYSDRRRRVLDIYLEKEEG (SEQ ID NO: 134)
108-150 REMTYRLARDMSHLIKEKGGLEGLYYSDRRRRVLDIYLEKEEG (SEQ ID NO: 135)
109-150 EMTYRLARDMSHLIKEKGGLEGLYYSDRRRRVLDIYLEKEEG (SEQ ID NO: 136)
110-150 MTYRLARDMSHLIKEKGGLEGLYYSDRRRRVLDIYLEKEEG (SEQ ID NO: 137)
111-150 TYRLARDMSHLIKEKGGLEGLYYSDRRRRVLDIYLEKEEG (SEQ ID NO: 138)
112-150 YRLARDMSHLIKEKGGLEGLYYSDRRRRVLDIYLEKEEG (SEQ ID NO: 139)
113-150 RLARDMSHLIKEKGGLEGLYYSDRRRRVLDIYLEKEEG (SEQ ID NO: 140)
114-150 LARDMSHLIKEKGGLEGLYYSDRRRRVLDIYLEKEEG (SEQ ID NO: 141)
115-150 ARDMSHLIKEKGGLEGLYYSDRRRRVLDIYLEKEEG (SEQ ID NO: 142)
116-150 RDMSHLIKEKGGLEGLYYSDRRRRVLDIYLEKEEG (SEQ ID NO: 143)
117-150 DMSHLIKEKGGLEGLYYSDRRRRVLDIYLEKEEG (SEQ ID NO: 144)
118-150 MSHLIKEKGGLEGLYYSDRRRRVLDIYLEKEEG (SEQ ID NO: 145)
119-150 SHLIKEKGGLEGLYYSDRRRRVLDIYLEKEEG (SEQ ID NO: 146)
120-150 HLIKEKGGLEGLYYSDRRRRVLDIYLEKEEG (SEQ ID NO: 147)
121-150 LIKEKGGLEGLYYSDRRRRVLDIYLEKEEG (SEQ ID NO: 148)
104-149 RVPLREMTYRLARDMSHLIKEKGGLEGLYYSDRRRRVLDIYLEKEE (SEQ ID NO: 149)
105-149 VPLREMTYRLARDMSHLIKEKGGLEGLYYSDRRRRVLDIYLEKEE (SEQ ID NO: 150)
106-149 PLREMTYRLARDMSHLIKEKGGLEGLYYSDRRRRVLDIYLEKEE (SEQ ID NO: 151)
107-149 LREMTYRLARDMSHLIKEKGGLEGLYYSDRRRRVLDIYLEKEE (SEQ ID NO: 152)
108-149 REMTYRLARDMSHLIKEKGGLEGLYYSDRRRRVLDIYLEKEE (SEQ ID NO: 153)
109-149 EMTYRLARDMSHLIKEKGGLEGLYYSDRRRRVLDIYLEKEE (SEQ ID NO: 154)
110-149 MTYRLARDMSHLIKEKGGLEGLYYSDRRRRVLDIYLEKEE (SEQ ID NO: 155)
111-149 TYRLARDMSHLIKEKGGLEGLYYSDRRRRVLDIYLEKEE (SEQ ID NO: 156)
112-149 YRLARDMSHLIKEKGGLEGLYYSDRRRRVLDIYLEKEE (SEQ ID NO: 157)
113-149 RLARDMSHLIKEKGGLEGLYYSDRRRRVLDIYLEKEE (SEQ ID NO: 158)
114-149 LARDMSHLIKEKGGLEGLYYSDRRRRVLDIYLEKEE (SEQ ID NO: 159)
115-149 ARDMSHLIKEKGGLEGLYYSDRRRRVLDIYLEKEE (SEQ ID NO: 160)
116-149 RDMSHLIKEKGGLEGLYYSDRRRRVLDIYLEKEE (SEQ ID NO: 161)
117-149 DMSHLIKEKGGLEGLYYSDRRRRVLDIYLEKEE (SEQ ID NO: 162)
118-149 MSHLIKEKGGLEGLYYSDRRRRVLDIYLEKEE (SEQ ID NO: 163)
119-149 SHLIKEKGGLEGLYYSDRRRRVLDIYLEKEE (SEQ ID NO: 164)
120-149 HLIKEKGGLEGLYYSDRRRRVLDIYLEKEE (SEQ ID NO: 165)
121-149 LIKEKGGLEGLYYSDRRRRVLDIYLEKEE (SEQ ID NO: 166)
104-148 RVPLREMTYRLARDMSHLIKEKGGLEGLYYSDRRRRVLDIYLEKE (SEQ ID NO: 167)
105-148 VPLREMTYRLARDMSHLIKEKGGLEGLYYSDRRRRVLDIYLEKE (SEQ ID NO: 168)
106-148 PLREMTYRLARDMSHLIKEKGGLEGLYYSDRRRRVLDIYLEKE (SEQ ID NO: 169)
107-148 LREMTYRLARDMSHLIKEKGGLEGLYYSDRRRRVLDIYLEKE (SEQ ID NO: 170)
108-148 REMTYRLARDMSHLIKEKGGLEGLYYSDRRRRVLDIYLEKE (SEQ ID NO: 171)
109-148 EMTYRLARDMSHLIKEKGGLEGLYYSDRRRRVLDIYLEKE (SEQ ID NO: 172)
110-148 MTYRLARDMSHLIKEKGGLEGLYYSDRRRRVLDIYLEKE (SEQ ID NO: 173)
111-148 TYRLARDMSHLIKEKGGLEGLYYSDRRRRVLDIYLEKE (SEQ ID NO: 174)
112-148 YRLARDMSHLIKEKGGLEGLYYSDRRRRVLDIYLEKE (SEQ ID NO: 175)
113-148 RLARDMSHLIKEKGGLEGLYYSDRRRRVLDIYLEKE (SEQ ID NO: 176)
114-148 LARDMSHLIKEKGGLEGLYYSDRRRRVLDIYLEKE (SEQ ID NO: 177)
115-148 ARDMSHLIKEKGGLEGLYYSDRRRRVLDIYLEKE (SEQ ID NO: 178)
116-148 RDMSHLIKEKGGLEGLYYSDRRRRVLDIYLEKE (SEQ ID NO: 179)
117-148 DMSHLIKEKGGLEGLYYSDRRRRVLDIYLEKE (SEQ ID NO: 180)
118-148 MSHLIKEKGGLEGLYYSDRRRRVLDIYLEKE (SEQ ID NO: 181)
119-148 SHLIKEKGGLEGLYYSDRRRRVLDIYLEKE (SEQ ID NO: 182)
120-148 HLIKEKGGLEGLYYSDRRRRVLDIYLEKE (SEQ ID NO: 183)
121-148 LIKEKGGLEGLYYSDRRRRVLDIYLEKE (SEQ ID NO: 184)
104-147 RVPLREMTYRLARDMSHLIKEKGGLEGLYYSDRRRRVLDIYLEK (SEQ ID NO: 185)
105-147 VPLREMTYRLARDMSHLIKEKGGLEGLYYSDRRRRVLDIYLEK (SEQ ID NO: 186)
106-147 PLREMTYRLARDMSHLIKEKGGLEGLYYSDRRRRVLDIYLEK (SEQ ID NO: 187)
107-147 LREMTYRLARDMSHLIKEKGGLEGLYYSDRRRRVLDIYLEK (SEQ ID NO: 188)
108-147 REMTYRLARDMSHLIKEKGGLEGLYYSDRRRRVLDIYLEK (SEQ ID NO: 189)
109-147 EMTYRLARDMSHLIKEKGGLEGLYYSDRRRRVLDIYLEK (SEQ ID NO: 190)
110-147 MTYRLARDMSHLIKEKGGLEGLYYSDRRRRVLDIYLEK (SEQ ID NO: 191)
111-147 TYRLARDMSHLIKEKGGLEGLYYSDRRRRVLDIYLEK (SEQ ID NO: 192)
112-147 YRLARDMSHLIKEKGGLEGLYYSDRRRRVLDIYLEK (SEQ ID NO: 193)
113-147 RLARDMSHLIKEKGGLEGLYYSDRRRRVLDIYLEK (SEQ ID NO: 194)
114-147 LARDMSHLIKEKGGLEGLYYSDRRRRVLDIYLEK (SEQ ID NO: 195)
115-147 ARDMSHLIKEKGGLEGLYYSDRRRRVLDIYLEK (SEQ ID NO: 196)
116-147 RDMSHLIKEKGGLEGLYYSDRRRRVLDIYLEK (SEQ ID NO: 197)
117-147 DMSHLIKEKGGLEGLYYSDRRRRVLDIYLEK (SEQ ID NO: 198)
118-147 MSHLIKEKGGLEGLYYSDRRRRVLDIYLEK (SEQ ID NO: 199)
119-147 SHLIKEKGGLEGLYYSDRRRRVLDIYLEK (SEQ ID NO: 200)
120-147 HLIKEKGGLEGLYYSDRRRRVLDIYLEK (SEQ ID NO: 201)
121-147 LIKEKGGLEGLYYSDRRRRVLDIYLEK (SEQ ID NO: 202)
104-146 RVPLREMTYRLARDMSHLIKEKGGLEGLYYSDRRRRVLDIYLE (SEQ ID NO: 203)
105-146 VPLREMTYRLARDMSHLIKEKGGLEGLYYSDRRRRVLDIYLE (SEQ ID NO: 204)
106-146 PLREMTYRLARDMSHLIKEKGGLEGLYYSDRRRRVLDIYLE (SEQ ID NO: 205)
107-146 LREMTYRLARDMSHLIKEKGGLEGLYYSDRRRRVLDIYLE (SEQ ID NO: 206)
108-146 REMTYRLARDMSHLIKEKGGLEGLYYSDRRRRVLDIYLE (SEQ ID NO: 207)
109-146 EMTYRLARDMSHLIKEKGGLEGLYYSDRRRRVLDIYLE (SEQ ID NO: 208)
110-146 MTYRLARDMSHLIKEKGGLEGLYYSDRRRRVLDIYLE (SEQ ID NO: 209)
111-146 TYRLARDMSHLIKEKGGLEGLYYSDRRRRVLDIYLE (SEQ ID NO: 210)
112-146 YRLARDMSHLIKEKGGLEGLYYSDRRRRVLDIYLE (SEQ ID NO: 211)
113-146 RLARDMSHLIKEKGGLEGLYYSDRRRRVLDIYLE (SEQ ID NO: 212)
114-146 LARDMSHLIKEKGGLEGLYYSDRRRRVLDIYLE (SEQ ID NO: 213)
115-146 ARDMSHLIKEKGGLEGLYYSDRRRRVLDIYLE (SEQ ID NO: 214)
116-146 RDMSHLIKEKGGLEGLYYSDRRRRVLDIYLE (SEQ ID NO: 215)
117-146 DMSHLIKEKGGLEGLYYSDRRRRVLDIYLE (SEQ ID NO: 216)
118-146 MSHLIKEKGGLEGLYYSDRRRRVLDIYLE (SEQ ID NO: 217)
119-146 SHLIKEKGGLEGLYYSDRRRRVLDIYLE (SEQ ID NO: 218)
120-146 HLIKEKGGLEGLYYSDRRRRVLDIYLE (SEQ ID NO: 219)
121-146 LIKEKGGLEGLYYSDRRRRVLDIYLE (SEQ ID NO: 220)
104-145 RVPLREMTYRLARDMSHLIKEKGGLEGLYYSDRRRRVLDIYL (SEQ ID NO: 221)
105-145 VPLREMTYRLARDMSHLIKEKGGLEGLYYSDRRRRVLDIYL (SEQ ID NO: 222)
106-145 PLREMTYRLARDMSHLIKEKGGLEGLYYSDRRRRVLDIYL (SEQ ID NO: 223)
107-145 LREMTYRLARDMSHLIKEKGGLEGLYYSDRRRRVLDIYL (SEQ ID NO: 224)
108-145 REMTYRLARDMSHLIKEKGGLEGLYYSDRRRRVLDIYL (SEQ ID NO: 225)
109-145 EMTYRLARDMSHLIKEKGGLEGLYYSDRRRRVLDIYL (SEQ ID NO: 226)
110-145 MTYRLARDMSHLIKEKGGLEGLYYSDRRRRVLDIYL (SEQ ID NO: 227)
111-145 TYRLARDMSHLIKEKGGLEGLYYSDRRRRVLDIYL (SEQ ID NO: 228)
112-145 YRLARDMSHLIKEKGGLEGLYYSDRRRRVLDIYL (SEQ ID NO: 229)
113-145 RLARDMSHLIKEKGGLEGLYYSDRRRRVLDIYL (SEQ ID NO: 230)
114-145 LARDMSHLIKEKGGLEGLYYSDRRRRVLDIYL (SEQ ID NO: 231)
115-145 ARDMSHLIKEKGGLEGLYYSDRRRRVLDIYL (SEQ ID NO: 232)
116-145 RDMSHLIKEKGGLEGLYYSDRRRRVLDIYL (SEQ ID NO: 233)
117-145 DMSHLIKEKGGLEGLYYSDRRRRVLDIYL (SEQ ID NO: 234)
118-145 MSHLIKEKGGLEGLYYSDRRRRVLDIYL (SEQ ID NO: 235)
119-145 SHLIKEKGGLEGLYYSDRRRRVLDIYL (SEQ ID NO: 236)
120-145 HLIKEKGGLEGLYYSDRRRRVLDIYL (SEQ ID NO: 237)
121-145 LIKEKGGLEGLYYSDRRRRVLDIYL (SEQ ID NO: 238)
104-144 RVPLREMTYRLARDMSHLIKEKGGLEGLYYSDRRRRVLDIY (SEQ ID NO: 239)
105-144 VPLREMTYRLARDMSHLIKEKGGLEGLYYSDRRRRVLDIY (SEQ ID NO: 240)
106-144 PLREMTYRLARDMSHLIKEKGGLEGLYYSDRRRRVLDIY (SEQ ID NO: 241)
107-144 LREMTYRLARDMSHLIKEKGGLEGLYYSDRRRRVLDIY (SEQ ID NO: 242)
108-144 REMTYRLARDMSHLIKEKGGLEGLYYSDRRRRVLDIY (SEQ ID NO: 243)
109-144 EMTYRLARDMSHLIKEKGGLEGLYYSDRRRRVLDIY (SEQ ID NO: 244)
110-144 MTYRLARDMSHLIKEKGGLEGLYYSDRRRRVLDIY (SEQ ID NO: 245)
111-144 TYRLARDMSHLIKEKGGLEGLYYSDRRRRVLDIY (SEQ ID NO: 246)
112-144 YRLARDMSHLIKEKGGLEGLYYSDRRRRVLDIY (SEQ ID NO: 247)
113-144 RLARDMSHLIKEKGGLEGLYYSDRRRRVLDIY (SEQ ID NO: 248)
114-144 LARDMSHLIKEKGGLEGLYYSDRRRRVLDIY (SEQ ID NO: 249)
115-144 ARDMSHLIKEKGGLEGLYYSDRRRRVLDIY (SEQ ID NO: 250)
116-144 RDMSHLIKEKGGLEGLYYSDRRRRVLDIY (SEQ ID NO: 251)
117-144 DMSHLIKEKGGLEGLYYSDRRRRVLDIY (SEQ ID NO: 252)
118-144 MSHLIKEKGGLEGLYYSDRRRRVLDIY (SEQ ID NO: 253)
119-144 SHLIKEKGGLEGLYYSDRRRRVLDIY (SEQ ID NO: 254)
120-144 HLIKEKGGLEGLYYSDRRRRVLDIY (SEQ ID NO: 255)
121-144 LIKEKGGLEGLYYSDRRRRVLDIY (SEQ ID NO: 256)
104-143 RVPLREMTYRLARDMSHLIKEKGGLEGLYYSDRRRRVLDI (SEQ ID NO: 257)
105-143 VPLREMTYRLARDMSHLIKEKGGLEGLYYSDRRRRVLDI (SEQ ID NO: 258)
106-143 PLREMTYRLARDMSHLIKEKGGLEGLYYSDRRRRVLDI (SEQ ID NO: 259)
107-143 LREMTYRLARDMSHLIKEKGGLEGLYYSDRRRRVLDI (SEQ ID NO: 260)
108-143 REMTYRLARDMSHLIKEKGGLEGLYYSDRRRRVLDI (SEQ ID NO: 261)
109-143 EMTYRLARDMSHLIKEKGGLEGLYYSDRRRRVLDI (SEQ ID NO: 262)
110-143 MTYRLARDMSHLIKEKGGLEGLYYSDRRRRVLDI (SEQ ID NO: 263)
111-143 TYRLARDMSHLIKEKGGLEGLYYSDRRRRVLDI (SEQ ID NO: 264)
112-143 YRLARDMSHLIKEKGGLEGLYYSDRRRRVLDI (SEQ ID NO: 265)
113-143 RLARDMSHLIKEKGGLEGLYYSDRRRRVLDI (SEQ ID NO: 266)
114-143 LARDMSHLIKEKGGLEGLYYSDRRRRVLDI (SEQ ID NO: 267)
115-143 ARDMSHLIKEKGGLEGLYYSDRRRRVLDI (SEQ ID NO: 268)
116-143 RDMSHLIKEKGGLEGLYYSDRRRRVLDI (SEQ ID NO: 269)
117-143 DMSHLIKEKGGLEGLYYSDRRRRVLDI (SEQ ID NO: 270)
118-143 MSHLIKEKGGLEGLYYSDRRRRVLDI (SEQ ID NO: 271)
119-143 SHLIKEKGGLEGLYYSDRRRRVLDI (SEQ ID NO: 272)
120-143 HLIKEKGGLEGLYYSDRRRRVLDI (SEQ ID NO: 273)
121-143 LIKEKGGLEGLYYSDRRRRVLDI (SEQ ID NO: 274)
or of homologous peptide sequences presenting at least 80% sequence identity, preferably 90% identity, with said HIV-2 Nef fragments.

The present invention is also illustrated by new proteins or polypeptides comprising said HIV-2 Nef fragments or homologous peptide sequences, provided that if present, the sequences adjacent to the respective N-terminal end and/or C-terminal end of the HIV-2 Nef fragments in said protein or polypeptide are different from the sequences adjacent to the respective N-terminal end and/or C-terminal end of the HIV-2 Nef fragments in the Nef proteins from which they derive.

In another particularly preferred illustrated by the above defined new protein or polypeptide, the immunosuppressive domain is mutated by deletion, substitution and/or insertion of at least one amino acid, and in particular the amino acid homologous to the amino acid at position 93 of SEQ ID NO: 1 is replaced by any amino acid different from E, in particular by W, F, M, Y, R, H or K, more particularly by R, H, or K, and preferably by R. Advantageously, such a new protein or polypeptide is devoid of immunosuppressive activity.

In yet another particularly preferred illustration of the above defined new protein or polypeptide, the immunosuppressive domain of a HIV-1 Nef protein is mutated by the substitution of the amino acid at position 93 of the sequence of said HIV-1 Nef protein by any amino acid different from E, in particular by W, F, M, Y, R, H or K, more particularly by R, H, or K, and preferably by R.

The present invention is also illustrated by a nucleic acid, characterized in that it codes for a new protein or polypeptide as defined above.

The present invention is also illustrated by peptidomimetics of the new proteins or polypeptides as defined above.

The present invention is also illustrated by antibodies or fragments thereof, scFv polypeptides, aptamers, or binding peptides, directed against a sequence ranging from the amino acid at position 80 to the amino acid at position 150 of a Nef protein, and in particular:
- against a sequence ranging from the amino acid at position 80 to the amino acid at position 120, more particularly from the amino acid at position 90 to the amino acid at position 120, of the sequence of a HIV-1 Nef protein
- against a sequence ranging from the amino acid at position 104 to the amino acid at position 150 of the sequence of a HIV-2 Nef protein.

The present invention is also illustrated by antibodies or fragments thereof, scFv polypeptides, aptamers, or binding peptides, directed against the new proteins or polypeptides as defined above, provided that said antibodies or fragments thereof, scFv polypeptides, or aptamers do not bind to Nef proteins wherein the immunosuppressive domains corresponds to that of said new proteins or polypeptides as defined above.

As intended herein the above defined antibodies or fragments thereof, scFv polypeptides, aptamers, or binding peptides, are specific for the new proteins or polypeptides according to the invention (*i.e.* proteins or polypeptides comprising the immunosuppressive domain of the Nef protein according to the invention). In particular, these antibodies or fragments thereof, scFv polypeptides, or aptamers do not bind to the immunosuppressive domain of the Nef protein in its natural setting, that is, these antibodies or fragments thereof, scFv polypeptides, or aptamers do not bind to natural Nef proteins.

The present invention is also illustrated by a pharmaceutical or vaccine composition, comprising as active substance an above defined new protein or polypeptide, or an above defined nucleic acid encoding said new protein or polypeptide, in association with a pharmaceutically acceptable carrier.

The present invention is also illustrated by the use of a protein or a polypeptide comprising or being constituted of a Nef protein or fragments thereof, wherein said protein or polypeptide presents an immunosuppressive activity, for the manufacture of a medicament intended for the prevention or the treatment of pathologies requiring an inhibition of the immune system, such as allergies, autoimmune diseases or graft rejections.

In an advantageous illustration, the invention relates to the above mentioned use of a new protein or polypeptide as defined above, wherein said new protein or polypeptide presents an immunosuppressive property, for the manufacture of a medicament intended for the prevention or the treatment of pathologies requiring an inhibition of the immune system, such as allergies, autoimmune diseases or graft rejections.

The present invention is also illustrated by the use of a protein or a polypeptide comprising or being constituted of a Nef protein or fragments thereof, for screening compounds liable to inhibit the immunosuppressive activity of Nef proteins.

The present invention is also illustrated by the use of a new protein or polypeptide as defined above, for screening compounds liable to inhibit the immunosuppressive activity of Nef proteins.

Advantageously, such compounds which are liable to inhibit the immunosuppressive activity of Nef proteins, can be used as anti-viral agents.

As intended herein the compounds to screen can be of any chemical nature. In particular, the compounds to screen are included in chemical compound libraries.

In a preferred illustration of the above defined use of a new protein or polypeptide as defined above, the sequence of the immunosuppressive domain of the Nef protein corresponds to a non-mutated sequence.

The present invention is also illustrated by compounds liable to inhibit the immunosuppressive activity of Nef proteins. Such compounds may be useful for the manufacture of pharmaceutical compositions, in particular intended for the prevention or the treatment of viral diseases, such as HIV or SIV infections.

The immunosuppressive-inhibitory activity of these compounds can be determined by measuring the immunosuppression index of a given Nef protein in the absence or in the presence of the compounds. A compound will be said to possess an immunosuppressive-inhibitory activity when the immunosuppression index of a given Nef protein in the presence of said compound is decreased with respect to the immunosuppression index of the same Nef protein in the absence of said compound.

The present invention is also illustrated by the use of ligands of the immunosuppressive domain of Nef proteins, such as antibodies or fragments thereof, scFv polypeptides, aptamers, or binding peptides, to screen for compounds liable to inhibit the immunosuppressive activity of Nef proteins.

The present invention is also illustrated by a method to screen for compounds liable to inhibit the immunosuppressive activity of Nef proteins, comprising the following steps:
- contacting a Nef protein, or a fragment thereof comprising the immunosuppressive domain of a Nef protein, or a new protein or polypeptide as defined above, with compounds to screen,
- selecting the compounds which bind to the immunosuppressive domain of the Nef protein, or of the fragment thereof comprising the immunosuppressive domain of said Nef protein, or of the new protein or polypeptide as defined above,
- optionally checking that the selected compounds inhibit the immunosuppressive activity of Nef proteins.

In a preferred illustration of the invention, the above mentioned screening method comprises the following steps:
- contacting a Nef protein, or a fragment thereof comprising the immunosuppressive domain of a Nef protein, or a new protein or polypeptide as defined above, with a compounds to screen and with a ligand of the immunosuppressive domain, such as an antibody, a scFv polypeptide, an aptamer, or a binding peptide,
- selecting the compounds which prevent the binding of the ligand to the Nef protein, or to the Nef fragment, or to a new protein or polypeptide as defined above and which do not bind to said ligand,
- optionally checking that the selected compounds inhibit the immunosuppressive activity of Nef proteins.

The present invention is also illustrated by a screening method for compounds liable to inhibit the immunosuppressive activity of Nef proteins, wherein compounds which bind to a Nef protein or a fragment thereof are selected and it is checked that said selected compounds inhibit the immunosuppressive activity of said Nef protein.

The present invention is also illustrated by the compounds which are selected according to the above defined screening method of the invention.

### DESCRIPTION OF THE FIGURES

**Figure 1**

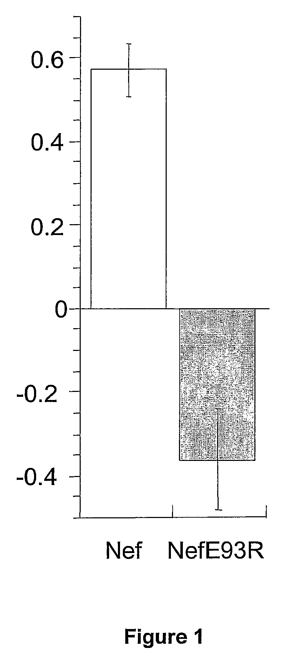

   Figure 1 represents the immunosuppression index (vertical axis) of wild type Nef (white column) and of its E93R mutant (grey column).
**Figure 2**

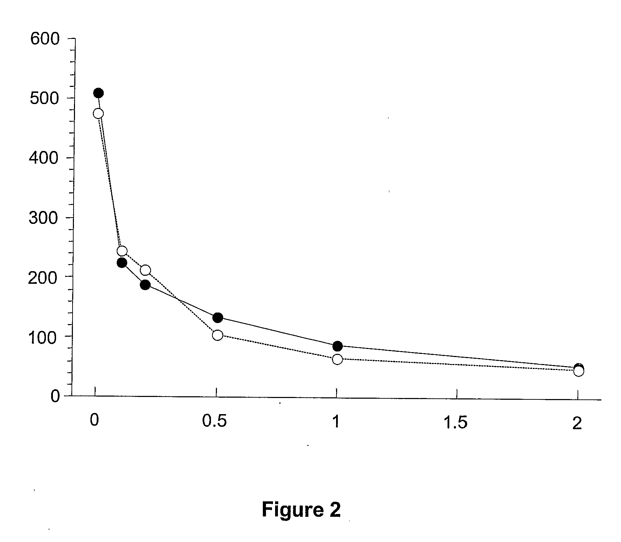

   Figure 2 represents the downregulation of CD4 expression (vertical axis, arbitrary units) by HeLa cells transformed with the indicated amount (horizontal axis, in µg) of wild type Nef expressing vectors (black circles, plain lines) or E93R Nef mutant expressing vectors (white circles, dotted lines).
**Figure 3**

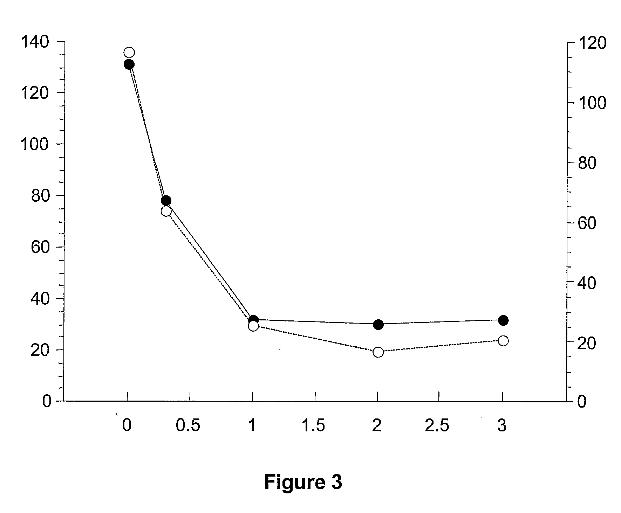

   Figure 3 represents the downregulation of MHC-I expression (vertical axis, arbitrary units, left for Nef, right for the E93R Nef mutant) by 293T cells transformed with the indicated amount (horizontal axis, in µg) of wild type Nef expressing vectors (black circles, plain lines) or E93R Nef mutant expressing vectors (white circles, dotted lines).
**Figure 4**
   Figure 4 represents a sequence alignment generated by the Clustal W software of Nef aminoacid sequences from independent HIV-1, HIV-2 and SIV isolates. The part of the sequences of the Nef proteins comprising the immunosuppressive domain is boxed. The amino acids corresponding or homologous to E93 of SEQ ID NO: 1 (NEF_HIVB1) are in bold. The stars represent positions for which amino acids are conserved; single points, positions for which amino acids are substantially conserved; and double points, positions for which amino acids possess similar physicochemical properties.
**Figure 5**

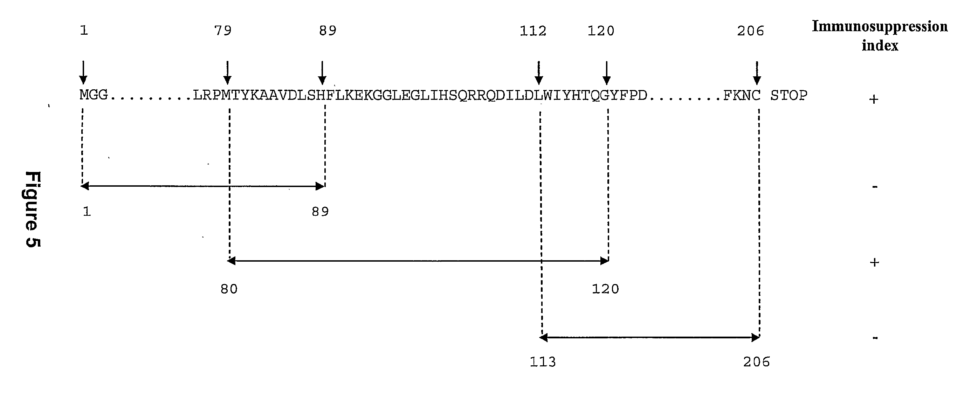

   Figure 5 represents the immunosuppression index of HIV-1 strain LAI Nef and of three of its fragments (1-89, 80-120 and 113-206). The partial sequence of HIV-1 strain LAI is presented on top of the figure with the position of several amino acids as well as the positions of the fragments. The presence (+) or absence (-) of an immunosuppressive activity for wild type Nef and for each fragment is indicated on the right (immunosuppression index).
**Figure 6A and Figure 6B**
   Figure 6A represents the immunosuppression index (vertical axis) of HIV-1 strain A1 Nef (left column) and for HIV-2 strain ST Nef (right column).
   Figure 6B represents the immunosuppression index (vertical axis) of SIV strain mac239 Nef (left column) and of the corresponding E125R mutant (right column).

### EXAMPLES

### EXAMPLE 1

### Cloning of the genes encoding wild type Nef and the E93R Nef mutant

HIV-1 strain LAI Nef was retrieved from pCDNA3-Nef (Peden K., Emerman M. and Montagnier L. 1991, Virology 185(2):661-672) (gift from O. Schwartz, Institut Pasteur, France) by PCR with high-fidelity Pfx Platinum polymerase (Invitrogen) and the following primers:
5'-ATACATGGCCCAGCCGGCCGGTGGCAAGTGGTCAAAAAGTAGT-3' (SEQ ID NO: 4) and
5'-ATACATGGATCCACGCGTTCAGCAGTTCTTGAAGTACTCCGG-3' (SEQ ID NO: 5).

The amplification product was digested with *Sfi*I and *Bam*HI and ligated in the pSecTag2A vector (Invitrogen) opened with the same enzymes. Nef preceded with the export signal sequence of the vector was then amplified with the following primers:
5'-ATACATACCGGTATGGAGACAGACACACTCCTGCTATG-3' (SEQ ID NO: 6), and
5'-ATACATGGATCCACGCGTTCAGCAGTTCTTGAAGTACTCCGG-3' (SEQ ID NO: 7).

The product was digested with *Age*I and *Mlu*I and ligated into the retroviral vector pDFG-MoTMtag (Mangeney & Heidmann (1998) Proc. Natl. Acad. Sci. U.S.A. 95:14920-5) digested with the same enzymes to obtain pDFG-expNef (SEQ ID NO: 16), expressing the exported version of HIV-Nef (SEQ ID NO: 17).

The mutation E93R was then introduced in pDFG-expNef by ligation of the three following fragments to yield pDFG-expNefE93R (SEQ ID NO: 18), expressing the exported version of E93R Nef (SEQ ID NO: 19):
1) the *Age*I*-Mlu*I fragment of the vector;
2) a PCR product obtained with primers
   5'-ATACATACCGGTATGGAGACAGACACACTC-3' (SEQ ID NO: 8) and
   5'-ATACATCTTAAGAAAGTGGCTAAGATCTACAGCTGCC-3' (SEQ ID NO: 9)
   and digested with *Afl*II;
3) a PCR product obtained with primers
   5'-ATACATCTTAAGCGAAAGGGGGGACTGGAAGGG-3' (SEQ ID NO: 10) and
   5'-ATACATACGCGTTCAGCAGTTCTTGAA-3' (SEQ ID NO: 11)
   digested with *Afl*II and *Mlu*I*.*

Nef and its mutant E93R were then retrieved from the pDFG-expNef vectors with the following primers:
5'-ATACATGTCGACCCAACTAGAACCATGGGTGGCAAGTGGTCAAAAAGTAG-3'(SEQ ID NO: 12), and
5'-ATACATACGCGTTCAGCAGTTCTTGAA-3' (SEQ ID NO: 13).

The product was digested with *Sal*I and *Mlu*I and ligated into phCMV-envT (Blaise et al. (2003) Proc. Natl. Acad. Sci. 100:13013-8) digested with *Xho*I and *Mlu*I*,* to yield respectively phCMV-Nef (SEQ ID NO: 20), expressing Nef (SEQ ID NO: 1), and phCMV-NefE93R (SEQ ID NO: 21), expressing E93R Nef (SEQ ID NO: 2).

Similarly, the sequences coding for Nef and the E93R Nef mutant with the export signal sequence were respectively extracted from pDFG-expNef and pDFG-expNefE93R and inserted into phCMV to yield phCMV-expNef (SEQ ID NO: 14) and phCMV-expNefE93R (SEQ ID NO: 15).

### EXAMPLE 2

### Determination of the immunosuppression index of wild type Nef and of the E93R Nef mutant

The immunosuppression index of Nef and of its E93R mutant were measured following the general procedure described in Mangeney & Heidmann (1998) Proc. Natl. Acad. Sci. U.S.A. 95:14920-5 and Mangeney et al. (2001) J. Gen. Virol. 82:2515-8.

Briefly, MCA205 cells were stably transformed by plasmids pDFG-expNef and pDFG-expNefE93R, or optionally by plasmids phCMV-expNef and phCMV-expNefE93R, respectively. 10⁶ MCA cells expressing either wild type Nef, the E93R Nef mutant or no exogenous protein were then injected into Balb/c mice and tumor areas were measured every other day. After 7 to 8 days the immunosuppression index was determined.

The immunosuppression index of a protein was calculated as (Aₚᵣₒₜₑᵢₙ-Aₙₒₙₑ)/Aₙₒₙₑ, where Aₚᵣₒₜₑᵢₙ and Aₙₒₙₑ are the peak tumor areas obtained with MCA cells expressing the proteins of interest (*i. e.* Nef or the E93R Nef mutant) and no exogenous protein, respectively.

The results are presented in **Figure 1****.** As can be seen, the immunosuppression index of Nef is approximately 0.6, which indicates that the size of the Nef expressing tumors is 1.6 times bigger than normal tumors, thus demonstrating that Nef is an immunosuppressive protein which inhibits the anti-tumor immune response. In contrast, the immunosuppressive index of the E93R Nef mutant is negative, thus demonstrating that this mutant has no immunosuppressive activity, and that tumors expressing this Nef mutant are more easily recognized and eliminated by the immune system than normal tumors.

### EXAMPLE 3

### Down-regulation of CD4 expression by Nef and its E93R mutant

HeLa cells were cotransfected with 1 µg of CMV-CD4 (Janvier et al. (2001) J. Virol. 75:3971-6) and the indicated amount of phCMV-Nef or phCMV-NefE93R. CD4 expression was then measured by FACS using a PC5-coupled anti-human CD4 antibody (IM2636, Immunotech). The results presented in **Figure 2** indicate that wild type Nef and the E93R Nef mutant downregulate CD4 expression to a similar extent. This implies that the structure of the E93R Nef mutant is unchanged with respect to that of wild type Nef.

### EXAMPLE 4

### Down-regulation of MHC-I expression by Nef and its E93R mutant

293T cells were cotransfected with 1 µg of CMV-HLA A2 (Le Gall et al. (2000) J. Virol. 74:9256-66) and the indicated amount of phCMV-Nef or phCMV-NefE93R. MHC-I expression was measured by FACS with PE-coupled anti human MHC-I antibody W6/32 (eBioscience).

The results presented in **Figure 3** indicate that wild type Nef and the E93R Nef mutant downregulate MHC-I expression to a similar extent. This also implies that the structure of the E93R Nef mutant is unchanged with respect to that of wild type Nef.

### EXAMPLE 5

### Determination of the localization of the immunosuppressive domain of wild type Nef

Based on the three-dimensional structure of Nef, three fragments of the Nef protein of HIV-1 strain LAI have been designed in order to determine the localization of the immunosuppressive domain of Nef:
1) a fragment extending from residue number 1 to residue number 89
2) a fragment extending from residue number 80 to residue number 120
3) a fragment extending from residue number 113 to residue number 206

Fragment 2 comprises the putative immunosuppressive domain, while fragments number 1 and 3 do not comprise this domain. Fragment 2 extents both ways from the putative immunosuppressive domain of Nef in order to include the whole two alpha-helical domains containing the putative immunosuppressive domain of Nef, according to the known core structure of the HIV-1 Nef protein (PBD entry 1EFN).

The DNAs coding for those fragments were generated by PCR using the Nef gene cloned into the pCDNA3 vector (Peden K., Emerman M. and Montagnier L. 1991, Virology 185(2):661-672) as a template and the following primers pairs:
- for fragment 1:
   - atacatggcccagccggccggtggcaagtggtcaaaaagtagt (SEQ ID NO: 22)
   - atacatacgcgttcagtggctaagatctacagctgcctt (SEQ ID NO: 23)
- for fragment 2:
   - atacatggcccagccggccacttacaaggcagctgtagatcttagc (SEQ ID NO: 24)
   - atacatacgtcgttcagccttgtgtgtggtagatccac (SEQ ID NO: 25)
- for fragment 3:
   - atacatggcccagccggccgatatccttgatctgtggatctaccac (SEQ ID NO: 26)
   - atacataacgcgttcagcagttcttgaagtactccgg (SEQ ID NO: 27)

The PCR products were digested with *Sfi*I and *Mlu*I and cloned into pDFG-expNef opened with the same enzymes, resulting in the genetic fusion of the fragments with the extracellular exportation signal peptide of the human Igκ light chain. Thus, the obtained constructs expressed extracellularly localized fragments of HIV-1 Nef. They were used in an *in vivo* immunosuppression assay as described in **Example 2.** A positive index (+) indicates that the considered fragment has *in vivo* immunosuppressive properties, whereas an index inferior or equal to zero (-) indicates that the considered fragment is devoid of such properties.

As illustrated in **Figure 5**, the fragment extending from residue 90 to residue 120 of HIV-1 Nef protein displays an immunosuppressive property *in vivo.* This fragment thus comprises the immunosuppressive domain of Nef. Fragments 1 and 3 indicate that this immunosuppressive domain could be further reduced to amino acids 90 to 112.

### EXAMPLE 6

### Determination of the immunosuppression index of additional wild type Nef proteins

The immunosuppression index of Nef was determined as described in **Example 2** for HIV-1 strain A1 Nef (SEQ ID NO: 28) and for HIV-2 strain ST Nef (SEQ ID NO: 29).

As expected, the results indicate that these Nef proteins are also immunosuppressive (**Figure 6A**)**.**

### EXAMPLE 7

### Determination of the immunosuppression index of a SIV Nef and of its E125R mutant

The immunosuppression index was determined for the SIV strain mac239 wild type Nef (SEQ ID NO: 30) and its E125R mutant (SEQ ID NO: 31) as described in **Example 2.** The E125R mutation in SIV mac239 Nef is homologous to the above defined E93R mutation of HIV-1 LAI and was introduced following a procedure similar to that described in **Example 1.**

The results indicate that the SIV Nef protein possesses an immunosuppressive activity while the E→R mutant is completely devoid of such an activity (**Figure 6B**).

## Claims

1. A pharmaceutical or vaccine composition, comprising as active substance, a mutated Nef protein obtained by the substitution of one amino acid within the immunosuppressive domain of a Nef protein, said Nef protein being constituted by one of the amino acid sequences of the group consisting of SEQ ID NO: 1, SEQ ID NO: 275, SEQ ID NO: 276, SEQ ID NO: 277, SEQ ID NO: 278, SEQ ID NO: 279, SEQ ID NO: 280, SEQ ID NO: 281, SEQ ID: 282 SEQ ID NO: 283, SEQ ID NO: 284, SEQ ID NO
: 285, SEQ ID NO: 286, SEQ ID NO: 287, SEQ ID NO: 288, SEQ ID NO: 289, SEQ ID NO: 290, SEQ ID NO: 291, SEQ ID NO: 292 and SEQ IDNO: 293, as depicted in Figure 4
said mutated Nef protein having substantially no immunosuppressive activity, wherein
- the amino acid at position 93 of one of SEQ ID NO: 1, SEQ ID NO: 275, SEQ ID NO: 277, SEQ ID NO: 278, SEQ ID NO: 279, SEQ ID NO: 282or
- the amino acid at position 94 of SEQ ID NO: 276, or
- the amino acid at position 96 of SEQ ID NO: 281, or
- the amino acid at position 103 of SEQ ID NO: 280, or
- the amino acid at position 109 of SEQ ID NO: 291, or
- the amino acid at position 112 of SEQ ID NO: 292, or
- the amino acid at position 114 of SEQ ID NO: 293, or
- the amino acid at position 124 of SEQ ID NO: 287, or
- the amino acid at position 125 of one of SEQ ID NO: 283, SEQ ID NO: 284, SEQ ID NO : 285, SEQ ID NO: 286, SEQ ID NO: 288, SEQ ID NO: 289, and SEQ ID NO: 290,
is replaced by R, H or K,
in association with a pharmaceutically acceptable carrier.

2. A pharmaceutical or vaccine composition according to claim 1, wherein
at least 60% of the CD4 and/or MHC-I downregulation functions are preserved in the mutated Nef protein with respect to the non mutated Nef protein.

3. A vaccine composition according to any of claims 1 or 2, wherein
- the amino acid at position 93 of one of SEQ ID NO: 1, SEQ ID NO: 275, SEQ ID NO: 277, SEQ ID NO: 278, SEQ ID NO: 279, SEQ ID NO: 282or
- the amino acid at position 94 of SEQ ID NO: 276, or
- the amino acid at position 96 of SEQ ID NO: 281, or
- the amino acid at position 103 of SEQ ID NO: 280, or
- the amino acid at position 109 of SEQ ID NO: 291, or
- the amino acid at position 112 of SEQ ID NO: 292, or
- the amino acid at position 114 of SEQ ID NO: 293, or
- the amino acid at position 124 of SEQ ID NO: 287, or
- the amino acid at position 125 of one of SEQ ID NO: 283, SEQ ID NO: 284, SEQ ID NO : 285, SEQ ID NO: 286, SEQ ID NO: 288, SEQ ID NO: 289, and SEQ ID NO: 290,
is replaced by R.

4. A pharmaceutical or vaccine composition according to any of claims 1 to 3, comprising as active substance a mutated Nef protein corresponding to SEQ ID NO: 2.

5. A pharmaceutical or vaccine composition, comprising as active substance a nucleic acid encoding a mutated Nef protein as defined in any of claims 1 to 4.

6. The use of a protein as defined in any of claims 1 to 4, or of a nucleic acid as defined in claim 5, for the manufacture of a medicament or a vaccine intended for the prevention and/or the treatment of viral diseases, such as HIV infections.

7. A protein represented by SEQ ID NO: 2 or SEQ ID NO: 31.

8. A nucleic acid coding for a protein represented by SEQ ID NO: 2 or SEQ ID NO: 31.

## Patentansprüche

1. Pharmazeutische oder Impfstoffzusammensetzung, umfassend als Wirkstoff ein mutiertes Nef-Protein, das durch Substitution einer Aminosäure innerhalb der immunsuppressiven Domäne eines Nef-Proteins erhalten wird, wobei das Nef-Protein aus einer der Aminosäuresequenzen der Gruppe bestehend aus SEQ ID NR: 1, SEQ ID NR: 275, SEQ ID NR: 276, SEQ ID NR: 277, SEQ ID NR: 278, SEQ ID NR: 279, SEQ ID NR: 280, SEQ ID NR: 281, SEQ ID NR: 282, SEQ ID NR: 283, SEQ ID NR: 284, SEQ ID NR: 285, SEQ ID NR: 286, SEQ ID NR: 287, SEQ ID NR: 288, SEQ ID NR: 289, SEQ ID NR: 290, SEQ ID NR: 291, SEQ ID NR: 292 und SEQ ID NR: 293 besteht, wie in Figur 4 dargestellt, wobei das mutierte Nef-Protein im Wesentlichen keine immunsuppressive Aktivität besitzt, wobei
- die Aminosäure an Position 93 einer von SEQ ID NR: 1, SEQ ID NR: 275, SEQ ID NR: 277, SEQ ID NR: 278, SEQ ID NR: 279, SEQ ID NR: 282 oder
- die Aminosäure an Position 94 von SEQ ID NR: 276 oder
- die Aminosäure an Position 96 von SEQ ID NR: 281 oder
- die Aminosäure an Position 103 von SEQ ID NR: 280 oder
- die Aminosäure an Position 109 von SEQ ID NR: 291 oder
- die Aminosäure an Position 112 von SEQ ID NR: 292 oder
- die Aminosäure an Position 114 von SEQ ID NR: 293 oder
- die Aminosäure an Position 124 von SEQ ID NR: 287 oder
- die Aminosäure an Position 125 einer von SEQ ID NR: 283, SEQ ID NR: 284, SEQ ID NR: 285, SEQ ID NR: 286, SEQ ID NR: 288, SEQ ID NR: 289 und SEQ ID NR: 290
durch R, H oder K ersetzt ist,
in Verbindung mit einem pharmazeutisch annehmbaren Träger.

2. Pharmazeutische oder Impfstoffzusammensetzung nach Anspruch 1, wobei
zumindest 60 % der CD4- und/oder MHC-I-Abwärtsregulierungsfunktionen in dem mutierten Nef-Protein in Bezug auf das nicht mutierte Nef-Protein konserviert sind.

3. Impfstoffzusammensetzung nach einem der Ansprüche 1 oder 2, wobei
- die Aminosäure an Position 93 einer von SEQ ID NR: 1, SEQ ID NR: 275, SEQ ID NR: 277, SEQ ID NR: 278, SEQ ID NR: 279, SEQ ID NR: 282 oder
- die Aminosäure an Position 94 von SEQ ID NR: 276 oder
- die Aminosäure an Position 96 von SEQ ID NR: 281 oder
- die Aminosäure an Position 103 von SEQ ID NR: 280 oder
- die Aminosäure an Position 109 von SEQ ID NR: 291 oder
- die Aminosäure an Position 112 von SEQ ID NR: 292 oder
- die Aminosäure an Position 114 von SEQ ID NR: 293 oder
- die Aminosäure an Position 124 von SEQ ID NR: 287 oder
- die Aminosäure an Position 125 einer von SEQ ID NR: 283, SEQ ID NR: 284, SEQ ID NR: 285, SEQ ID NR: 286, SEQ ID NR: 288, SEQ ID NR: 289 und SEQ ID NR: 290
durch R ersetzt ist.

4. Pharmazeutische oder Impfstoffzusammensetzung nach einem der Ansprüche 1 bis 3, umfassend als Wirkstoff ein mutiertes Nef-Protein entsprechend SEQ ID NR: 2.

5. Pharmazeutische oder Impfstoffzusammensetzung, umfassend als Wirkstoff eine Nukleinsäure, die für ein mutiertes Nef-Protein, wie in einem der Ansprüche 1 bis 4 definiert, codiert.

6. Verwendung eines Proteins, wie in einem der Ansprüche 1 bis 4 definiert, oder einer Nukleinsäure, wie in Anspruch 5 definiert, für die Herstellung eines Medikaments oder eines Impfstoffs, das bzw. der zur Vorbeugung und/oder Behandlung von Viruserkrankungen, wie HIV-Infektionen, bestimmt ist.

7. Protein, dargestellt durch SEQ ID NR: 2 oder SEQ ID NR: 31.

8. Nukleinsäure, die für ein Protein codiert, das durch SEQ ID NR: 2 oder SEQ ID NR: 31 dargestellt ist.

## Revendications

1. Une composition pharmaceutique ou vaccinale, comprenant en tant que substance active, une protéine Nef mutée obtenue par la substitution d'un acide aminé dans le domaine immunosuppresseur d'une protéine Nef, ladite protéine Nef étant constituée par une des séquences d'acides aminés du groupe consistant en : SEQ ID NO :1, SEQ ID NO :275, SEQ ID NO :276, SEQ ID NO :277, SEQ ID NO :278, SEQ ID NO :279, SEQ ID NO :280, SEQ ID NO :281, SEQ ID NO : 282, SEQ ID NO :283, SEQ ID NO : 284, SEQ ID NO : 285, SEQ ID NO : 286, SEQ ID NO : 287, SEQ ID NO : 288, SEQ ID NO : 289, SEQ ID NO : 290, SEQ ID NO : 291, SEQ ID NO : 292, SEQ ID NO : 293, comme représenté sur la figure 4
ladite protéine Nef mutée n'ayant essentiellement aucune activité immunosuppressive, dans laquelle
- l'acide aminé en position 93 d'une des SEQ ID NO : 1, SEQ ID NO : 275, SEQ ID NO : 277, SEQ ID NO : 278, SEQ ID NO : 279, SEQ ID NO : 282, ou
- l'acide aminé en position 94 de SEQ ID NO : 276, ou
- l'acide aminé en position 96 de SEQ ID NO : 281, ou
- l'acide aminé en position 103 de SEQ ID NO : 280, ou
- l'acide aminé en position 109 de SEQ ID NO : 291, ou
- l'acide aminé en position 112 de SEQ ID NO : 292, ou
- l'acide aminé en position 114 de SEQ ID NO : 293, ou
- l'acide aminé en position 124 de SEQ ID NO : 287, ou
- l'acide aminé en position 125 d'une des SEQ ID NO : 283, SEQ ID NO : 284, SEQ ID NO : 285, SEQ ID NO : 286, SEQ ID NO : 288, SEQ ID NO : 289, et SEQ ID NO : 290,
est remplacé par R, H ou K,
en association avec un véhicule pharmaceutiquement acceptable.

2. Une composition pharmaceutique ou vaccinale selon la revendication 1, dans laquelle au moins 60% des fonctions de régulation négative de CD4 et/ou MHC-I sont préservés dans la protéine Nef mutée par rapport à la protéine Nef non mutée.

3. Une composition vaccinale selon la revendication 1 ou 2, dans laquelle
- l'acide aminé en position 93 d'une des SEQ ID NO : 1, SEQ ID NO : 275, SEQ ID NO : 277, SEQ ID NO : 278, SEQ ID NO : 279, SEQ ID NO : 282, ou
- l'acide aminé en position 94 de SEQ ID NO : 276, ou
- l'acide aminé en position 96 de SEQ ID NO : 281, ou
- l'acide aminé en position 103 de SEQ ID NO : 280, ou
- l'acide aminé en position 109 de SEQ ID NO : 291, ou
- l'acide aminé en position 112 de SEQ ID NO : 292, ou
- l'acide aminé en position 114 de SEQ ID NO : 293, ou
- l'acide aminé en position 124 de SEQ ID NO : 287, ou
- l'acide aminé en position 125 d'une des SEQ ID NO : 283, SEQ ID NO : 284, SEQ ID NO : 285, SEQ ID NO : 286, SEQ ID NO : 288, SEQ ID NO : 289, et SEQ ID NO : 290,
est remplacé par R.

4. Une composition pharmaceutique ou vaccinale selon l'une des revendications 1 à 3, comprenant en tant que substance active une protéine Nef mutée correspondant à SEQ ID NO : 2.

5. Une composition pharmaceutique ou vaccinale, comprenant en tant que substance active un acide nucléique codant pour une protéine Nef mutée telle que définie à l'une des revendications 1 à 4.

6. Utilisation d'une protéine telle que définie selon l'une quelconque des revendications 1 à 4, ou d'un acide nucléique tel que défini selon la revendication 5, pour la fabrication d'un médicament ou d'un vaccin destiné à la prévention et/ou au traitement de maladies virales, telles que les infections au VIH.

7. Une protéine représentée par SEQ ID NO : 2 ou SEQ ID NO : 31.

8. Un acide nucléique codant une protéine représentée par SEQ ID NO : 2 ou SEQ ID NO : 31.


### SEQUENCE LISTING

<110> INSTITUT GUSTAVE ROUSSY CENTRE NATIONAL DE LA RECHERCHE SCIENTIFIQUE UNIVERSITE PARIS XI
<120> USE OF THE IMMUNOSUPPRESSIVE FUNCTION OF AN ACCESSORY PROTEIN OF THE
   HUMAN OR SIMIAN IMMUNDEFICIENCY VIRUS FOR THE PREPARATION OF A VACCINE
<130> WOB 04 CG IGR NEFI
<160> 274
<170> PatentIn version 3.1
<210> 1
   <211> 206
   <212> PRT
   <213> Human immunodeficiency virus type 1
<400> 1
   Met Gly Gly Lys Trp Ser Lys Ser Ser Val Val Gly Trp Pro Thr Val
   1               5                   10                  15
   Arg Glu Arg Met Arg Arg Ala Glu Pro Ala Ala Asp Gly Val Gly Ala
               20                  25                  30
   Ala Ser Arg Asp Leu Glu Lys His Gly Ala Ile Thr Ser Ser Asn Thr
           35                  40                  45
   Ala Ala Thr Asn Ala Ala Cys Ala Trp Leu Glu Ala Gln Glu Glu Glu
       50                  55                  60
   Glu Val Gly Phe Pro Val Thr Pro Gln Val Pro Leu Arg Pro Met Thr
   65                  70                  75                  80
   Tyr Lys Ala Ala Val Asp Leu Ser His Phe Leu Lys Glu Lys Gly Gly
                   85                  90                  95
   Leu Glu Gly Leu Ile His Ser Gln Arg Arg Gln Asp Ile Leu Asp Leu
               100                 105                 110
   Trp Ile Tyr His Thr Gln Gly Tyr Phe Pro Asp Trp Gln Asn Tyr Thr
           115                 120                 125
   Pro Gly Pro Gly Val Arg Tyr Pro Leu Thr Phe Gly Trp Cys Tyr Lys
       130                 135                 140
   Leu Val Pro Val Glu Pro Asp Lys Val Glu Glu Ala Asn Lys Gly Glu
   145                 150                 155                 160
   Asn Thr Ser Leu Leu His Pro Val Ser Leu His Gly Met Asp Asp Pro
                   165                 170                 175
   Glu Arg Glu Val Leu Glu Trp Arg Phe Asp Ser Arg Leu Ala Phe His
               180                 185                 190
   His Val Ala Arg Glu Leu His Pro Glu Tyr Phe Lys Asn Cys
           195                 200                 205
<210> 2
   <211> 206
   <212> PRT
   <213> Artificial sequence
<220>
   <223> E93R HIV-1 Nef mutant
<400> 2
   Met Gly Gly Lys Trp Ser Lys Ser Ser Val Val Gly Trp Pro Thr Val
   1 5 10 15
   Arg Glu Arg Met Arg Arg Ala Glu Pro Ala Ala Asp Gly Val Gly Ala
   20 25 30
   Ala Ser Arg Asp Leu Glu Lys His Gly Ala Ile Thr Ser Ser Asn Thr
   35 40 45
   Ala Ala Thr Asn Ala Ala Cys Ala Trp Leu Glu Ala Gln Glu Glu Glu
   50 55 60
   Glu Val Gly Phe Pro Val Thr Pro Gln Val Pro Leu Arg Pro Met Thr
   65 70 75 80
   Tyr Lys Ala Ala Val Asp Leu Ser His Phe Leu Lys Arg Lys Gly Gly
   85 90 95
   Leu Glu Gly Leu Ile His Ser Gln Arg Arg Gln Asp Ile Leu Asp Leu
   100 105 110
   Trp Ile Tyr His Thr Gln Gly Tyr Phe Pro Asp Trp Gln Asn Tyr Thr
   115 120 125
   Pro Gly Pro Gly Val Arg Tyr Pro Leu Thr Phe Gly Trp Cys Tyr Lys
   130 135 140
   Leu Val Pro Val Glu Pro Asp Lys Val Glu Glu Ala Asn Lys Gly Glu
   145 150 155 160
   Asn Thr Ser Leu Leu His Pro Val Ser Leu His Gly Met Asp Asp Pro
   165 170 175
   Glu Arg Glu Val Leu Glu Trp Arg Phe Asp Ser Arg Leu Ala Phe His
   180 185 190
   His Val Ala Arg Glu Leu His Pro Glu Tyr Phe Lys Asn Cys
   195 200 205
<210> 3
   <211> 16
   <212> PRT
   <213> Artificial sequence
<220>
   <223> Nef immunosuppressive domain consensus sequence
<220>
   <221> MISC_FEATURE
   <222> (2) .. (2)
   <223> I, L, V, F or R
<220>
   <221> MISC_FEATURE
   <222> (4) .. (4)
   <223> M, L, or F
<220>
   <221> MISC_FEATURE
   <222> (6) .. (6)
   <223> H, D, or F
<220>
   <221> MISC_FEATURE
   <222> (7) .. (7)
   <223> F or L
<220>
   <221> MISC_FEATURE
   <222> (8) .. (8)
   <223> I or L
<220>
   <221> MISC_FEATURE
   <222> (10) .. (10)
   <223> Any amino acid different from E
<220>
   <221> MISC_FEATURE
   <222> (11) .. (11)
   <223> K, Q, or R
<220>
   <221> MISC_FEATURE
   <222> (13) .. (13)
   <223> G or no amino acid
<220>
   <221> MISC_FEATURE
   <222> (15) .. (15)
   <223> E, D, or R
<400> 3
   Ala Xaa Asp Xaa Ser Xaa Xaa Xaa Lys Xaa Xaa Gly Xaa Leu Xaa Gly
   1               5                   10                  15
<210> 4
   <211> 43
   <212> DNA
   <213> Artificial sequence
<220>
   <223> PCR primer
<400> 4
   atacatggcc cagccggccg gtggcaagtg gtcaaaaagt agt 43
<210> 5
   <211> 42
   <212> DNA
   <213> Artificial sequence
<220>
   <223> PCR primer
<400> 5
   atacatggat ccacgcgttc agcagttctt gaagtactcc gg 42
<210> 6
   <211> 38
   <212> DNA
   <213> Artificial sequence
<220>
   <223> PCR primer
<400> 6
   atacataccg gtatggagac agacacactc ctgctatg 38
<210> 7
   <211> 42
   <212> DNA
   <213> Artificial sequence
<220>
   <223> PCR primer
<400> 7
   atacatggat ccacgcgttc agcagttctt gaagtactcc gg 42
<210> 8
   <211> 30
   <212> DNA
   <213> Artificial sequence
<220>
   <223> PCR primer
<400> 8
   atacataccg gtatggagac agacacactc 30
<210> 9
   <211> 37
   <212> DNA
   <213> Artificial sequence
<220>
   <223> PCR primer
<400> 9
   atacatctta agaaagtggc taagatctac agctgcc 37
<210> 10
   <211> 33
   <212> DNA
   <213> Artificial sequence
<220>
   <223> PCR primer
<400> 10
   atacatctta agcgaaaggg gggactggaa ggg 33
<210> 11
   <211> 27
   <212> DNA
   <213> Artificial seuqence
<220>
   <223> PCR primer
<400> 11
   atacatacgc gttcagcagt tcttgaa 27
<210> 12
   <211> 50
   <212> DNA
   <213> Artificial sequence
<220>
   <223> PCR primer
<400> 12
   atacatgtcg acccaactag aaccatgggt ggcaagtggt caaaaagtag 50
<210> 13
   <211> 27
   <212> DNA
   <213> Artificial sequence
<220>
   <223> PCR primer
<400> 13
   atacatacgc gttcagcagt tcttgaa 27
<210> 14
   <211> 5361
   <212> DNA
   <213> Artificial sequence
<220>
   <223> Nef expressing vector
<400> 14
   gcggccgctc tagagagctt ggcccattgc atacgttgta tccatatcat aatatgtaca      60
   tttatattgg ctcatgtcca acattaccgc catgttgaca ttgattattg actagttatt     120
   aatagtaatc aattacgggg tcattagttc atagcccata tatggagttc cgcgttacat     180
   aacttacggt aaatggcccg cctggctgac cgcccaacga cccccgccca ttgacgtcaa     240
   taatgacgta tgttcccata gtaacgccaa tagggacttt ccattgacgt caatgggtgg     300
   agtatttacg gtaaactgcc cacttggcag tacatcaagt gtatcatatg ccaagtacgc     360
   cccctattga cgtcaatgac ggtaaatggc ccgcctggca ttatgcccag tacatgacct     420
   tatgggactt tcctacttgg cagtacatct acgtattagt catcgctatt accatggtga     480
   tgcggttttg gcagtacatc aatgggcgtg gatagcggtt tgactcacgg ggatttccaa     540
   gtctccaccc cattgacgtc aatgggagtt tgttttggca ccaaaatcaa cgggactttc     600
   caaaatgtcg taacaactcc gccccattga cgcaaatggg cggtaggcgt gtacggtggg     660
   aggtctatat aagcagagct cgtttagtga accgtcagat cgcctggaga cgccatccac     720
   gctgttttga cctccataga agacaccggg accgatccag cctccggtcg accgatcctg     780
   agaacttcag ggtgagtttg gggacccttg attgttcttt ctttttcgct attgtaaaat     840
   tcatgttata tggagggggc aaagttttca gggtgttgtt tagaatggga agatgtccct     900
   tgtatcacca tggaccctca tgataatttt gtttctttca ctttctactc tgttgacaac     960
   cattgtctcc tcttattttc ttttcatttt ctgtaacttt ttcgttaaac tttagcttgc    1020
   atttgtaacg aatttttaaa ttcacttttg tttatttgtc agattgtaag tactttctct    1080
   aatcactttt ttttcaaggc aatcagggta tattatattg tacttcagca cagttttaga    1140
   gaacaattgt tataattaaa tgataaggta gaatatttct gcatataaat tctggctggc    1200
   gtggaaatat tcttattggt agaaacaact acaccctggt catcatcctg cctttctctt    1260
   tatggttaca atgatataca ctgtttgaga tgaggataaa atactctgag tccaaaccgg    1320
   gcccctctgc taaccatgtt catgccttct tctctttcct acagctcctg ggcaacgtgc    1380
   tggttgttgt gctgtctcat cattttggca aagaattcct cgacggatcc ctcgacccaa    1440
   ctagaaccat ggggtgggag cagcatctcg agacctggaa aaacatggag caatcacaag    1500
   tagcaataca gcagctacca atgctgcttg tgcctggcta gaagcacaag aggaggagga    1560
   ggtgggtttt ccagtcacac ctcaggtacc tttaagacca atgacttaca aggcagctgt    1620
   agatcttagc cactttctta aggaaaaggg gggactggaa gggctaattc actcccaacg    1680
   aagacaagat atccttgatc tgtggatcta ccacacacaa ggctacttcc ctgattggca    1740
   gaactacaca ccagggccag gggtcagata tccactgacc tttggatggt gctacaagct 1800
   agtaccagtt gagccagata aggtagaaga ggccaataaa ggagagaaca ccagcttgtt 1860
   acaccctgtg agcctgcatg gaatggatga ccctgagaga gaagtgttag agtggaggtt 1920
   tgacagccgc ctagcatttc atcacgtggc ccgagagctg catccggagt acttcaagaa 1980
   ctgctgaacg cgtcaggtgc aggctgccta tcagaaggtg gtggctggtg tggccaatgc 2040
   cctggctcac aaataccact gagatctttt tccctctgcc aaaaattatg gggacatcat 2100
   gaagcccctt gagcatctga cttctggcta ataaaggaaa tttattttca ttgcaatagt 2160
   gtgttggaat tttttgtgtc tctcactcgg aaggacatat gggagggcaa atcatttaaa 2220
   acatcagaat gagtatttgg tttagagttt ggcaacatat gcccatatgc tggctgccat 2280
   gaacaaaggt tggctataaa gaggtcatca gtatatgaaa cagccccctg ctgtccattc 2340
   cttattccat agaaaagcct tgacttgagg ttagattttt tttatatttt gttttgtgtt 2400
   atttttttct ttaacatccc taaaattttc cttacatgtt ttactagcca gatttttcct 2460
   cctctcctga ctactcccag tcatagctgt ccctcttctc ttatggagat ccctcgacgg 2520
   atcggccgca attcgtaatc atgtcatagc tgtttcctgt gtgaaattgt tatccgctca 2580
   caattccaca caacatacga gccggaagca taaagtgtaa agcctggggt gcctaatgag 2640
   tgagctaact cacattaatt gcgttgcgct cactgcccgc tttccagtcg ggaaacctgt 2700
   cgtgccagct gcattaatga atcggccaac gcgcggggag aggcggtttg cgtattgggc 2760
   gctcttccgc ttcctcgctc actgactcgc tgcgctcggt cgttcggctg cggcgagcgg 2820
   tatcagctca ctcaaaggcg gtaatacggt tatccacaga atcaggggat aacgcaggaa 2880
   agaacatgtg agcaaaaggc cagcaaaagg ccaggaaccg taaaaaggcc gcgttgctgg 2940
   cgtttttcca taggctccgc ccccctgacg agcatcacaa aaatcgacgc tcaagtcaga 3000
   ggtggcgaaa cccgacagga ctataaagat accaggcgtt tccccctgga agctccctcg 3060
   tgcgctctcc tgttccgacc ctgccgctta ccggatacct gtccgccttt ctcccttcgg 3120
   gaagcgtggc gctttctcat agctcacgct gtaggtatct cagttcggtg taggtcgttc 3180
   gctccaagct gggctgtgtg cacgaacccc ccgttcagcc cgaccgctgc gccttatccg 3240
   gtaactatcg tcttgagtcc aacccggtaa gacacgactt atcgccactg gcagcagcca 3300
   ctggtaacag gattagcaga gcgaggtatg taggcggtgc tacagagttc ttgaagtggt 3360
   ggcctaacta cggctacact agaagaacag tatttggtat ctgcgctctg ctgaagccag 3420
   ttaccttcgg aaaaagagtt ggtagctctt gatccggcaa acaaaccacc gctggtagcg 3480
   gtggtttttt tgtttgcaag cagcagatta cgcgcagaaa aaaaggatct caagaagatc 3540
   ctttgatctt ttctacgggg tctgacgctc agtggaacga aaactcacgt taagggattt 3600
   tggtcatgag attatcaaaa aggatcttca cctagatcct tttaaattaa aaatgaagtt 3660
   ttaaatcaat ctaaagtata tatgagtaaa cttggtctga cagttaccaa tgcttaatca 3720
   gtgaggcacc tatctcagcg atctgtctat ttcgttcatc catagttgcc tgactccccg 3780
   tcgtgtagat aactacgata cgggagggct taccatctgg ccccagtgct gcaatgatac 3840
   cgcgagaccc acgctcaccg gctccagatt tatcagcaat aaaccagcca gccggaaggg 3900
   ccgagcgcag aagtggtcct gcaactttat ccgcctccat ccagtctatt aattgttgcc 3960
   gggaagctag agtaagtagt tcgccagtta atagtttgcg caacgttgtt gccattgcta 4020
   caggcatcgt ggtgtcacgc tcgtcgtttg gtatggcttc attcagctcc ggttcccaac 4080
   gatcaaggcg agttacatga tcccccatgt tgtgcaaaaa agcgggttag ctccttcggt 4140
   cctccgatcg ttgtcagaag taagttggcc gcagtgttat cactcatggt tatggcagca 4200
   ctgcataatt ctcttactgt catgccatcc gtaagatgct tttctgtgac tggtgagtac 4260
   tcaaccaagt cattctgaga atagtgtatg cggcgaccga gttgctcttg cccggcgtca 4320
   atacgggata ataccgcgcc acatagcaga actttaaaag tgctcatcat tggaaaacgt 4380
   tcttcggggc gaaaactctc aaggatctta ccgctgttga gatccagttc gatgtaaccc 4440
   actcgtgcac ccaactgatc ttcagcatct tttactttca ccagcgtttc tgggtgagca 4500
   aaaacaggaa ggcaaaatgc cgcaaaaaag ggaataaggg cgacacggaa atgttgaata 4560
   ctcatactct tcctttttca atattattga agcatttatc agggttattg tctcatgagc 4620
   ggatacatat ttgaatgtat ttagaaaaat aaacaaatag gggttccgcg cacatttccc 4680
   cgaaaagtgc cacctaaatt gtaagcgtta atattttgtt aaaattcgcg ttaaattttt 4740
   gttaaatcag ctcatttttt aaccaatagg ccgaaatcgg caaaatccct tataaatcaa 4800
   aagaatagac cgagataggg ttgagtgttg ttccagtttg gaacaagagt ccactattaa 4860
   agaacgtgga ctccaacgtc aaagggcgaa aaaccgtcta tcagggcgat ggcccactac 4920
   gtgaaccatc accctaatca agttttttgg ggtcgaggtg ccgtaaagca ctaaatcgga 4980
   accctaaagg gagcccccga tttagagctt gacggggaaa gccggcgaac gtggcgagaa 5040
   aggaagggaa gaaagcgaaa ggagcgggcg ctagggcgct ggcaagtgta gcggtcacgc 5100
   tgcgcgtaac caccacaccc gccgcgctta atgcgccgct acagggcgcg tcccattcgc 5160
   cattcaggct gcgcaactgt tgggaagggc gatcggtgcg ggcctcttcg ctattacgcc 5220
   agctggcgaa agggggatgt gctgcaaggc gattaagttg ggtaacgcca gggttttccc 5280
   agtcacgacg ttgtaaaacg acggccagtg agcgcgcgta atacgactca ctatagggcg 5340
   aattggagct ccaccgcggt g 5361
<210> 15
   <211> 5361
   <212> DNA
   <213> Artificial sequence
<220>
   <223> E93R Nef mutant expressing vector
<400> 15
   gcggccgctc tagagagctt ggcccattgc atacgttgta tccatatcat aatatgtaca      60
   tttatattgg ctcatgtcca acattaccgc catgttgaca ttgattattg actagttatt     120
   aatagtaatc aattacgggg tcattagttc atagcccata tatggagttc cgcgttacat     180
   aacttacggt aaatggcccg cctggctgac cgcccaacga cccccgccca ttgacgtcaa     240
   taatgacgta tgttcccata gtaacgccaa tagggacttt ccattgacgt caatgggtgg     300
   agtatttacg gtaaactgcc cacttggcag tacatcaagt gtatcatatg ccaagtacgc     360
   cccctattga cgtcaatgac ggtaaatggc ccgcctggca ttatgcccag tacatgacct     420
   tatgggactt tcctacttgg cagtacatct acgtattagt catcgctatt accatggtga     480
   tgcggttttg gcagtacatc aatgggcgtg gatagcggtt tgactcacgg ggatttccaa     540
   gtctccaccc cattgacgtc aatgggagtt tgttttggca ccaaaatcaa cgggactttc     600
   caaaatgtcg taacaactcc gccccattga cgcaaatggg cggtaggcgt gtacggtggg     660
   aggtctatat aagcagagct cgtttagtga accgtcagat cgcctggaga cgccatccac     720
   gctgttttga cctccataga agacaccggg accgatccag cctccggtcg accgatcctg     780
   agaacttcag ggtgagtttg gggacccttg attgttcttt ctttttcgct attgtaaaat     840
   tcatgttata tggagggggc aaagttttca gggtgttgtt tagaatggga agatgtccct     900
   tgtatcacca tggaccctca tgataatttt gtttctttca ctttctactc tgttgacaac     960
   cattgtctcc tcttattttc ttttcatttt ctgtaacttt ttcgttaaac tttagcttgc    1020
   atttgtaacg aatttttaaa ttcacttttg tttatttgtc agattgtaag tactttctct    1080
   aatcactttt ttttcaaggc aatcagggta tattatattg tacttcagca cagttttaga    1140
   gaacaattgt tataattaaa tgataaggta gaatatttct gcatataaat tctggctggc    1200
   gtggaaatat tcttattggt agaaacaact acaccctggt catcatcctg cctttctctt    1260
   tatggttaca atgatataca ctgtttgaga tgaggataaa atactctgag tccaaaccgg    1320
   gcccctctgc taaccatgtt catgccttct tctctttcct acagctcctg ggcaacgtgc    1380
   tggttgttgt gctgtctcat cattttggca aagaattcct cgacggatcc ctcgacccaa    1440
   ctagaaccat ggggtgggag cagcatctcg agacctggaa aaacatggag caatcacaag    1500
   tagcaataca gcagctacca atgctgcttg tgcctggcta gaagcacaag aggaggagga    1560
   ggtgggtttt ccagtcacac ctcaggtacc tttaagacca atgacttaca aggcagctgt    1620
   agatcttagc cactttctta agcgaaaggg gggactggaa gggctaattc actcccaacg    1680
   aagacaagat atccttgatc tgtggatcta ccacacacaa ggctacttcc ctgattggca    1740
   gaactacaca ccagggccag gggtcagata tccactgacc tttggatggt gctacaagct    1800
   agtaccagtt gagccagata aggtagaaga ggccaataaa ggagagaaca ccagcttgtt    1860
   acaccctgtg agcctgcatg gaatggatga ccctgagaga gaagtgttag agtggaggtt 1920
   tgacagccgc ctagcatttc atcacgtggc ccgagagctg catccggagt acttcaagaa 1980
   ctgctgaacg cgtcaggtgc aggctgccta tcagaaggtg gtggctggtg tggccaatgc 2040
   cctggctcac aaataccact gagatctttt tccctctgcc aaaaattatg gggacatcat 2100
   gaagcccctt gagcatctga cttctggcta ataaaggaaa tttattttca ttgcaatagt 2160
   gtgttggaat tttttgtgtc tctcactcgg aaggacatat gggagggcaa atcatttaaa 2220
   acatcagaat gagtatttgg tttagagttt ggcaacatat gcccatatgc tggctgccat 2280
   gaacaaaggt tggctataaa gaggtcatca gtatatgaaa cagccccctg ctgtccattc 2340
   cttattccat agaaaagcct tgacttgagg ttagattttt tttatatttt gttttgtgtt 2400
   atttttttct ttaacatccc taaaattttc cttacatgtt ttactagcca gatttttcct 2460
   cctctcctga ctactcccag tcatagctgt ccctcttctc ttatggagat ccctcgacgg 2520
   atcggccgca attcgtaatc atgtcatagc tgtttcctgt gtgaaattgt tatccgctca 2580
   caattccaca caacatacga gccggaagca taaagtgtaa agcctggggt gcctaatgag 2640
   tgagctaact cacattaatt gcgttgcgct cactgcccgc tttccagtcg ggaaacctgt 2700
   cgtgccagct gcattaatga atcggccaac gcgcggggag aggcggtttg cgtattgggc 2760
   gctcttccgc ttcctcgctc actgactcgc tgcgctcggt cgttcggctg cggcgagcgg 2820
   tatcagctca ctcaaaggcg gtaatacggt tatccacaga atcaggggat aacgcaggaa 2880
   agaacatgtg agcaaaaggc cagcaaaagg ccaggaaccg taaaaaggcc gcgttgctgg 2940
   cgtttttcca taggctccgc ccccctgacg agcatcacaa aaatcgacgc tcaagtcaga 3000
   ggtggcgaaa cccgacagga ctataaagat accaggcgtt tccccctgga agctccctcg 3060
   tgcgctctcc tgttccgacc ctgccgctta ccggatacct gtccgccttt ctcccttcgg 3120
   gaagcgtggc gctttctcat agctcacgct gtaggtatct cagttcggtg taggtcgttc 3180
   gctccaagct gggctgtgtg cacgaacccc ccgttcagcc cgaccgctgc gccttatccg 3240
   gtaactatcg tcttgagtcc aacccggtaa gacacgactt atcgccactg gcagcagcca 3300
   ctggtaacag gattagcaga gcgaggtatg taggcggtgc tacagagttc ttgaagtggt 3360
   ggcctaacta cggctacact agaagaacag tatttggtat ctgcgctctg ctgaagccag 3420
   ttaccttcgg aaaaagagtt ggtagctctt gatccggcaa acaaaccacc gctggtagcg 3480
   gtggtttttt tgtttgcaag cagcagatta cgcgcagaaa aaaaggatct caagaagatc 3540
   ctttgatctt ttctacgggg tctgacgctc agtggaacga aaactcacgt taagggattt 3600
   tggtcatgag attatcaaaa aggatcttca cctagatcct tttaaattaa aaatgaagtt 3660
   ttaaatcaat ctaaagtata tatgagtaaa cttggtctga cagttaccaa tgcttaatca 3720
   gtgaggcacc tatctcagcg atctgtctat ttcgttcatc catagttgcc tgactccccg 3780
   tcgtgtagat aactacgata cgggagggct taccatctgg ccccagtgct gcaatgatac 3840
   cgcgagaccc acgctcaccg gctccagatt tatcagcaat aaaccagcca gccggaaggg 3900
   ccgagcgcag aagtggtcct gcaactttat ccgcctccat ccagtctatt aattgttgcc 3960
   gggaagctag agtaagtagt tcgccagtta atagtttgcg caacgttgtt gccattgcta 4020
   caggcatcgt ggtgtcacgc tcgtcgtttg gtatggcttc attcagctcc ggttcccaac 4080
   gatcaaggcg agttacatga tcccccatgt tgtgcaaaaa agcggttagc ctccttcggt 4140
   cctccgatcg ttgtcagaag taagttggcc gcagtgttat cactcatggt tatggcagca 4200
   ctgcataatt ctcttactgt catgccatcc gtaagatgct tttctgtgac tggtgagtac 4260
   tcaaccaagt cattctgaga atagtgtatg cggcgaccga gttgctcttg cccggcgtca 4320
   atacgggata ataccgcgcc acatagcaga actttaaaag tgctcatcat tggaaaacgt 4380
   tcttcggggc gaaaactctc aaggatctta ccgctgttga gatccagttc gatgtaaccc 4440
   actcgtgcac ccaactgatc ttcagcatct tttactttca ccagcgtttc tgggtgagca 4500
   aaaacaggaa ggcaaaatgc cgcaaaaaag ggaataaggg cgacacggaa atgttgaata 4560
   ctcatactct tcctttttca atattattga agcatttatc agggttattg tctcatgagc 4620
   ggatacatat ttgaatgtat ttagaaaaat aaacaaatag gggttccgcg cacatttccc 4680
   cgaaaagtgc cacctaaatt gtaagcgtta atattttgtt aaaattcgcg ttaaattttt 4740
   gttaaatcag ctcatttttt aaccaatagg ccgaaatcgg caaaatccct tataaatcaa 4800
   aagaatagac cgagataggg ttgagtgttg ttccagtttg gaacaagagt ccactattaa 4860
   agaacgtgga ctccaacgtc aaagggcgaa aaaccgtcta tcagggcgat ggcccactac 4920
   gtgaaccatc accctaatca agttttttgg ggtcgaggtg ccgtaaagca ctaaatcgga 4980
   accctaaagg gagcccccga tttagagctt gacggggaaa gccggcgaac gtggcgagaa 5040
   aggaagggaa gaaagcgaaa ggagcgggcg ctagggcgct ggcaagtgta gcggtcacgc 5100
   tgcgcgtaac caccacaccc gccgcgctta atgcgccgct acagggcgcg tcccattcgc 5160
   cattcaggct gcgcaactgt tgggaagggc gatcggtgcg ggcctcttcg ctattacgcc 5220
   agctggcgaa agggggatgt gctgcaaggc gattaagttg ggtaacgcca gggttttccc 5280
   agtcacgacg ttgtaaaacg acggccagtg agcgcgcgta atacgactca ctatagggcg 5340
   aattggagct ccaccgcggt g 5361
<210> 16
   <211> 10216
   <212> DNA
   <213> Artificial sequence
<220>
   <223> pDFG-expNef vector expressing Nef fused to an export signal seque nce
<400> 16
   ggatccgcgg gattagtcca atttgttaaa gacaggatat cagtggtcca ggctctagtt 60
   ttgactcaac aatatcacca gctgaagcct atagagtacg agccatagat aaaataaaag     120
   attttattta gtctccagaa aaagggggga atgaaagacc ccacctgtag gtttggcaag     180
   ctagcttaag taacgccatt ttgcaaggca tggaaaaata cataactgag aatagagaag     240
   ttcagatcaa ggtcaggaac agatggaaca gctgaatatg ggccaaacag gatatctgtg     300
   gtaagcagtt cctgccccgg ctcagggcca agaacagatg gaacagctga atatgggcca     360
   aacaggatat ctgtggtaag cagttcctgc cccggctcag ggccaagaac agatggtccc     420
   cagatgcggt ccagccctca gcagtttcta gagaaccatc agatgtttcc agggtgcccc     480
   aaggacctga aatgaccctg tgccttattt gaactaacca atcagttcgc ttctcgcttc     540
   tgttcgcgcg cttctgctcc ccgagctcaa taaaagagcc cacaacccct cactcggggc     600
   gccagtcctc cgattgactg agtcgcccgg gtacccgtgt atccaataaa ccctcttgca     660
   gttgcatccg acttgtggtc tcgctgttcc ttgggagggt ctcctctgag tgattgacta     720
   cccgtcagcg ggggtctttc acatgcagca tgtatcaaaa ttaatttggt tttttttctt     780
   aagtatttac attaaatggc catagtactt aaagttacat tggcttcctt gaaataaaca     840
   tggagtattc agaatgtgtc ataaatattt ctaattttaa gatagtatct ccattggctt     900
   tctacttttt cttttatttt tttttgtcct ctgtcttcca tttgttgttg ttgttgtttg     960
   tttgtttgtt tgttggttgg ttggttaatt tttttttaaa gatcctacac tatagttcaa    1020
   gctagactat tagctactct gtaacccagg gtgaccttga agtcatgggt agcctgctgt    1080
   tttagccttc ccacatctaa gattacaggt atgagctatc atttttggta tattgattga    1140
   ttgattgatt gatgtgtgtg tgtgtgattg tgtttgtgtg tgtgattgtg tatatgtgtg    1200
   tatggttgtg tgtgattgtg tgtatgtatg tttgtgtgtg attgtgtgtg tgtgattgtg    1260
   catgtgtgtg tgtgtgattg tgtttatgtg tatgattgtg tgtgtgtgtg tgtgtgtgtg    1320
   tgtgtgtgtg tgtgtgtgtg tgttgtgtat atatatttat ggtagtgaga ggcaacgctc    1380
   cggctcaggt gtcaggttgg tttttgagac agagtctttc acttagcttg gaattcttga    1440
   agacgaaagg gcctcgtgat acgcctattt ttataggtta atgtcatgat aataatggtt    1500
   tcttagacgt caggtggcac ttttcgggga aatgtgcgcg gaacccctat ttgtttattt    1560
   ttctaaatac attcaaatat gtatccgctc atgagacaat aaccctgata aatgcttcaa    1620
   taatattgaa aaaggaagag tatgagtatt caacatttcc gtgtcgccct tattcccttt    1680
   tttgcggcat tttgccttcc tgtttttgct cacccagaaa cgctggtgaa agtaaaagat    1740
   gctgaagatc agttgggtgc acgagtgggt tacatcgaac tggatctcaa cagcggtaag    1800
   atccttgaga gttttcgccc cgaagaacgt tttccaatga tgagcacttt taaagttctg    1860
   ctatgtggcg cggtattatc ccgtgttgac gccgggcaac agcaactcgg tcgccgcata    1920
   cactattctc agaatgactt ggttgagtac tcaccagtca cagaaaagca tcttacggat    1980
   ggcatgacag taagagaatt atgcagtgct gccataacca tgagtgataa cactgcggcc 2040
   aacttacttc tgacaacgat cggaggaccg aaggagctaa ccgctttttt gcacaacatg 2100
   ggggatcatg taactcgcct tgatcgttgg gaaccggagc tgaatgaagc cataccaaac 2160
   gacgagcgtg acaccacgat gcctgcagca atggcaacaa cgttgcgcaa actattaact 2220
   ggcgaactac ttactctagc ttcccggcaa caattaatag actggatgga ggcggataaa 2280
   gttgcaggac cacttctgcg ctcggccctt ccggctggct ggtttattgc tgataaatct 2340
   ggagccggtg agcgtgggtc tcgcggtatc attgcagcac tggggccaga tggtaagccc 2400
   tcccgtatcg tagttatcta cacgacgggg agtcaggcaa ctatggatga acgaaataga 2460
   cagatcgctg agataggtgc ctcactgatt aagcattggt aactgtcaga ccaagtttac 2520
   tcatatatac tttagattga tttaaaactt catttttaat ttaaaaggat ctaggtgaag 2580
   atcctttttg ataatctcat gaccaaaatc ccttaacgtg agttttcgtt ccactgagcg 2640
   tcagaccccg tagaaaagat caaaggatct tcttgagatc ctttttttct gcgcgtaatc 2700
   tgctgcttgc aaacaaaaaa accaccgcta ccagcggtgg tttgtttgcc ggatcaagag 2760
   ctaccaactc tttttccgaa ggtaactggc ttcagcagag cgcagatacc aaatactgtc 2820
   cttctagtgt agccgtagtt aggccaccac ttcaagaact ctgtagcacc gcctacatac 2880
   ctcgctctgc taatcctgtt accagtggct gctgccagtg gcgataagtc gtgtcttacc 2940
   gggttggact caagacgata gttaccggat aaggcgcagc ggtcgggctg aacggggggt 3000
   tcgtgcacac agcccagctt ggagcgaacg acctacaccg aactgagata cctacagcgt 3060
   gagctatgag aaagcgccac gcttcccgaa gggagaaagg cggacaggta tccggtaagc 3120
   ggcagggtcg gaacaggaga gcgcacgagg gagcttccag ggggaaacgc ctggtatctt 3180
   tatagtcctg tcgggtttcg ccacctctga cttgagcgtc gatttttgtg atgctcgtca 3240
   ggggggcgga gcctatggaa aaacgccagc aacgcggcct ttttacggtt cctggccttt 3300
   tgctggcctt ttgctcacat gttctttcct gcgttatccc ctgattctgt ggataaccgt 3360
   attaccgcct ttgagtgagc tgataccgct cgccgcagcc gaacgaccga gcgcagcgag 3420
   tcagtgagcg aggaagcgga agagcgcctg atgcggtatt ttctccttac gcatctgtgc 3480
   ggtatttcac accgcatata tggtgcactc tcagtacaat ctgctctgat gccgcatagt 3540
   taagccagta tacactccgc tatcgctacg tgactgggtc atggctgcgc cccgacaccc 3600
   gccaacaccc gctgacgcgc cctgacgggc ttgtctgctc ccggcatccg cttacagaca 3660
   agctgtgacc gtctccggga gctgcatgtg tcagaggttt tcaccgtcat caccgaaacg 3720
   cgcgaggcag ctgcggtaaa gctcatcagc gtggtcgtga agcgattcac agatgtctgc 3780
   ctgttcatcc gcgtccagct cgttgagttt ctccagaagc gttaatgtct ggcttctgat 3840
   aaagcgggcc atgttaaggg cggttttttc ctgtttggtc acttgatgcc tccgtgtaag 3900
   ggggaatttc tgttcatggg ggtaatgata ccgatgaaac gagagaggat gctcacgata 3960
   cgggttactg atgatgaaca tgcccggtta ctggaacgtt gtgagggtaa acaactggcg 4020
   gtatggatgc ggcgggacca gagaaaaatc actcagggtc aatgccagcg cttcgttaat 4080
   acagatgtag gtgttccaca gggtagccag cagcatcctg cgatgcagat ccggaacata 4140
   atggtgcagg gcgctgactt ccgcgtttcc agactttacg aaacacggaa accgaagacc 4200
   attcatgttg ttgctcaggt cgcagacgtt ttgcagcagc agtcgcttca cgttcgctcg 4260
   cgtatcggtg attcattctg ctaaccagta aggcaacccc gccagcctag ccgggtcctc 4320
   aacgacagga gcacgatcat gcgcacccgt ggccaggacc caacgctgcc cgagatgcgc 4380
   cgcgtgcggc tgctggagat ggcggacgcg atggatatgt tctgccaagg gttggtttgc 4440
   gcattcacag ttctccgcaa gaattgattg gctccaattc ttggagtggt gaatccgtta 4500
   gcgaggtgcc gccggcttcc attcaggtcg aggtggcccg gctccatgca ccgcgacgca 4560
   acgcggggag gcagacaagg tatagggcgg cgcctacaat ccatgccaac ccgttccatg 4620
   tgctcgccga ggcggcataa atcgccgtga cgatcagcgg tccagtgatc gaagttaggc 4680
   tggtaagagc cgcgagcgat ccttgaagct gtccctgatg gtcgtcatct acctgcctgg 4740
   acagcatggc ctgcaacgcg ggcatcccga tgccgccgga agcgagaaga atcataatgg 4800
   ggaaggccat ccagcctcgc gtcgcgaacg ccagcaagac gtagcccagc gcgtcggccg 4860
   ccatgccggc gataatggcc tgcttctcgc cgaaacgttt ggtggcggga ccagtgacga 4920
   aggcttgagc gagggcgtgc aagattccga ataccgcaag cgacaggccg atcatcgtcg 4980
   cgctccagcg aaagcggtcc tcgccgaaaa tgacccagag cgctgccggc acctgtccta 5040
   cgagttgcat gataaagaag acagtcataa gtgcggcgac gatagtcatg ccccgcgccc 5100
   accggaagga gctgactggg ttgaaggctc tcaagggcat cggtcgacgc tctcccttat 5160
   gcgactcctg cattaggaag cagcccagta gtaggttgag gccgttgagc accgccgccg 5220
   caaggaatgg tgcatgcaag gagatggcgc ccaacagtcc cccggccacg gggcctgcca 5280
   ccatacccac gccgaaacaa gcgctcatga gcccgaagtg gcgagcccga tcttccccat 5340
   cggtgatgtc ggcgatatag gcgccagcaa ccgcacctgt ggcgccggtg atgccggcca 5400
   cgatgcgtcc ggcgtagagg atcgatccac aggacgggtg tggtcgccat gatcgcgtag 5460
   tcgatagtgg ctccaagtag cgaagcgagc aggactgggc ggcggccaaa gcggtcggac 5520
   agtgctccga gaacgggtgc gcatagaaat tgcatcaacg catatagcgc tagcagcacg 5580
   ccatagtgac tggcgatgct gtcggaatgg acgatatccc gcaagaggcc cggcagtacc 5640
   ggcataacca agcctatgcc tacagcatcc agggtgacgg tgccgaggat gacgatgagc 5700
   gcattgttag atttcataca cggtgcctga ctgcgttagc aatttaactg tgataaacta 5760
   ccgcattaaa gctttgctct taggagtttc ctaatacatc ccaaactcaa atatataaag 5820
   catttgactt gttctatgcc ctagggggcg gggggaagct aagccagctt tttttaacat 5880
   ttaaaatgtt aattccattt taaatgcaca gatgttttta tttcataagg gtttcaatgt 5940
   gcatgaatgc tgcaatattc ctgttaccaa agctagtata aataaaaata gataaacgtg 6000
   gaaattactt agagtttctg tcattaacgt ttccttcctc agttgacaac ataaatgcgc 6060
   tgctgagaag ccagtttgca tctgtcagga tcaatttccc attatgccag tcatattaat 6120
   tactagtcaa ttagttgatt tttatttttg acatatacat gtgaaagacc ccacctgtag 6180
   gtttggcaag ctagcttaag taacgccatt ttgcaaggca tggaaaaata cataactgag 6240
   agggccaaac aggatatctg tggtaagcag ttcatagaaa agttcagatc aaggtcagga 6300
   acagatggaa cagctgaata tctgccccgg ctcagggcca agaacagatg gaacagctga 6360
   atatgggcca aacaggatat ctgtggtaag cagttcctgc cccggctcag ggccaagaac 6420
   agatggtccc cagatgcggt ccagccctca gcagtttcta gagaaccatc agatgtttcc 6480
   agggtgcccc aaggacctga aatgaccctg tgccttattt gaactaacca atcagttcgc 6540
   ttctcgcttc tgttcgcgcg cttctgctcc ccgagctcaa taaaagagcc cacaacccct 6600
   cactcggcgc gccagtcctc cgattgactg agtcgcccgg gtacccgtgt atccaataaa 6660
   ccctcttgca gttgcatccg acttgtggtc tcgctgttcc ttgggagggt ctcctctgag 6720
   tgattgacta cccgtcagcg ggggtctttc atttgggggc tcgtccggga tcgggagacc 6780
   cctgcccagg gaccaccgac ccaccaccgg gaggtaagct ggccagcaac ttatctgtgt 6840
   ctgtccgatt gtctagtgtc tatgactgat tttatgcgcc tgcgtcggta ctagttagct 6900
   aactagctct gtatctggcg gacccgtggt ggaactgacg agttcggaac acccggccgc 6960
   aaccctggga gacgtcccag ggacttcggg ggccgttttt gtggcccgac ctgagtccaa 7020
   aaatcccgat cgttttggac tctttggtgc acccccctta gaggagggat atgtggttct 7080
   ggtaggagac gagaacctaa aacagttccc gcctccgtct gaatttttgc tttcggtttg 7140
   ggaccgaagc cgcgccgcgc gtcttgtctg ctgcagcatc gttctgtgtt gtctctgtct 7200
   gactgtgttt ctgtatttgt ctgaaaatat gggcccgccg cggcgggcca gactgttacc 7260
   actcccttaa gtttgacctt aggtcactgg aaagatgtcg agcggatcgc tcacaaccag 7320
   tcggtagatg tcaagaagag acgttgggtt accttctgct ctgcagaatg gccaaccttt 7380
   aacgtcggat ggccgcgaga cggcaccttt aaccgagacc tcatcaccca ggttaagatc 7440
   aaggtctttt cacctggccc gcatggacac ccagaccagg tcccctacat cgtgacctgg 7500
   gaagccttgg cttttgaccc ccctccctgg gtcaagccct ttgtacaccc taagcctccg 7560
   cctcctcttc ctccatccgc cccgtctctc ccccttgaac ctcctcgttc gaccccgcct 7620
   cgatcctccc tttatccagc cctcactcct tctctaggcg cccccatatg gccatatgag 7680
   atcttatatg gggcaccccc gccccttgta aacttccctg accctgacat gacaagagtt 7740
   actaacagcc cctctctcca agctcactta caggctctct acttagtcca gcacgaagtc 7800
   tggagacctc tggcggcagc ctaccaagaa caactggacc gaccggtatg gagacagaca 7860
   cactcctgct atgggtactg ctgctctggg ttccaggttc cactggtgac gcggcccagc 7920
   cggccggtgg caagtggtca aaaagtagtg tggttggatg gcctactgta agggaaagaa 7980
   tgagacgagc tgagccagca gcagatgggg tgggagcagc atctcgagac ctggaaaaac 8040
   atggagcaat cacaagtagc aatacagcag ctaccaatgc tgcttgtgcc tggctagaag 8100
   cacaagagga ggaggaggtg ggttttccag tcacacctca ggtaccttta agaccaatga 8160
   cttacaaggc agctgtagat cttagccact ttcttaagga aaagggggga ctggaagggc 8220
   taattcactc ccaacgaaga caagatatcc ttgatctgtg gatctaccac acacaaggct 8280
   acttccctga ttggcagaac tacacaccag ggccaggggt cagatatcca ctgacctttg 8340
   gatggtgcta caagctagta ccagttgagc cagataaggt agaagaggcc aataaaggag 8400
   agaacaccag cttgttacac cctgtgagcc tgcatggaat ggatgaccct gagagagaag 8460
   tgttagagtg gaggtttgac agccgcctag catttcatca cgtggcccga gagctgcatc 8520
   cggagtactt caagaactgc tgaacgcgtt gactcgagaa ttccggcccc tctccctccc 8580
   ccccccctaa cgttactggc cgaagccgct tggaataagg ccggtgtgcg tttgtctata 8640
   tgttattttc caccatattg ccgtcttttg gcaatgtgag ggcccggaaa cctggccctg 8700
   tcttcttgac gagcattcct aggggtcttt cccctctcgc caaaggaatg caaggtctgt 8760
   tgaatgtcgt gaaggaagca gttcctctgg aagcttcttg aagacaaaca acgtctgtag 8820
   cgaccctttg caggcagcgg aaccccccac ctggcgacag gtgcctctgc ggccaaaagc 8880
   cacgtgtata agatacacct gcaaaggcgg cacaacccca gtgccacgtt gtgagttgga 8940
   tagttgtgga aagagtcaaa tggctctcct caagcgtatt caacaagggg ctgaaggatg 9000
   cccagaaggt accccattgt atgggatctg atctggggcc tcggtgcaca tgctttacat 9060
   gtgtttagtc gaggttaaaa aacgtctagg ccccccgaac cacggggacg tggttttcct 9120
   ttgaaaaaca cgatgataat atggccacaa ccatggaaaa agcctgaact caccgcgacg 9180
   tctgtcgaga agtttctgat cgaaaagttc gacagcgtct ccgacctgat gcagctctcg 9240
   gagggcgaag aatctcgtgc tttcagcttc gatgtaggag ggcgtggata tgtcctgcgg 9300
   gtaaatagct gcgccgatgg tttctacaaa gatcgttatg tttatcggca ctttgcatcg 9360
   gccgcgctcc cgattccgga agtgcttgac attggggaat tcagcgagag cctgacctat 9420
   tgcatctccc gccgtgcaca gggtgtcacg ttgcaagacc tgcctgaaac cgaactgccc 9480
   gctgttctgc agccggtcgc ggaggccatg gatgcgatcg ctgcggccga tcttagccag 9540
   acgagcgggt tcggcccatt cggaccgcaa ggaatcggtc aatacactac atggcgtgat 9600
   ttcatatgcg cgattgctga tccccatgtg tatcactggc aaactgtgat ggacgacacc 9660
   gtcagtgcgt ccgtcgcgca ggctctcgat gagctgatgc tttgggccga ggactgcccc 9720
   gaagtccggc acctcgtgca cgcggatttc ggctccaaca atgtcctgac ggacaatggc 9780
   cgcataacag cggtcattga ctggagcgag gcgatgttcg gggattccca atacgaggtc    9840
   gccaacatct tcttctggag gccgtggttg gcttgtatgg agcagcagac gcgctacttc    9900
   gagcggaggc atccggagct tgcaggatcg ccgcggctcc gggcgtatat gctccgcatt    9960
   ggtcttgacc aactctatca gagcttggtt gacggcaatt tcgatgatgc agcttgggcg   10020
   cagggtcgat gcgacgcaat cgtccgatcc ggagccggga ctgtcgggcg tacacaaatc   10080
   gcccgcagaa gcgcggccgt ctggaccgat ggctgtgtag aagtactcgc cgatagtgga   10140
   aaccgacgcc ccagcactcg tccgagggga tcgggagatg ggggaggcta actgaaacac   10200
   ggaaggagac aatacc                                                   10216
<210> 17
   <211> 231
   <212> PRT
   <213> Artificial sequence
<220>
   <223> Nef fused to an export signal sequence
<400> 17
   Met Glu Thr Asp Thr Leu Leu Leu Trp Val Leu Leu Leu Trp Val Pro
   1               5                   10                  15
   Gly Ser Thr Gly Asp Ala Ala Gln Pro Ala Gly Gly Lys Trp Ser Lys
               20                  25                  30
   Ser Ser Val Val Gly Trp Pro Thr Val Arg Glu Arg Met Arg Arg Ala
           35                  40                  45
   Glu Pro Ala Ala Asp Gly Val Gly Ala Ala Ser Arg Asp Leu Glu Lys
       50                  55                  60
   His Gly Ala Ile Thr Ser Ser Asn Thr Ala Ala Thr Asn Ala Ala Cys
   65                  70                  75                  80
   Ala Trp Leu Glu Ala Gln Glu Glu Glu Glu Val Gly Phe Pro Val Thr
                   85                  90                  95
   Pro Gln Val Pro Leu Arg Pro Met Thr Tyr Lys Ala Ala Val Asp Leu
               100                 105                 110
   Ser His Phe Leu Lys Glu Lys Gly Gly Leu Glu Gly Leu Ile His Ser
           115                 120                 125
   Gln Arg Arg Gln Asp Ile Leu Asp Leu Trp Ile Tyr His Thr Gln Gly
       130                 135                 140
   Tyr Phe Pro Asp Trp Gln Asn Tyr Thr Pro Gly Pro Gly Val Arg Tyr
   145                 150                 155                 160
   Pro Leu Thr Phe Gly Trp Cys Tyr Lys Leu Val Pro Val Glu Pro Asp
                   165                 170                 175
   Lys Val Glu Glu Ala Asn Lys Gly Glu Asn Thr Ser Leu Leu His Pro
               180                 185                 190
   Val Ser Leu His Gly Met Asp Asp Pro Glu Arg Glu Val Leu Glu Trp
           195                 200                 205
   Arg Phe Asp Ser Arg Leu Ala Phe His His Val Ala Arg Glu Leu His
       210                 215                 220
   Pro Glu Tyr Phe Lys Asn Cys
   225                 230
<210> 18
   <211> 10216
   <212> DNA
   <213> Artificial sequence
<220>
   <223> pDFG-expNefE93R vector expressing the E93R Nef mutant fused to an export signal sequence
<400> 18
<210> 19
   <211> 231
   <212> PRT
   <213> Artificial sequence
<220>
   <223> E93R Nef mutant fused to an export signal sequence
<400> 19
   Met Glu Thr Asp Thr Leu Leu Leu Trp Val Leu Leu Leu Trp Val Pro
   1               5                   10                  15
   Gly Ser Thr Gly Asp Ala Ala Gln Pro Ala Gly Gly Lys Trp Ser Lys
               20                  25                  30
   Ser Ser Val Val Gly Trp Pro Thr Val Arg Glu Arg Met Arg Arg Ala
           35                  40                  45
   Glu Pro Ala Ala Asp Gly Val Gly Ala Ala Ser Arg Asp Leu Glu Lys
       50                  55                  60
   His Gly Ala Ile Thr Ser Ser Asn Thr Ala Ala Thr Asn Ala Ala Cys
   65                  70                  75                  80
   Ala Trp Leu Glu Ala Gln Glu Glu Glu Glu Val Gly Phe Pro Val Thr
                   85                  90                  95
   Pro Gln Val Pro Leu Arg Pro Met Thr Tyr Lys Ala Ala Val Asp Leu
               100                 105                 110
   Ser His Phe Leu Lys Arg Lys Gly Gly Leu Glu Gly Leu Ile His Ser
           115                 120                 125
   Gln Arg Arg Gln Asp Ile Leu Asp Leu Trp Ile Tyr His Thr Gln Gly
       130                 135                 140
   Tyr Phe Pro Asp Trp Gln Asn Tyr Thr Pro Gly Pro Gly Val Arg Tyr
   145                 150                 155                 160
   Pro Leu Thr Phe Gly Trp Cys Tyr Lys Leu Val Pro Val Glu Pro Asp
                   165                 170                 175
   Lys Val Glu Glu Ala Asn Lys Gly Glu Asn Thr Ser Leu Leu His Pro
               180                 185                 190
   Val Ser Leu His Gly Met Asp Asp Pro Glu Arg Glu Val Leu Glu Trp
           195                 200                 205
   Arg Phe Asp Ser Arg Leu Ala Phe His His Val Ala Arg Glu Leu His
       210                 215                 220
   Pro Glu Tyr Phe Lys Asn Cys
   225                 230
<210> 20
   <211> 5443
   <212> DNA
   <213> Artificial sequence
<220>
   <223> phCMV-Nef vector expressing Nef
<400> 20
   gcggccgctc tagagagctt ggcccattgc atacgttgta tccatatcat aatatgtaca 60
   tttatattgg ctcatgtcca acattaccgc catgttgaca ttgattattg actagttatt    120
   aatagtaatc aattacgggg tcattagttc atagcccata tatggagttc cgcgttacat    180
   aacttacggt aaatggcccg cctggctgac cgcccaacga cccccgccca ttgacgtcaa    240
   taatgacgta tgttcccata gtaacgccaa tagggacttt ccattgacgt caatgggtgg    300
   agtatttacg gtaaactgcc cacttggcag tacatcaagt gtatcatatg ccaagtacgc    360
   cccctattga cgtcaatgac ggtaaatggc ccgcctggca ttatgcccag tacatgacct    420
   tatgggactt tcctacttgg cagtacatct acgtattagt catcgctatt accatggtga    480
   tgcggttttg gcagtacatc aatgggcgtg gatagcggtt tgactcacgg ggatttccaa    540
   gtctccaccc cattgacgtc aatgggagtt tgttttggca ccaaaatcaa cgggactttc    600
   caaaatgtcg taacaactcc gccccattga cgcaaatggg cggtaggcgt gtacggtggg    660
   aggtctatat aagcagagct cgtttagtga accgtcagat cgcctggaga cgccatccac    720
   gctgttttga cctccataga agacaccggg accgatccag cctccggtcg accgatcctg    780
   agaacttcag ggtgagtttg gggacccttg attgttcttt ctttttcgct attgtaaaat    840
   tcatgttata tggagggggc aaagttttca gggtgttgtt tagaatggga agatgtccct    900
   tgtatcacca tggaccctca tgataatttt gtttctttca ctttctactc tgttgacaac    960
   cattgtctcc tcttattttc ttttcatttt ctgtaacttt ttcgttaaac tttagcttgc   1020
   atttgtaacg aatttttaaa ttcacttttg tttatttgtc agattgtaag tactttctct   1080
   aatcactttt ttttcaaggc aatcagggta tattatattg tacttcagca cagttttaga   1140
   gaacaattgt tataattaaa tgataaggta gaatatttct gcatataaat tctggctggc   1200
   gtggaaatat tcttattggt agaaacaact acaccctggt catcatcctg cctttctctt   1260
   tatggttaca atgatataca ctgtttgaga tgaggataaa atactctgag tccaaaccgg   1320
   gcccctctgc taaccatgtt catgccttct tctctttcct acagctcctg ggcaacgtgc   1380
   tggttgttgt gctgtctcat cattttggca aagaattcct cgacggatcc ctcgacccaa   1440
   ctagaaccat gggtggcaag tggtcaaaaa gtagtgtggt tggatggcct actgtaaggg   1500
   aaagaatgag acgagctgag ccagcagcag atggggtggg agcagcatct cgagacctgg   1560
   aaaaacatgg agcaatcaca agtagcaata cagcagctac caatgctgct tgtgcctggc   1620
   tagaagcaca agaggaggag gaggtgggtt ttccagtcac acctcaggta cctttaagac   1680
   caatgactta caaggcagct gtagatctta gccactttct taaggaaaag gggggactgg   1740
   aagggctaat tcactcccaa cgaagacaag atatccttga tctgtggatc taccacacac   1800
   aaggctactt ccctgattgg cagaactaca caccagggcc aggggtcaga tatccactga   1860
   cctttggatg gtgctacaag ctagtaccag ttgagccaga taaggtagaa gaggccaata   1920
   aaggagagaa caccagcttg ttacaccctg tgagcctgca tggaatggat gaccctgaga   1980
   gagaagtgtt agagtggagg tttgacagcc gcctagcatt tcatcacgtg gcccgagagc   2040
   tgcatccgga gtacttcaag aactgctgaa cgcgtcaggt gcaggctgcc tatcagaagg   2100
   tggtggctgg tgtggccaat gccctggctc acaaatacca ctgagatctt tttccctctg   2160
   ccaaaaatta tggggacatc atgaagcccc ttgagcatct gacttctggc taataaagga   2220
   aatttatttt cattgcaata gtgtgttgga attttttgtg tctctcactc ggaaggacat   2280
   atgggagggc aaatcattta aaacatcaga atgagtattt ggtttagagt ttggcaacat   2340
   atgcccatat gctggctgcc atgaacaaag gttggctata aagaggtcat cagtatatga   2400
   aacagccccc tgctgtccat tccttattcc atagaaaagc cttgacttga ggttagattt   2460
   tttttatatt ttgttttgtg ttattttttt ctttaacatc cctaaaattt tccttacatg   2520
   ttttactagc cagatttttc ctcctctcct gactactccc agtcatagct gtccctcttc   2580
   tcttatggag atccctcgac ggatcggccg caattcgtaa tcatgtcata gctgtttcct   2640
   gtgtgaaatt gttatccgct cacaattcca cacaacatac gagccggaag cataaagtgt   2700
   aaagcctggg gtgcctaatg agtgagctaa ctcacattaa ttgcgttgcg ctcactgccc   2760
   gctttccagt cgggaaacct gtcgtgccag ctgcattaat gaatcggcca acgcgcgggg   2820
   agaggcggtt tgcgtattgg gcgctcttcc gcttcctcgc tcactgactc gctgcgctcg   2880
   gtcgttcggc tgcggcgagc ggtatcagct cactcaaagg cggtaatacg gttatccaca   2940
   gaatcagggg ataacgcagg aaagaacatg tgagcaaaag gccagcaaaa ggccaggaac   3000
   cgtaaaaagg ccgcgttgct ggcgtttttc cataggctcc gcccccctga cgagcatcac   3060
   aaaaatcgac gctcaagtca gaggtggcga aacccgacag gactataaag ataccaggcg   3120
   tttccccctg gaagctccct cgtgcgctct cctgttccga ccctgccgct taccggatac   3180
   ctgtccgcct ttctcccttc gggaagcgtg gcgctttctc atagctcacg ctgtaggtat   3240
   ctcagttcgg tgtaggtcgt tcgctccaag ctgggctgtg tgcacgaacc ccccgttcag   3300
   cccgaccgct gcgccttatc cggtaactat cgtcttgagt ccaacccggt aagacacgac   3360
   ttatcgccac tggcagcagc cactggtaac aggattagca gagcgaggta tgtaggcggt   3420
   gctacagagt tcttgaagtg gtggcctaac tacggctaca ctagaagaac agtatttggt   3480
   atctgcgctc tgctgaagcc agttaccttc ggaaaaagag ttggtagctc ttgatccggc   3540
   aaacaaacca ccgctggtag cggtggtttt tttgtttgca agcagcagat tacgcgcaga   3600
   aaaaaaggat ctcaagaaga tcctttgatc ttttctacgg ggtctgacgc tcagtggaac   3660
   gaaaactcac gttaagggat tttggtcatg agattatcaa aaaggatctt cacctagatc   3720
   cttttaaatt aaaaatgaag ttttaaatca atctaaagta tatatgagta aacttggtct   3780
   gacagttacc aatgcttaat cagtgaggca cctatctcag cgatctgtct atttcgttca   3840
   tccatagttg cctgactccc cgtcgtgtag ataactacga tacgggaggg cttaccatct   3900
   ggccccagtg ctgcaatgat accgcgagac ccacgctcac cggctccaga tttatcagca   3960
   ataaaccagc cagccggaag ggccgagcgc agaagtggtc ctgcaacttt atccgcctcc   4020
   atccagtcta ttaattgttg ccgggaagct agagtaagta gttcgccagt taatagtttg   4080
   cgcaacgttg ttgccattgc tacaggcatc gtggtgtcac gctcgtcgtt tggtatggct   4140
   tcattcagct ccggttccca acgatcaagg cgagttacat gatcccccat gttgtgcaaa   4200
   aaagcgggtt agctccttcg gtcctccgat cgttgtcaga agtaagttgg ccgcagtgtt   4260
   atcactcatg gttatggcag cactgcataa ttctcttact gtcatgccat ccgtaagatg   4320
   cttttctgtg actggtgagt actcaaccaa gtcattctga gaatagtgta tgcggcgacc   4380
   gagttgctct tgcccggcgt caatacggga taataccgcg ccacatagca gaactttaaa   4440
   agtgctcatc attggaaaac gttcttcggg gcgaaaactc tcaaggatct taccgctgtt   4500
   gagatccagt tcgatgtaac ccactcgtgc acccaactga tcttcagcat cttttacttt   4560
   caccagcgtt tctgggtgag caaaaacagg aaggcaaaat gccgcaaaaa agggaataag   4620
   ggcgacacgg aaatgttgaa tactcatact cttccttttt caatattatt gaagcattta   4680
   tcagggttat tgtctcatga gcggatacat atttgaatgt atttagaaaa ataaacaaat   4740
   aggggttccg cgcacatttc cccgaaaagt gccacctaaa ttgtaagcgt taatattttg   4800
   ttaaaattcg cgttaaattt ttgttaaatc agctcatttt ttaaccaata ggccgaaatc   4860
   ggcaaaatcc cttataaatc aaaagaatag accgagatag ggttgagtgt tgttccagtt   4920
   tggaacaaga gtccactatt aaagaacgtg gactccaacg tcaaagggcg aaaaaccgtc   4980
   tatcagggcg atggcccact acgtgaacca tcaccctaat caagtttttt ggggtcgagg   5040
   tgccgtaaag cactaaatcg gaaccctaaa gggagccccc gatttagagc ttgacgggga   5100
   aagccggcga acgtggcgag aaaggaaggg aagaaagcga aaggagcggg cgctagggcg   5160
   ctggcaagtg tagcggtcac gctgcgcgta accaccacac ccgccgcgct taatgcgccg   5220
   ctacagggcg cgtcccattc gccattcagg ctgcgcaact gttgggaagg gcgatcggtg   5280
   cgggcctctt cgctattacg ccagctggcg aaagggggat gtgctgcaag gcgattaagt   5340
   tgggtaacgc cagggttttc ccagtcacga cgttgtaaaa cgacggccag tgagcgcgcg   5400
   taatacgact cactataggg cgaattggag ctccaccgcg gtg                      5443
<210> 21
   <211> 5443
   <212> DNA
   <213> Artificial sequence
<220>
   <223> phCMV-NefE93R vector expressing the E93R Nef mutant
<400> 21
   gcggccgctc tagagagctt ggcccattgc atacgttgta tccatatcat aatatgtaca 60
   tttatattgg ctcatgtcca acattaccgc catgttgaca ttgattattg actagttatt 120
   aatagtaatc aattacgggg tcattagttc atagcccata tatggagttc cgcgttacat 180
   aacttacggt aaatggcccg cctggctgac cgcccaacga cccccgccca ttgacgtcaa 240
   taatgacgta tgttcccata gtaacgccaa tagggacttt ccattgacgt caatgggtgg 300
   agtatttacg gtaaactgcc cacttggcag tacatcaagt gtatcatatg ccaagtacgc 360
   cccctattga cgtcaatgac ggtaaatggc ccgcctggca ttatgcccag tacatgacct 420
   tatgggactt tcctacttgg cagtacatct acgtattagt catcgctatt accatggtga 480
   tgcggttttg gcagtacatc aatgggcgtg gatagcggtt tgactcacgg ggatttccaa 540
   gtctccaccc cattgacgtc aatgggagtt tgttttggca ccaaaatcaa cgggactttc 600
   caaaatgtcg taacaactcc gccccattga cgcaaatggg cggtaggcgt gtacggtggg 660
   aggtctatat aagcagagct cgtttagtga accgtcagat cgcctggaga cgccatccac 720
   gctgttttga cctccataga agacaccggg accgatccag cctccggtcg accgatcctg 780
   agaacttcag ggtgagtttg gggacccttg attgttcttt ctttttcgct attgtaaaat 840
   tcatgttata tggagggggc aaagttttca gggtgttgtt tagaatggga agatgtccct 900
   tgtatcacca tggaccctca tgataatttt gtttctttca ctttctactc tgttgacaac 960
   cattgtctcc tcttattttc ttttcatttt ctgtaacttt ttcgttaaac tttagcttgc 1020
   atttgtaacg aatttttaaa ttcacttttg tttatttgtc agattgtaag tactttctct 1080
   aatcactttt ttttcaaggc aatcagggta tattatattg tacttcagca cagttttaga 1140
   gaacaattgt tataattaaa tgataaggta gaatatttct gcatataaat tctggctggc 1200
   gtggaaatat tcttattggt agaaacaact acaccctggt catcatcctg cctttctctt 1260
   tatggttaca atgatataca ctgtttgaga tgaggataaa atactctgag tccaaaccgg 1320
   gcccctctgc taaccatgtt catgccttct tctctttcct acagctcctg ggcaacgtgc 1380
   tggttgttgt gctgtctcat cattttggca aagaattcct cgacggatcc ctcgacccaa 1440
   ctagaaccat gggtggcaag tggtcaaaaa gtagtgtggt tggatggcct actgtaaggg 1500
   aaagaatgag acgagctgag ccagcagcag atggggtggg agcagcatct cgagacctgg 1560
   aaaaacatgg agcaatcaca agtagcaata cagcagctac caatgctgct tgtgcctggc 1620
   tagaagcaca agaggaggag gaggtgggtt ttccagtcac acctcaggta cctttaagac 1680
   caatgactta caaggcagct gtagatctta gccactttct taagcgaaag gggggactgg 1740
   aagggctaat tcactcccaa cgaagacaag atatccttga tctgtggatc taccacacac 1800
   aaggctactt ccctgattgg cagaactaca caccagggcc aggggtcaga tatccactga 1860
   cctttggatg gtgctacaag ctagtaccag ttgagccaga taaggtagaa gaggccaata 1920
   aaggagagaa caccagcttg ttacaccctg tgagcctgca tggaatggat gaccctgaga 1980
   gagaagtgtt agagtggagg tttgacagcc gcctagcatt tcatcacgtg gcccgagagc 2040
   tgcatccgga gtacttcaag aactgctgaa cgcgtcaggt gcaggctgcc tatcagaagg 2100
   tggtggctgg tgtggccaat gccctggctc acaaatacca ctgagatctt tttccctctg 2160
   ccaaaaatta tggggacatc atgaagcccc ttgagcatct gacttctggc taataaagga 2220
   aatttatttt cattgcaata gtgtgttgga attttttgtg tctctcactc ggaaggacat 2280
   atgggagggc aaatcattta aaacatcaga atgagtattt ggtttagagt ttggcaacat 2340
   atgcccatat gctggctgcc atgaacaaag gttggctata aagaggtcat cagtatatga 2400
   aacagccccc tgctgtccat tccttattcc atagaaaagc cttgacttga ggttagattt 2460
   tttttatatt ttgttttgtg ttattttttt ctttaacatc cctaaaattt tccttacatg 2520
   ttttactagc cagatttttc ctcctctcct gactactccc agtcatagct gtccctcttc 2580
   tcttatggag atccctcgac ggatcggccg caattcgtaa tcatgtcata gctgtttcct 2640
   gtgtgaaatt gttatccgct cacaattcca cacaacatac gagccggaag cataaagtgt 2700
   aaagcctggg gtgcctaatg agtgagctaa ctcacattaa ttgcgttgcg ctcactgccc 2760
   gctttccagt cgggaaacct gtcgtgccag ctgcattaat gaatcggcca acgcgcgggg 2820
   agaggcggtt tgcgtattgg gcgctcttcc gcttcctcgc tcactgactc gctgcgctcg 2880
   gtcgttcggc tgcggcgagc ggtatcagct cactcaaagg cggtaatacg gttatccaca 2940
   gaatcagggg ataacgcagg aaagaacatg tgagcaaaag gccagcaaaa ggccaggaac 3000
   cgtaaaaagg ccgcgttgct ggcgtttttc cataggctcc gcccccctga cgagcatcac 3060
   aaaaatcgac gctcaagtca gaggtggcga aacccgacag gactataaag ataccaggcg 3120
   tttccccctg gaagctccct cgtgcgctct cctgttccga ccctgccgct taccggatac 3180
   ctgtccgcct ttctcccttc gggaagcgtg gcgctttctc atagctcacg ctgtaggtat 3240
   ctcagttcgg tgtaggtcgt tcgctccaag ctgggctgtg tgcacgaacc ccccgttcag 3300
   cccgaccgct gcgccttatc cggtaactat cgtcttgagt ccaacccggt aagacacgac 3360
   ttatcgccac tggcagcagc cactggtaac aggattagca gagcgaggta tgtaggcggt 3420
   gctacagagt tcttgaagtg gtggcctaac tacggctaca ctagaagaac agtatttggt 3480
   atctgcgctc tgctgaagcc agttaccttc ggaaaaagag ttggtagctc ttgatccggc 3540
   aaacaaacca ccgctggtag cggtggtttt tttgtttgca agcagcagat tacgcgcaga 3600
   aaaaaaggat ctcaagaaga tcctttgatc ttttctacgg ggtctgacgc tcagtggaac 3660
   gaaaactcac gttaagggat tttggtcatg agattatcaa aaaggatctt cacctagatc 3720
   cttttaaatt aaaaatgaag ttttaaatca atctaaagta tatatgagta aacttggtct 3780
   gacagttacc aatgcttaat cagtgaggca cctatctcag cgatctgtct atttcgttca 3840
   tccatagttg cctgactccc cgtcgtgtag ataactacga tacgggaggg cttaccatct 3900
   ggccccagtg ctgcaatgat accgcgagac ccacgctcac cggctccaga tttatcagca 3960
   ataaaccagc cagccggaag ggccgagcgc agaagtggtc ctgcaacttt atccgcctcc 4020
   atccagtcta ttaattgttg ccgggaagct agagtaagta gttcgccagt taatagtttg 4080
   cgcaacgttg ttgccattgc tacaggcatc gtggtgtcac gctcgtcgtt tggtatggct 4140
   tcattcagct ccggttccca acgatcaagg cgagttacat gatcccccat gttgtgcaaa 4200
   aaagcgggtt agctccttcg gtcctccgat cgttgtcaga agtaagttgg ccgcagtgtt 4260
   atcactcatg gttatggcag cactgcataa ttctcttact gtcatgccat ccgtaagatg 4320
   cttttctgtg actggtgagt actcaaccaa gtcattctga gaatagtgta tgcggcgacc 4380
   gagttgctct tgcccggcgt caatacggga taataccgcg ccacatagca gaactttaaa 4440
   agtgctcatc attggaaaac gttcttcggg gcgaaaactc tcaaggatct taccgctgtt 4500
   gagatccagt tcgatgtaac ccactcgtgc acccaactga tcttcagcat cttttacttt 4560
   caccagcgtt tctgggtgag caaaaacagg aaggcaaaat gccgcaaaaa agggaataag 4620
   ggcgacacgg aaatgttgaa tactcatact cttccttttt caatattatt gaagcattta 4680
   tcagggttat tgtctcatga gcggatacat atttgaatgt atttagaaaa ataaacaaat 4740
   aggggttccg cgcacatttc cccgaaaagt gccacctaaa ttgtaagcgt taatattttg 4800
   ttaaaattcg cgttaaattt ttgttaaatc agctcatttt ttaaccaata ggccgaaatc 4860
   ggcaaaatcc cttataaatc aaaagaatag accgagatag ggttgagtgt tgttccagtt 4920
   tggaacaaga gtccactatt aaagaacgtg gactccaacg tcaaagggcg aaaaaccgtc 4980
   tatcagggcg atggcccact acgtgaacca tcaccctaat caagtttttt ggggtcgagg 5040
   tgccgtaaag cactaaatcg gaaccctaaa gggagccccc gatttagagc ttgacgggga 5100
   aagccggcga acgtggcgag aaaggaaggg aagaaagcga aaggagcggg cgctagggcg 5160
   ctggcaagtg tagcggtcac gctgcgcgta accaccacac ccgccgcgct taatgcgccg 5220
   ctacagggcg cgtcccattc gccattcagg ctgcgcaact gttgggaagg gcgatcggtg 5280
   cgggcctctt cgctattacg ccagctggcg aaagggggat gtgctgcaag gcgattaagt 5340
   tgggtaacgc cagggttttc ccagtcacga cgttgtaaaa cgacggccag tgagcgcgcg 5400
   taatacgact cactataggg cgaattggag ctccaccgcg gtg 5443
<210> 22
   <211> 43
   <212> DNA
   <213> Artificial sequence
<220>
   <223> PCR primer
<400> 22
   atacatggcc cagccggccg gtggcaagtg gtcaaaaagt agt 43
<210> 23
   <211> 39
   <212> DNA
   <213> Artificial sequence
<220>
   <223> PCR primer
<400> 23
   atacatacgc gttcagtggc taagatctac agctgcctt 39
<210> 24
   <211> 46
   <212> DNA
   <213> Artificial sequence
<220>
   <223> PCR primer
<400> 24
   atacatggcc cagccggcca cttacaaggc agctgtagat cttagc 46
<210> 25
   <211> 38
   <212> DNA
   <213> Artificial sequence
<220>
   <223> PCR primer
<400> 25
   atacatacgt cgttcagcct tgtgtgtggt agatccac 38
<210> 26
   <211> 46
   <212> DNA
   <213> Artificial sequence
<220>
   <223> PCR primer
<400> 26
   atacatggcc cagccggccg atatccttga tctgtggatc taccac 46
<210> 27
   <211> 37
   <212> DNA
   <213> Artificial sequence
<220>
   <223> PCR primer
<400> 27
   atacataacg cgttcagcag ttcttgaagt actccgg 37
<210> 28
   <211> 206
   <212> PRT
   <213> Human immunodeficiency virus type 1
<400> 28
   Met Gly Gly Lys Trp Ser Lys Cys Ser Gly Gly Gly Trp Ser Thr Ile
   1               5                   10                  15
   Arg Glu Arg Met Arg Arg Ala Glu Pro Ala Ala Asp Gly Val Gly Pro
               20                  25                  30
   Val Ser Arg Asp Leu Glu Lys His Gly Ala Val Thr Ser Ser Asn Thr
           35                  40                  45
   Ala Thr Asn Asn Ala Asp Cys Ala Trp Leu Glu Ala Gln Glu Asp Glu
       50                  55                  60
   Asp Val Gly Phe Pro Val Arg Pro Gln Val Pro Leu Arg Pro Met Thr
   65                  70                  75                  80
   Tyr Lys Ala Ala Leu Asp Leu Ser His Phe Leu Lys Glu Lys Gly Gly
                   85                  90                  95
   Leu Glu Gly Leu Ile His Ser Gln Lys Arg Gln Asp Ile Leu Asp Leu
               100                 105                 110
   Trp Val Tyr His Thr Gln Gly Tyr Phe Pro Ser Trp Gln Asn Tyr Thr
           115                 120                 125
   Pro Gly Pro Gly Thr Arg Tyr Pro Leu Thr Tyr Gly Trp Cys Phe Lys
       130                 135                 140
   Leu Leu Pro Val Glu Pro Glu Lys Val Glu Glu Ala Asn Glu Gly Glu
   145                 150                 155                 160
   Asn Asn Cys Leu Leu His Pro Met Ser Gln His Gly Met Glu Asp Pro
                   165                 170                 175
   Glu Arg Glu Val Leu Val Trp Lys Phe Asp Ser Arg Leu Ala Phe His
               180                 185                 190
   His Met Ala Arg Glu Leu His Pro Glu Tyr Tyr Lys Asn Cys
           195                 200                 205
<210> 29
   <211> 255
   <212> PRT
   <213> Human immunodeficiency virus type 2
<400> 29
   Met Gly Ala Ser Gly Ser Lys Lys Arg Ser Glu Pro Ser Arg Gly Leu
   1               5                   10                  15
   Arg Glu Arg Leu Leu Gln Thr Pro Gly Glu Ala Ser Gly Gly His Trp
               20                  25                  30
   Asp Lys Leu Gly Gly Glu Tyr Leu Gln Ser Gln Glu Gly Ser Gly Arg
           35                  40                  45
   Gly Gln Lys Ser Pro Ser Cys Glu Gly Arg Arg Tyr Gln Gln Gly Asp
       50                  55                  60
   Phe Met Asn Thr Pro Trp Arg Ala Pro Ala Glu Gly Glu Lys Gly Ser
   65                  70                  75                  80
   Tyr Lys Gln Gln Asn Met Asp Asp Val Asp Ser Asp Asp Asp Asp Leu
                   85                  90                  95
   Val Gly Val Pro Val Thr Pro Arg Val Pro Leu Arg Glu Met Thr Tyr
               100                 105                 110
   Arg Leu Ala Arg Asp Met Ser His Leu Ile Lys Glu Lys Gly Gly Leu
           115                 120                 125
   Glu Gly Leu Tyr Tyr Ser Asp Arg Arg Arg Arg Val Leu Asp Ile Tyr
       130                 135                 140
   Leu Glu Lys Glu Glu Gly Ile Ile Gly Asp Trp Gln Asn Tyr Thr His
   145                 150                 155                 160
   Gly Pro Gly Val Arg Tyr Pro Lys Phe Phe Gly Trp Leu Trp Lys Leu
                   165                 170                 175
   Val Pro Val Asp Val Pro Gln Glu Gly Asp Asp Ser Glu Thr His Cys
               180                 185                 190
   Leu Val His Pro Ala Gln Thr Ser Arg Phe Asp Asp Pro His Gly Glu
           195                 200                 205
   Thr Leu Val Trp Arg Phe Asp Pro Thr Leu Ala Phe Ser Tyr Glu Ala
       210                 215                 220
   Phe Ile Arg Tyr Pro Glu Glu Phe Gly Tyr Lys Ser Gly Leu Pro Glu
   225                 230                 235                 240
   Asp Glu Trp Lys Ala Arg Leu Lys Ala Arg Gly Ile Pro Phe Ser
                   245                 250                 255
<210> 30
   <211> 263
   <212> PRT
   <213> Simian immunodeficiency virus
<400> 30
   Met Gly Gly Ala Ile Ser Met Arg Arg Ser Arg Pro Ser Gly Asp Leu
   1 5 10 15
   Arg Gln Arg Leu Leu Arg Ala Arg Gly Glu Thr Tyr Gly Arg Leu Leu
               20                  25                  30
   Gly Glu Val Glu Asp Gly Tyr Ser Gln Ser Pro Gly Gly Leu Asp Lys
           35                  40                  45
   Gly Leu Ser Ser Leu Ser Cys Glu Gly Gln Lys Tyr Asn Gln Gly Gln
       50                  55                  60
   Tyr Met Asn Thr Pro Trp Arg Asn Pro Ala Glu Glu Arg Glu Lys Leu
   65                  70                  75                  80
   Ala Tyr Arg Lys Gln Asn Met Asp Asp Ile Asp Glu Glu Asp Asp Asp
                   85                  90                  95
   Leu Val Gly Val Ser Val Arg Pro Lys Val Pro Leu Arg Thr Met Ser
               100                 105                 110
   Tyr Lys Leu Ala Ile Asp Met Ser His Phe Ile Lys Glu Lys Gly Gly
           115                 120                 125
   Leu Glu Gly Ile Tyr Tyr Ser Ala Arg Arg His Arg Ile Leu Asp Ile
       130                 135                 140
   Tyr Leu Glu Lys Glu Glu Gly Ile Ile Pro Asp Trp Gln Asp Tyr Thr
   145                 150                 155                 160
   Ser Gly Pro Gly Ile Arg Tyr Pro Lys Thr Phe Gly Trp Leu Trp Lys
                   165                 170                 175
   Leu Val Pro Val Asn Val Ser Asp Glu Ala Gln Glu Asp Glu Glu His
               180                 185                 190
   Tyr Leu Met His Pro Ala Gln Thr Ser Gln Trp Asp Asp Pro Trp Gly
           195                 200                 205
   Glu Val Leu Ala Trp Lys Phe Asp Pro Thr Leu Ala Tyr Thr Tyr Glu
       210                 215                 220
   Ala Tyr Val Arg Tyr Pro Glu Glu Phe Gly Ser Lys Ser Gly Leu Ser
   225                 230                 235                 240
   Glu Glu Glu Val Arg Arg Arg Leu Thr Ala Arg Gly Leu Leu Asn Met
                   245                 250                 255
   Ala Asp Lys Lys Glu Thr Arg
               260
<210> 31
   <211> 263
   <212> PRT
   <213> Artificial sequence
<220>
   <223> E125R SIV Nef mutant
<400> 31
   Met Gly Gly Ala Ile Ser Met Arg Arg Ser Arg Pro Ser Gly Asp Leu
   1               5                   10                  15
   Arg Gln Arg Leu Leu Arg Ala Arg Gly Glu Thr Tyr Gly Arg Leu Leu
               20                  25                  30
   Gly Glu Val Glu Asp Gly Tyr Ser Gln Ser Pro Gly Gly Leu Asp Lys
           35                  40                  45
   Gly Leu Ser Ser Leu Ser Cys Glu Gly Gln Lys Tyr Asn Gln Gly Gln
       50                  55                  60
   Tyr Met Asn Thr Pro Trp Arg Asn Pro Ala Glu Glu Arg Glu Lys Leu
   65                  70                  75                  80
   Ala Tyr Arg Lys Gln Asn Met Asp Asp Ile Asp Glu Glu Asp Asp Asp
                   85                  90                  95
   Leu Val Gly Val Ser Val Arg Pro Lys Val Pro Leu Arg Thr Met Ser
               100                 105                 110
   Tyr Lys Leu Ala Ile Asp Met Ser His Phe Ile Lys Arg Lys Gly Gly
           115                 120                 125
   Leu Glu Gly Ile Tyr Tyr Ser Ala Arg Arg His Arg Ile Leu Asp Ile
       130                 135                 140
   Tyr Leu Glu Lys Glu Glu Gly Ile Ile Pro Asp Trp Gln Asp Tyr Thr
   145                 150                 155                 160
   Ser Gly Pro Gly Ile Arg Tyr Pro Lys Thr Phe Gly Trp Leu Trp Lys
                   165                 170                 175
   Leu Val Pro Val Asn Val Ser Asp Glu Ala Gln Glu Asp Glu Glu His
               180                 185                 190
   Tyr Leu Met His Pro Ala Gln Thr Ser Gln Trp Asp Asp Pro Trp Gly
           195                 200                 205
   Glu Val Leu Ala Trp Lys Phe Asp Pro Thr Leu Ala Tyr Thr Tyr Glu
       210                 215                 220
   Ala Tyr Val Arg Tyr Pro Glu Glu Phe Gly Ser Lys Ser Gly Leu Ser
   225 230 235 240
   Glu Glu Glu Val Arg Arg Arg Leu Thr Ala Arg Gly Leu Leu Asn Met
   245 250 255
   Ala Asp Lys Lys Glu Thr Arg
   260
<210> 32
   <211> 41
   <212> PRT
   <213> Artificial sequence
<220>
   <223> 80-120 HIV-1 Nef fragment
<400> 32
   Thr Tyr Lys Ala Ala Val Asp Leu Ser His Phe Leu Lys Glu Lys Gly
   1 5 10 15
   Gly Leu Glu Gly Leu Ile His Ser Gln Arg Arg Gln Asp Ile Leu Asp
   20 25 30
   Leu Trp Ile Tyr His Thr Gln Gly Tyr
   35 40
<210> 33
   <211> 40
   <212> PRT
   <213> Artificial sequence
<220>
   <223> 81-120 HIV-1 Nef fragment
<400> 33
   Tyr Lys Ala Ala Val Asp Leu Ser His Phe Leu Lys Glu Lys Gly Gly
   1 5 10 15
   Leu Glu Gly Leu Ile His Ser Gln Arg Arg Gln Asp Ile Leu Asp Leu
   20 25 30
   Trp Ile Tyr His Thr Gln Gly Tyr
   35 40
<210> 34
   <211> 39
   <212> PRT
   <213> Artificial sequence
<220>
   <223> 82-120 HIV-1 Nef fragment
<400> 34
   Lys Ala Ala Val Asp Leu Ser His Phe Leu Lys Glu Lys Gly Gly Leu
   1               5                   10                  15
   Glu Gly Leu Ile His Ser Gln Arg Arg Gln Asp Ile Leu Asp Leu Trp
               20                  25                  30
   Ile Tyr His Thr Gln Gly Tyr
           35
<210> 35
   <211> 38
   <212> PRT
   <213> Artificial sequence
<220>
   <223> 83-120 HIV-1 Nef fragment
<400> 35
   Ala Ala Val Asp Leu Ser His Phe Leu Lys Glu Lys Gly Gly Leu Glu
   1               5                   10                  15
   Gly Leu Ile His Ser Gln Arg Arg Gln Asp Ile Leu Asp Leu Trp Ile
               20                  25                  30
   Tyr His Thr Gln Gly Tyr
           35
<210> 36
   <211> 37
   <212> PRT
   <213> Artificial sequence
<220>
   <223> 84-120 HIV-1 Nef fragment
<400> 36
   Ala Val Asp Leu Ser His Phe Leu Lys Glu Lys Gly Gly Leu Glu Gly
   1               5                   10                  15
   Leu Ile His Ser Gln Arg Arg Gln Asp Ile Leu Asp Leu Trp Ile Tyr
               20                  25                  30
   His Thr Gln Gly Tyr
           35
<210> 37
   <211> 36
   <212> PRT
   <213> Artificial sequence
<220>
   <223> 85-120 HIV-1 Nef fragment
<400> 37
   Val Asp Leu Ser His Phe Leu Lys Glu Lys Gly Gly Leu Glu Gly Leu
   1               5                   10                  15
   Ile His Ser Gln Arg Arg Gln Asp Ile Leu Asp Leu Trp Ile Tyr His
               20                  25                  30
   Thr Gln Gly Tyr
           35
<210> 38
   <211> 35
   <212> PRT
   <213> Artificial sequence
<220>
   <223> 86-120 HIV-1 Nef fragment
<400> 38
   Asp Leu Ser His Phe Leu Lys Glu Lys Gly Gly Leu Glu Gly Leu Ile
   1               5                   10                  15
   His Ser Gln Arg Arg Gln Asp Ile Leu Asp Leu Trp Ile Tyr His Thr
               20                  25                  30
   Gln Gly Tyr
           35
<210> 39
   <211> 34
   <212> PRT
   <213> Artificial sequence
<220>
   <223> 87-120 HIV-1 Nef fragment
<400> 39
   Leu Ser His Phe Leu Lys Glu Lys Gly Gly Leu Glu Gly Leu Ile His
   1               5                   10                  15
   Ser Gln Arg Arg Gln Asp Ile Leu Asp Leu Trp Ile Tyr His Thr Gln
               20                  25                  30
   Gly Tyr
<210> 40
   <211> 33
   <212> PRT
   <213> Artificial sequence
<220>
   <223> 88-120 HIV-1 Nef fragment
<400> 40
   Ser His Phe Leu Lys Glu Lys Gly Gly Leu Glu Gly Leu Ile His Ser
   1               5                   10                  15
   Gln Arg Arg Gln Asp Ile Leu Asp Leu Trp Ile Tyr His Thr Gln Gly
               20                  25                  30
   Tyr
<210> 41
   <211> 32
   <212> PRT
   <213> Artificial sequence
<220>
   <223> 89-120 HIV-1 Nef fragment
<400> 41
   His Phe Leu Lys Glu Lys Gly Gly Leu Glu Gly Leu Ile His Ser Gln
   1               5                   10                  15
   Arg Arg Gln Asp Ile Leu Asp Leu Trp Ile Tyr His Thr Gln Gly Tyr
               20                  25                  30
<210> 42
   <211> 31
   <212> PRT
   <213> Artificial sequence
<220>
   <223> 90-120 HIV-1 Nef fragment
<400> 42
   Phe Leu Lys Glu Lys Gly Gly Leu Glu Gly Leu Ile His Ser Gln Arg
   1 5 10 15
   Arg Gln Asp Ile Leu Asp Leu Trp Ile Tyr His Thr Gln Gly Tyr
   20 25 30
<210> 43
   <211> 40
   <212> PRT
   <213> Artificial sequence
<220>
   <223> 80-119 HIV-1 Nef fragment
<400> 43
   Thr Tyr Lys Ala Ala Val Asp Leu Ser His Phe Leu Lys Glu Lys Gly
   1 5 10 15
   Gly Leu Glu Gly Leu Ile His Ser Gln Arg Arg Gln Asp Ile Leu Asp
   20 25 30
   Leu Trp Ile Tyr His Thr Gln Gly
   35 40
<210> 44
   <211> 39
   <212> PRT
   <213> Artificial sequence
<220>
   <223> 81-119 HIV-1 Nef fragment
<400> 44
   Tyr Lys Ala Ala Val Asp Leu Ser His Phe Leu Lys Glu Lys Gly Gly
   1 5 10 15
   Leu Glu Gly Leu Ile His Ser Gln Arg Arg Gln Asp Ile Leu Asp Leu
   20 25 30
   Trp Ile Tyr His Thr Gln Gly
   35
<210> 45
   <211> 38
   <212> PRT
   <213> Artificial sequence
<220>
   <223> 82-119 HIV-1 Nef fragment
<400> 45
   Lys Ala Ala Val Asp Leu Ser His Phe Leu Lys Glu Lys Gly Gly Leu
   1 5 10 15
   Glu Gly Leu Ile His Ser Gln Arg Arg Gln Asp Ile Leu Asp Leu Trp
   20 25 30
   Ile Tyr His Thr Gln Gly
   35
<210> 46
   <211> 37
   <212> PRT
   <213> Artificial sequence
<220>
   <223> 83-119 HIV-1 Nef fragment
<400> 46
   Ala Ala Val Asp Leu Ser His Phe Leu Lys Glu Lys Gly Gly Leu Glu
   1 5 10 15
   Gly Leu Ile His Ser Gln Arg Arg Gln Asp Ile Leu Asp Leu Trp Ile
   20 25 30
   Tyr His Thr Gln Gly
   35
<210> 47
   <211> 36
   <212> PRT
   <213> Artificial sequence
<220>
   <223> 84-119 HIV-1 Nef fragment
<400> 47
   Ala Val Asp Leu Ser His Phe Leu Lys Glu Lys Gly Gly Leu Glu Gly
   1 5 10 15
   Leu Ile His Ser Gln Arg Arg Gln Asp Ile Leu Asp Leu Trp Ile Tyr
   20 25 30
   His Thr Gln Gly
   35
<210> 48
   <211> 35
   <212> PRT
   <213> Artificial sequence
<220>
   <223> 85-119 HIV-1 Nef fragment
<400> 48
   Val Asp Leu Ser His Phe Leu Lys Glu Lys Gly Gly Leu Glu Gly Leu
   1               5                   10                  15
   Ile His Ser Gln Arg Arg Gln Asp Ile Leu Asp Leu Trp Ile Tyr His
               20                  25                  30
   Thr Gln Gly
           35
<210> 49
   <211> 34
   <212> PRT
   <213> Artificial sequence
<220>
   <223> 86-119 HIV-1 Nef fragment
<400> 49
   Asp Leu Ser His Phe Leu Lys Glu Lys Gly Gly Leu Glu Gly Leu Ile
   1               5                   10                  15
   His Ser Gln Arg Arg Gln Asp Ile Leu Asp Leu Trp Ile Tyr His Thr
               20                  25                  30
   Gln Gly
<210> 50
   <211> 33
   <212> PRT
   <213> Artificial sequence
<220>
   <223> 87-119 HIV-1 Nef fragment
<400> 50
   Leu Ser His Phe Leu Lys Glu Lys Gly Gly Leu Glu Gly Leu Ile His
   1               5                   10                  15
   Ser Gln Arg Arg Gln Asp Ile Leu Asp Leu Trp Ile Tyr His Thr Gln
               20                  25                  30
   Gly
<210> 51
   <211> 32
   <212> PRT
   <213> Artificial sequence
<220>
   <223> 88-119 HIV-1 Nef fragment
<400> 51
   Ser His Phe Leu Lys Glu Lys Gly Gly Leu Glu Gly Leu Ile His Ser
   1               5                   10                  15
   Gln Arg Arg Gln Asp Ile Leu Asp Leu Trp Ile Tyr His Thr Gln Gly
               20                  25                  30
<210> 52
   <211> 31
   <212> PRT
   <213> Artificial sequence
<220>
   <223> 89-119 HIV-1 Nef fragment
<400> 52
   His Phe Leu Lys Glu Lys Gly Gly Leu Glu Gly Leu Ile His Ser Gln
   1               5                   10                  15
   Arg Arg Gln Asp Ile Leu Asp Leu Trp Ile Tyr His Thr Gln Gly
               20                  25                  30
<210> 53
   <211> 30
   <212> PRT
   <213> Artificial sequence
<220>
   <223> 90-119 HIV-1 Nef fragment
<400> 53
   Phe Leu Lys Glu Lys Gly Gly Leu Glu Gly Leu Ile His Ser Gln Arg
   1               5                   10                  15
   Arg Gln Asp Ile Leu Asp Leu Trp Ile Tyr His Thr Gln Gly
               20                  25                  30
<210> 54
   <211> 39
   <212> PRT
   <213> Artificial sequence
<220>
   <223> 80-118 HIV-1 Nef fragment
<400> 54
   Thr Tyr Lys Ala Ala Val Asp Leu Ser His Phe Leu Lys Glu Lys Gly
   1               5                   10                  15
   Gly Leu Glu Gly Leu Ile His Ser Gln Arg Arg Gln Asp Ile Leu Asp
               20                  25                  30
   Leu Trp Ile Tyr His Thr Gln
           35
<210> 55
   <211> 38
   <212> PRT
   <213> Artificial sequence
<220>
   <223> 81-118 HIV-1 Nef fragment
<400> 55
   Tyr Lys Ala Ala Val Asp Leu Ser His Phe Leu Lys Glu Lys Gly Gly
   1               5                   10                  15
   Leu Glu Gly Leu Ile His Ser Gln Arg Arg Gln Asp Ile Leu Asp Leu
               20                  25                  30
   Trp Ile Tyr His Thr Gln
           35
<210> 56
   <211> 37
   <212> PRT
   <213> Artificial sequence
<220>
   <223> 82-118 HIV-1 Nef fragment
<400> 56
   Lys Ala Ala Val Asp Leu Ser His Phe Leu Lys Glu Lys Gly Gly Leu
   1               5                   10                  15
   Glu Gly Leu Ile His Ser Gln Arg Arg Gln Asp Ile Leu Asp Leu Trp
               20                  25                  30
   Ile Tyr His Thr Gln
           35
<210> 57
   <211> 36
   <212> PRT
   <213> Artificial sequence
<220>
   <223> 83-118 HIV-1 Nef fragment
<400> 57
   Ala Ala Val Asp Leu Ser His Phe Leu Lys Glu Lys Gly Gly Leu Glu
   1               5                   10                  15
   Gly Leu Ile His Ser Gln Arg Arg Gln Asp Ile Leu Asp Leu Trp Ile
               20                  25                  30
   Tyr His Thr Gln
           35
<210> 58
   <211> 35
   <212> PRT
   <213> Artificial sequence
<220>
   <223> 84-118 HIV-1 Nef fragment
<400> 58
   Ala Val Asp Leu Ser His Phe Leu Lys Glu Lys Gly Gly Leu Glu Gly
   1               5                   10                  15
   Leu Ile His Ser Gln Arg Arg Gln Asp Ile Leu Asp Leu Trp Ile Tyr
               20                  25                  30
   His Thr Gln
                   35
<210> 59
   <211> 34
   <212> PRT
   <213> Artificial sequence
<220>
   <223> 85-118 HIV-1 Nef fragment
<400> 59
   Val Asp Leu Ser His Phe Leu Lys Glu Lys Gly Gly Leu Glu Gly Leu
   1               5                   10                  15
   Ile His Ser Gln Arg Arg Gln Asp Ile Leu Asp Leu Trp Ile Tyr His
               20                  25                  30
   Thr Gln
<210> 60
   <211> 33
   <212> PRT
   <213> Artificial sequence
<220>
   <223> 86-118 HIV-1 Nef fragment
<400> 60
   Asp Leu Ser His Phe Leu Lys Glu Lys Gly Gly Leu Glu Gly Leu Ile
   1               5                   10                  15
   His Ser Gln Arg Arg Gln Asp Ile Leu Asp Leu Trp Ile Tyr His Thr
               20                  25                  30
   Gln
<210> 61
   <211> 32
   <212> PRT
   <213> Artificial sequence
<220>
   <223> 87-118 HIV-1 Nef fragment
<400> 61
   Leu Ser His Phe Leu Lys Glu Lys Gly Gly Leu Glu Gly Leu Ile His
   1               5                   10                  15
   Ser Gln Arg Arg Gln Asp Ile Leu Asp Leu Trp Ile Tyr His Thr Gln
               20                  25                  30
<210> 62
   <211> 31
   <212> PRT
   <213> Artificial sequence
<220>
   <223> 88-118 HIV-1 Nef fragment
<400> 62
   Ser His Phe Leu Lys Glu Lys Gly Gly Leu Glu Gly Leu Ile His Ser
   1               5                   10                  15
   Gln Arg Arg Gln Asp Ile Leu Asp Leu Trp Ile Tyr His Thr Gln
               20                  25                  30
<210> 63
   <211> 30
   <212> PRT
   <213> Artificial sequence
<220>
   <223> 89-118 HIV-1 Nef fragment
<400> 63
   His Phe Leu Lys Glu Lys Gly Gly Leu Glu Gly Leu Ile His Ser Gln
   1               5                   10                  15
   Arg Arg Gln Asp Ile Leu Asp Leu Trp Ile Tyr His Thr Gln
               20                  25                  30
<210> 64
   <211> 29
   <212> PRT
   <213> Artificial sequence
<220>
   <223> 90-118 HIV-1 Nef fragment
<400> 64
   Phe Leu Lys Glu Lys Gly Gly Leu Glu Gly Leu Ile His Ser Gln Arg
   1               5                   10                  15
   Arg Gln Asp Ile Leu Asp Leu Trp Ile Tyr His Thr Gln
               20                  25
<210> 65
   <211> 38
   <212> PRT
   <213> Artificial sequence
<220>
   <223> 80-117 HIV-1 Nef fragment
<400> 65
   Thr Tyr Lys Ala Ala Val Asp Leu Ser His Phe Leu Lys Glu Lys Gly
   1 5 10 15
   Gly Leu Glu Gly Leu Ile His Ser Gln Arg Arg Gln Asp Ile Leu Asp
   20 25 30
   Leu Trp Ile Tyr His Thr
   35
<210> 66
   <211> 37
   <212> PRT
   <213> Artificial sequence
<220>
   <223> 81-117 HIV-1 Nef fragment
<400> 66
   Tyr Lys Ala Ala Val Asp Leu Ser His Phe Leu Lys Glu Lys Gly Gly
   1 5 10 15
   Leu Glu Gly Leu Ile His Ser Gln Arg Arg Gln Asp Ile Leu Asp Leu
   20 25 30
   Trp Ile Tyr His Thr
   35
<210> 67
   <211> 36
   <212> PRT
   <213> Artificial sequence
<220>
   <223> 82-117 HIV-1 Nef fragment
<400> 67
   Lys Ala Ala Val Asp Leu Ser His Phe Leu Lys Glu Lys Gly Gly Leu
   1 5 10 15
   Glu Gly Leu Ile His Ser Gln Arg Arg Gln Asp Ile Leu Asp Leu Trp
   20 25 30
   Ile Tyr His Thr
   35
<210> 68
   <211> 35
   <212> PRT
   <213> Artificial sequence
<220>
   <223> 83-117 HIV-1 Nef fragment
<400> 68
   Ala Ala Val Asp Leu Ser His Phe Leu Lys Glu Lys Gly Gly Leu Glu
   1 5 10 15
   Gly Leu Ile His Ser Gln Arg Arg Gln Asp Ile Leu Asp Leu Trp Ile
   20 25 30
   Tyr His Thr
   35
<210> 69
   <211> 34
   <212> PRT
   <213> Artificial sequence
<220>
   <223> 84-117 HIV-1 Nef fragment
<400> 69
   Ala Val Asp Leu Ser His Phe Leu Lys Glu Lys Gly Gly Leu Glu Gly
   1 5 10 15
   Leu Ile His Ser Gln Arg Arg Gln Asp Ile Leu Asp Leu Trp Ile Tyr
   20 25 30
   His Thr
<210> 70
   <211> 33
   <212> PRT
   <213> Artificial sequence
<220>
   <223> 85-117 HIV-1 Nef fragment
<400> 70
   Val Asp Leu Ser His Phe Leu Lys Glu Lys Gly Gly Leu Glu Gly Leu
   1 5 10 15
   Ile His Ser Gln Arg Arg Gln Asp Ile Leu Asp Leu Trp Ile Tyr His
   20 25 30
   Thr
<210> 71
   <211> 32
   <212> PRT
   <213> Artificial sequence
<220>
   <223> 86-117 HIV-1 Nef fragment
<400> 71
   Asp Leu Ser His Phe Leu Lys Glu Lys Gly Gly Leu Glu Gly Leu Ile
   1               5                   10                  15
   His Ser Gln Arg Arg Gln Asp Ile Leu Asp Leu Trp Ile Tyr His Thr
               20                  25                  30
<210> 72
   <211> 31
   <212> PRT
   <213> Artificial sequence
<220>
   <223> 87-117 HIV-1 Nef fragment
<400> 72
   Leu Ser His Phe Leu Lys Glu Lys Gly Gly Leu Glu Gly Leu Ile His
   1               5                   10                  15
   Ser Gln Arg Arg Gln Asp Ile Leu Asp Leu Trp Ile Tyr His Thr
               20                  25                  30
<210> 73
   <211> 30
   <212> PRT
   <213> Artificial sequence
<220>
   <223> 88-117 HIV-1 Nef fragment
<400> 73
   Ser His Phe Leu Lys Glu Lys Gly Gly Leu Glu Gly Leu Ile His Ser
   1               5                   10                  15
   Gln Arg Arg Gln Asp Ile Leu Asp Leu Trp Ile Tyr His Thr
               20                  25                  30
<210> 74
   <211> 29
   <212> PRT
   <213> Artificial sequence
<220>
   <223> 89-117 HIV-1 Nef fragment
<400> 74
   His Phe Leu Lys Glu Lys Gly Gly Leu Glu Gly Leu Ile His Ser Gln
   1               5                   10                  15
   Arg Arg Gln Asp Ile Leu Asp Leu Trp Ile Tyr His Thr
               20                  25
<210> 75
   <211> 28
   <212> PRT
   <213> Artificial sequence
<220>
   <223> 90-117 HIV-1 Nef fragment
<400> 75
   Phe Leu Lys Glu Lys Gly Gly Leu Glu Gly Leu Ile His Ser Gln Arg
   1               5                   10                  15
   Arg Gln Asp Ile Leu Asp Leu Trp Ile Tyr His Thr
               20                  25
<210> 76
   <211> 37
   <212> PRT
   <213> Artificial sequence
<220>
   <223> 80-116 HIV-1 Nef fragment
<400> 76
   Thr Tyr Lys Ala Ala Val Asp Leu Ser His Phe Leu Lys Glu Lys Gly
   1               5                   10                  15
   Gly Leu Glu Gly Leu Ile His Ser Gln Arg Arg Gln Asp Ile Leu Asp
               20                  25                  30
   Leu Trp Ile Tyr His
           35
<210> 77
   <211> 36
   <212> PRT
   <213> Artificial sequence
<220>
   <223> 81-116 HIV-1 Nef fragment
<400> 77
   Tyr Lys Ala Ala Val Asp Leu Ser His Phe Leu Lys Glu Lys Gly Gly
   1 5 10 15
   Leu Glu Gly Leu Ile His Ser Gln Arg Arg Gln Asp Ile Leu Asp Leu
   20 25 30
   Trp Ile Tyr His
   35
<210> 78
   <211> 35
   <212> PRT
   <213> Artificial sequence
<220>
   <223> 82-116 HIV-1 Nef fragment
<400> 78
   Lys Ala Ala Val Asp Leu Ser His Phe Leu Lys Glu Lys Gly Gly Leu
   1 5 10 15
   Glu Gly Leu Ile His Ser Gln Arg Arg Gln Asp Ile Leu Asp Leu Trp
   20 25 30
   Ile Tyr His
   35
<210> 79
   <211> 34
   <212> PRT
   <213> Artificial sequence
<220>
   <223> 83-116 HIV-1 Nef fragment
<400> 79
   Ala Ala Val Asp Leu Ser His Phe Leu Lys Glu Lys Gly Gly Leu Glu
   1 5 10 15
   Gly Leu Ile His Ser Gln Arg Arg Gln Asp Ile Leu Asp Leu Trp Ile
   20 25 30
   Tyr His
<210> 80
   <211> 33
   <212> PRT
   <213> Artificial sequence
<220>
   <223> 84-116 HIV-1 Nef fragment
<400> 80
   Ala Val Asp Leu Ser His Phe Leu Lys Glu Lys Gly Gly Leu Glu Gly
   1               5                   10                  15
   Leu Ile His Ser Gln Arg Arg Gln Asp Ile Leu Asp Leu Trp Ile Tyr
               20                  25                  30
   His
<210> 81
   <211> 32
   <212> PRT
   <213> Artificial sequence
<220>
   <223> 85-116 HIV-1 Nef fragment
<400> 81
   Val Asp Leu Ser His Phe Leu Lys Glu Lys Gly Gly Leu Glu Gly Leu
   1               5                   10                  15
   Ile His Ser Gln Arg Arg Gln Asp Ile Leu Asp Leu Trp Ile Tyr His
               20                  25                  30
<210> 82
   <211> 31
   <212> PRT
   <213> Artificial sequence
<220>
   <223> 86-116 HIV-1 Nef fragment
<400> 82
   Asp Leu Ser His Phe Leu Lys Glu Lys Gly Gly Leu Glu Gly Leu Ile
   1               5                   10                  15
   His Ser Gln Arg Arg Gln Asp Ile Leu Asp Leu Trp Ile Tyr His
               20                  25                  30
<210> 83
   <211> 30
   <212> PRT
   <213> Artificial sequence
<220>
   <223> 87-116 HIV-1 Nef fragment
<400> 83
   Leu Ser His Phe Leu Lys Glu Lys Gly Gly Leu Glu Gly Leu Ile His
   1               5                   10                  15
   Ser Gln Arg Arg Gln Asp Ile Leu Asp Leu Trp Ile Tyr His
               20                  25                  30
<210> 84
   <211> 29
   <212> PRT
   <213> Artificial sequence
<220>
   <223> 88-116 HIV-1 Nef fragment
<400> 84
   Ser His Phe Leu Lys Glu Lys Gly Gly Leu Glu Gly Leu Ile His Ser
   1               5                   10                  15
   Gln Arg Arg Gln Asp Ile Leu Asp Leu Trp Ile Tyr His
                       20                  25
<210> 85
   <211> 28
   <212> PRT
   <213> Artificial sequence
<220>
   <223> 89-116 HIV-1 Nef fragment
<400> 85
   His Phe Leu Lys Glu Lys Gly Gly Leu Glu Gly Leu Ile His Ser Gln
   1               5                   10                  15
   Arg Arg Gln Asp Ile Leu Asp Leu Trp Ile Tyr His
               20                  25
<210> 86
   <211> 27
   <212> PRT
   <213> Artificial sequence
<220>
   <223> 90-116 HIV-1 Nef fragment
<400> 86
   Phe Leu Lys Glu Lys Gly Gly Leu Glu Gly Leu Ile His Ser Gln Arg
   1               5                   10                  15
   Arg Gln Asp Ile Leu Asp Leu Trp Ile Tyr His
               20                  25
<210> 87
   <211> 36
   <212> PRT
   <213> Artificial sequence
<220>
   <223> 80-115 HIV-1 Nef fragment
<400> 87
   Thr Tyr Lys Ala Ala Val Asp Leu Ser His Phe Leu Lys Glu Lys Gly
   1               5                   10                  15
   Gly Leu Glu Gly Leu Ile His Ser Gln Arg Arg Gln Asp Ile Leu Asp
               20                  25                  30
   Leu Trp Ile Tyr
           35
<210> 88
   <211> 35
   <212> PRT
   <213> Artificial sequence
<220>
   <223> 81-115 HIV-1 Nef fragment
<400> 88
   Tyr Lys Ala Ala Val Asp Leu Ser His Phe Leu Lys Glu Lys Gly Gly
   1               5                   10                  15
   Leu Glu Gly Leu Ile His Ser Gln Arg Arg Gln Asp Ile Leu Asp Leu
               20                  25                  30
   Trp Ile Tyr
           35
<210> 89
   <211> 34
   <212> PRT
   <213> Artificial sequence
<220>
   <223> 82-115 HIV-1 Nef fragment
<400> 89
   Lys Ala Ala Val Asp Leu Ser His Phe Leu Lys Glu Lys Gly Gly Leu
   1               5                   10                  15
   Glu Gly Leu Ile His Ser Gln Arg Arg Gln Asp Ile Leu Asp Leu Trp
               20                  25                  30
   Ile Tyr
<210> 90
   <211> 33
   <212> PRT
   <213> Artificial sequence
<220>
   <223> 83-115 HIV-1 Nef fragment
<400> 90
   Ala Ala Val Asp Leu Ser His Phe Leu Lys Glu Lys Gly Gly Leu Glu
   1               5                   10                  15
   Gly Leu Ile His Ser Gln Arg Arg Gln Asp Ile Leu Asp Leu Trp Ile
               20                  25                  30
   Tyr
<210> 91
   <211> 32
   <212> PRT
   <213> Artificial sequence
<220>
   <223> 84-115 HIV-1 Nef fragment
<400> 91
   Ala Val Asp Leu Ser His Phe Leu Lys Glu Lys Gly Gly Leu Glu Gly
   1               5                   10                  15
   Leu Ile His Ser Gln Arg Arg Gln Asp Ile Leu Asp Leu Trp Ile Tyr
               20                  25                  30
<210> 92
   <211> 31
   <212> PRT
   <213> Artificial sequence
<220>
   <223> 85-115 HIV-1 Nef fragment
<400> 92
   Val Asp Leu Ser His Phe Leu Lys Glu Lys Gly Gly Leu Glu Gly Leu
   1 5 10 15
   Ile His Ser Gln Arg Arg Gln Asp Ile Leu Asp Leu Trp Ile Tyr
   20 25 30
<210> 93
   <211> 30
   <212> PRT
   <213> Artificial sequence
<220>
   <223> 86-115 HIV-1 Nef fragment
<400> 93
   Asp Leu Ser His Phe Leu Lys Glu Lys Gly Gly Leu Glu Gly Leu Ile
   1 5 10 15
   His Ser Gln Arg Arg Gln Asp Ile Leu Asp Leu Trp Ile Tyr
   20 25 30
<210> 94
   <211> 29
   <212> PRT
   <213> Artificial sequence
<220>
   <223> 87-115 HIV-1 Nef fragment
<400> 94
   Leu Ser His Phe Leu Lys Glu Lys Gly Gly Leu Glu Gly Leu Ile His
   1 5 10 15
   Ser Gln Arg Arg Gln Asp Ile Leu Asp Leu Trp Ile Tyr
   20 25
<210> 95
   <211> 28
   <212> PRT
   <213> Artificial sequence
<220>
   <223> 88-115 HIV-1 Nef fragment
<400> 95
   Ser His Phe Leu Lys Glu Lys Gly Gly Leu Glu Gly Leu Ile His Ser
   1 5 10 15
   Gln Arg Arg Gln Asp Ile Leu Asp Leu Trp Ile Tyr
   20 25
<210> 96
   <211> 27
   <212> PRT
   <213> Artificial sequence
<220>
   <223> 89-115 HIV-1 Nef fragment
<400> 96
   His Phe Leu Lys Glu Lys Gly Gly Leu Glu Gly Leu Ile His Ser Gln
   1 5 10 15
   Arg Arg Gln Asp Ile Leu Asp Leu Trp Ile Tyr
   20 25
<210> 97
   <211> 26
   <212> PRT
   <213> Artificial sequence
<220>
   <223> 90-115 HIV-1 Nef fragment
<400> 97
   Phe Leu Lys Glu Lys Gly Gly Leu Glu Gly Leu Ile His Ser Gln Arg
   1 5 10 15
   Arg Gln Asp Ile Leu Asp Leu Trp Ile Tyr
   20 25
<210> 98
   <211> 35
   <212> PRT
   <213> Artificial sequence
<220>
   <223> 80-114 HIV-1 Nef fragment
<400> 98
   Thr Tyr Lys Ala Ala Val Asp Leu Ser His Phe Leu Lys Glu Lys Gly
   1 5 10 15
   Gly Leu Glu Gly Leu Ile His Ser Gln Arg Arg Gln Asp Ile Leu Asp
   20 25 30
   Leu Trp Ile
   35
<210> 99
   <211> 34
   <212> PRT
   <213> Artificial sequence
<220>
   <223> 81-114 HIV-1 Nef fragment
<400> 99
   Tyr Lys Ala Ala Val Asp Leu Ser His Phe Leu Lys Glu Lys Gly Gly
   1               5                   10                  15
   Leu Glu Gly Leu Ile His Ser Gln Arg Arg Gln Asp Ile Leu Asp Leu
               20                  25                  30
   Trp Ile
<210> 100
   <211> 33
   <212> PRT
   <213> Artificial sequence
<220>
   <223> 82-114 HIV-1 Nef fragment
<400> 100
   Lys Ala Ala Val Asp Leu Ser His Phe Leu Lys Glu Lys Gly Gly Leu
   1               5                   10                  15
   Glu Gly Leu Ile His Ser Gln Arg Arg Gln Asp Ile Leu Asp Leu Trp
               20                  25                  30
   Ile
<210> 101
   <211> 32
   <212> PRT
   <213> Artificial sequence
<220>
   <223> 83-114 HIV-1 Nef fragment
<400> 101
   Ala Ala Val Asp Leu Ser His Phe Leu Lys Glu Lys Gly Gly Leu Glu
   1               5                   10                  15
   Gly Leu Ile His Ser Gln Arg Arg Gln Asp Ile Leu Asp Leu Trp Ile
               20                  25                  30
<210> 102
   <211> 31
   <212> PRT
   <213> Artificial sequence
<220>
   <223> 84-114 HIV-1 Nef fragment
<400> 102
   Ala Val Asp Leu Ser His Phe Leu Lys Glu Lys Gly Gly Leu Glu Gly
   1               5                   10                  15
   Leu Ile His Ser Gln Arg Arg Gln Asp Ile Leu Asp Leu Trp Ile
               20                  25                  30
<210> 103
   <211> 30
   <212> PRT
   <213> Artificial sequence
<220>
   <223> 85-114 HIV-1 Nef fragment
<400> 103
   Val Asp Leu Ser His Phe Leu Lys Glu Lys Gly Gly Leu Glu Gly Leu
   1               5                   10                  15
   Ile His Ser Gln Arg Arg Gln Asp Ile Leu Asp Leu Trp Ile
               20                  25                  30
<210> 104
   <211> 29
   <212> PRT
   <213> Artificial sequence
<220>
   <223> 86-114 HIV-1 Nef fragment
<400> 104
   Asp Leu Ser His Phe Leu Lys Glu Lys Gly Gly Leu Glu Gly Leu Ile
   1               5                   10                  15
   His Ser Gln Arg Arg Gln Asp Ile Leu Asp Leu Trp Ile
               20                  25
<210> 105
   <211> 28
   <212> PRT
   <213> Artificial sequence
<220>
   <223> 87-114 HIV-1 Nef fragment
<400> 105
   Leu Ser His Phe Leu Lys Glu Lys Gly Gly Leu Glu Gly Leu Ile His
   1               5                   10                  15
   Ser Gln Arg Arg Gln Asp Ile Leu Asp Leu Trp Ile
               20                  25
<210> 106
   <211> 27
   <212> PRT
   <213> Artificial sequence
<220>
   <223> 88-114 HIV-1 Nef fragment
<400> 106
   Ser His Phe Leu Lys Glu Lys Gly Gly Leu Glu Gly Leu Ile His Ser
   1               5                   10                  15
   Gln Arg Arg Gln Asp Ile Leu Asp Leu Trp Ile
               20                  25
<210> 107
   <211> 26
   <212> PRT
   <213Artificial sequence
<220>
   <223> 89-114 HIV-1 Nef fragment
<400> 107
   His Phe Leu Lys Glu Lys Gly Gly Leu Glu Gly Leu Ile His Ser Gln
   1               5                   10                  15
   Arg Arg Gln Asp Ile Leu Asp Leu Trp Ile
               20                  25
<210> 108
   <211> 25
   <212> PRT
   <213> Artificial sequence
<220>
   <223> 90-114 HIV-1 Nef fragment
<400> 108
   Phe Leu Lys Glu Lys Gly Gly Leu Glu Gly Leu Ile His Ser Gln Arg
   1               5                   10                  15
   Arg Gln Asp Ile Leu Asp Leu Trp Ile
               20                  25
<210> 109
   <211> 34
   <212> PRT
   <213> Artificial sequence
<220>
   <223> 80-113 HIV-1 Nef fragment
<400> 109
   Thr Tyr Lys Ala Ala Val Asp Leu Ser His Phe Leu Lys Glu Lys Gly
   1               5                   10                  15
   Gly Leu Glu Gly Leu Ile His Ser Gln Arg Arg Gln Asp Ile Leu Asp
               20                  25                  30
   Leu Trp
<210> 110
   <211> 33
   <212> PRT
   <213> Artificial sequence
<220>
   <223> 81-113 HIV-1 Nef fragment
<400> 110
   Tyr Lys Ala Ala Val Asp Leu Ser His Phe Leu Lys Glu Lys Gly Gly
   1               5                   10                  15
   Leu Glu Gly Leu Ile His Ser Gln Arg Arg Gln Asp Ile Leu Asp Leu
               20                  25                  30
   Trp
<210> 111
   <211> 32
   <212> PRT
   <213> Artificial sequence
<220>
   <223> 82-113 HIV-1 Nef fragment
<400> 111
   Lys Ala Ala Val Asp Leu Ser His Phe Leu Lys Glu Lys Gly Gly Leu
   1               5                   10                  15
   Glu Gly Leu Ile His Ser Gln Arg Arg Gln Asp Ile Leu Asp Leu Trp
               20                  25                  30
<210> 112
   <211> 31
   <212> PRT
   <213> Artificial sequence
<220>
   <223> 83-113 HIV-1 Nef fragment
<400> 112
   Ala Ala Val Asp Leu Ser His Phe Leu Lys Glu Lys Gly Gly Leu Glu
   1               5                   10                  15
   Gly Leu Ile His Ser Gln Arg Arg Gln Asp Ile Leu Asp Leu Trp
               20                  25                  30
<210> 113
   <211> 30
   <212> PRT
   <213> Artificial sequence
<220>
   <223> 84-113 HIV-1 Nef fragment
<400> 113
   Ala Val Asp Leu Ser His Phe Leu Lys Glu Lys Gly Gly Leu Glu Gly
   1               5                   10                  15
   Leu Ile His Ser Gln Arg Arg Gln Asp Ile Leu Asp Leu Trp
               20                  25                  30
<210> 114
   <211> 29
   <212> PRT
   <213> Artificial sequence
<220>
   <223> 85-113 HIV-1 Nef fragment
<400> 114
   Val Asp Leu Ser His Phe Leu Lys Glu Lys Gly Gly Leu Glu Gly Leu
   1               5                   10                  15
   Ile His Ser Gln Arg Arg Gln Asp Ile Leu Asp Leu Trp
               20                  25
<210> 115
   <211> 28
   <212> PRT
   <213> Artificial sequence
<220>
   <223> 86-113 HIV-1 Nef fragment
<400> 115
   Asp Leu Ser His Phe Leu Lys Glu Lys Gly Gly Leu Glu Gly Leu Ile
   1               5                   10                  15
   His Ser Gln Arg Arg Gln Asp Ile Leu Asp Leu Trp
               20                  25
<210> 116
   <211> 27
   <212> PRT
   <213> Artificial sequence
<220>
   <223> 87-113 HIV-1 Nef fragment
<400> 116
   Leu Ser His Phe Leu Lys Glu Lys Gly Gly Leu Glu Gly Leu Ile His
   1               5                   10                  15
   Ser Gln Arg Arg Gln Asp Ile Leu Asp Leu Trp
               20                  25
<210> 117
   <211> 26
   <212> PRT
   <213> Artificial sequence
<220>
   <223> 88-113 HIV-1 Nef fragment
<400> 117
   Ser His Phe Leu Lys Glu Lys Gly Gly Leu Glu Gly Leu Ile His Ser
   1               5                   10                  15
   Gln Arg Arg Gln Asp Ile Leu Asp Leu Trp
               20                  25
<210> 118
   <211> 25
   <212> PRT
   <213> Artificial sequence
<220>
   <223> 89-113 HIV-1 Nef fragment
<400> 118
   His Phe Leu Lys Glu Lys Gly Gly Leu Glu Gly Leu Ile His Ser Gln
   1               5                   10                  15
   Arg Arg Gln Asp Ile Leu Asp Leu Trp
               20                  25
<210> 119
   <211> 24
   <212> PRT
   <213> Artificial sequence
<220>
   <223> 90-113 HIV-1 Nef fragment
<400> 119
   Phe Leu Lys Glu Lys Gly Gly Leu Glu Gly Leu Ile His Ser Gln Arg
   1               5                   10                  15
   Arg Gln Asp Ile Leu Asp Leu Trp
               20
<210> 120
   <211> 33
   <212> PRT
   <213> Artificial sequence
<220>
   <223> 80-112 HIV-1 Nef fragment
<400> 120
   Thr Tyr Lys Ala Ala Val Asp Leu Ser His Phe Leu Lys Glu Lys Gly
   1               5                   10                  15
   Gly Leu Glu Gly Leu Ile His Ser Gln Arg Arg Gln Asp Ile Leu Asp
               20                  25                  30
   Leu
<210> 121
   <211> 32
   <212> PRT
   <213> Artificial sequence
<220>
   <223> 81-112 HIV-1 Nef fragment
<400> 121
   Tyr Lys Ala Ala Val Asp Leu Ser His Phe Leu Lys Glu Lys Gly Gly
   1 5 10 15
   Leu Glu Gly Leu Ile His Ser Gln Arg Arg Gln Asp Ile Leu Asp Leu
   20 25 30
<210> 122
   <211> 31
   <212> PRT
   <213> Artificial sequence
<220>
   <223> 82-112 HIV-1 Nef fragment
<400> 122
   Lys Ala Ala Val Asp Leu Ser His Phe Leu Lys Glu Lys Gly Gly Leu
   1 5 10 15
   Glu Gly Leu Ile His Ser Gln Arg Arg Gln Asp Ile Leu Asp Leu
   20 25 30
<210> 123
   <211> 30
   <212> PRT
   <213> Artificial sequence
<220>
   <223> 83-112 HIV-1 Nef fragment
<400> 123
   Ala Ala Val Asp Leu Ser His Phe Leu Lys Glu Lys Gly Gly Leu Glu
   1 5 10 15
   Gly Leu Ile His Ser Gln Arg Arg Gln Asp Ile Leu Asp Leu
   20 25 30
<210> 124
   <211> 29
   <212> PRT
   <213> Artificial sequence
<220>
   <223> 84-112 HIV-1 Nef fragment
<400> 124
   Ala Val Asp Leu Ser His Phe Leu Lys Glu Lys Gly Gly Leu Glu Gly
   1 5 10 15
   Leu Ile His Ser Gln Arg Arg Gln Asp Ile Leu Asp Leu
   20 25
<210> 125
   <211> 28
   <212> PRT
   <213> Artificial sequence
<220>
   <223> 85-112 HIV-1 Nef fragment
<400> 125
   Val Asp Leu Ser His Phe Leu Lys Glu Lys Gly Gly Leu Glu Gly Leu
   1 5 10 15
   Ile His Ser Gln Arg Arg Gln Asp Ile Leu Asp Leu
   20 25
<210> 126
   <211> 27
   <212> PRT
   <213> Artificial sequence
<220>
   <223> 86-112 HIV-1 Nef fragment
<400> 126
   Asp Leu Ser His Phe Leu Lys Glu Lys Gly Gly Leu Glu Gly Leu Ile
   1 5 10 15
   His Ser Gln Arg Arg Gln Asp Ile Leu Asp Leu
   20 25
<210> 127
   <211> 26
   <212> PRT
   <213> Artificial sequence
<220>
   <223> 87-112 HIV-1 Nef fragment
<400> 127
   Leu Ser His Phe Leu Lys Glu Lys Gly Gly Leu Glu Gly Leu Ile His
   1 5 10 15
   Ser Gln Arg Arg Gln Asp Ile Leu Asp Leu
   20 25
<210> 128
   <211> 25
   <212> PRT
   <213> Artificial sequence
<220>
   <223> 88-112 HIV-1 Nef fragment
<400> 128
   Ser His Phe Leu Lys Glu Lys Gly Gly Leu Glu Gly Leu Ile His Ser
   1               5                   10                  15
   Gln Arg Arg Gln Asp Ile Leu Asp Leu
               20                  25
<210> 129
   <211> 24
   <212> PRT
   <213> Artificial sequence
<220>
   <223> 89-112 HIV-1 Nef fragment
<400> 129
   His Phe Leu Lys Glu Lys Gly Gly Leu Glu Gly Leu Ile His Ser Gln
   1               5                   10                  15
   Arg Arg Gln Asp Ile Leu Asp Leu
               20
<210> 130
   <211> 23
   <212> PRT
   <213> Artificial sequence
<220>
   <223> 90-112 HIV-1 Nef fragment
<400> 130
   Phe Leu Lys Glu Lys Gly Gly Leu Glu Gly Leu Ile His Ser Gln Arg
   1               5                   10                  15
   Arg Gln Asp Ile Leu Asp Leu
               20
<210> 131
   <211> 47
   <212> PRT
   <213> Artificial sequence
<220>
   <223> 104-150 HIV-2 Nef fragment
<400> 131
   Arg Val Pro Leu Arg Glu Met Thr Tyr Arg Leu Ala Arg Asp Met Ser
   1 5 10 15
   His Leu Ile Lys Glu Lys Gly Gly Leu Glu Gly Leu Tyr Tyr Ser Asp
   20 25 30
   Arg Arg Arg Arg Val Leu Asp Ile Tyr Leu Glu Lys Glu Glu Gly
   35 40 45
<210> 132
   <211> 46
   <212> PRT
   <213> Artificial sequence
<220>
   <223> 105-150 HIV-2 Nef fragment
<400> 132
   Val Pro Leu Arg Glu Met Thr Tyr Arg Leu Ala Arg Asp Met Ser His
   1 5 10 15
   Leu Ile Lys Glu Lys Gly Gly Leu Glu Gly Leu Tyr Tyr Ser Asp Arg
   20 25 30
   Arg Arg Arg Val Leu Asp Ile Tyr Leu Glu Lys Glu Glu Gly
   35 40 45
<210> 133
   <211> 45
   <212> PRT
   <213> Artificial sequence
<220>
   <223> 106-150 HIV-2 Nef fragment
<400> 133
   Pro Leu Arg Glu Met Thr Tyr Arg Leu Ala Arg Asp Met Ser His Leu
   1 5 10 15
   Ile Lys Glu Lys Gly Gly Leu Glu Gly Leu Tyr Tyr Ser Asp Arg Arg
   20 25 30
   Arg Arg Val Leu Asp Ile Tyr Leu Glu Lys Glu Glu Gly
   35 40 45
<210> 134
   <211> 44
   <212> PRT
   <213> Artificial sequence
<220>
   <223> 107-150 HIV-2 Nef fragment
<400> 134
   Leu Arg Glu Met Thr Tyr Arg Leu Ala Arg Asp Met Ser His Leu Ile
   1               5                   10                  15
   Lys Glu Lys Gly Gly Leu Glu Gly Leu Tyr Tyr Ser Asp Arg Arg Arg
               20                  25                  30
   Arg Val Leu Asp Ile Tyr Leu Glu Lys Glu Glu Gly
           35                  40
<210> 135
   <211> 43
   <212> PRT
   <213> Artificial sequence
<220>
   <223> 108-150 HIV-2 Nef fragment
<400> 135
   Arg Glu Met Thr Tyr Arg Leu Ala Arg Asp Met Ser His Leu Ile Lys
   1               5                   10                  15
   Glu Lys Gly Gly Leu Glu Gly Leu Tyr Tyr Ser Asp Arg Arg Arg Arg
               20                  25                  30
   Val Leu Asp Ile Tyr Leu Glu Lys Glu Glu Gly
           35                  40
<210> 136
   <211> 42
   <212> PRT
   <213> Artificial sequence
<220>
   <223> 109-150 HIV-2 Nef fragment
<400> 136
   Glu Met Thr Tyr Arg Leu Ala Arg Asp Met Ser His Leu Ile Lys Glu
   1               5                   10                  15
   Lys Gly Gly Leu Glu Gly Leu Tyr Tyr Ser Asp Arg Arg Arg Arg Val
               20                  25                  30
   Leu Asp Ile Tyr Leu Glu Lys Glu Glu Gly
           35                  40
<210> 137
   <211> 41
   <212> PRT
   <213> Artificial sequence
<220>
   <223> 110-150 HIV-2 Nef fragment
<400> 137
   Met Thr Tyr Arg Leu Ala Arg Asp Met Ser His Leu Ile Lys Glu Lys
   1               5                   10                  15
   Gly Gly Leu Glu Gly Leu Tyr Tyr Ser Asp Arg Arg Arg Arg Val Leu
               20                  25                  30
   Asp Ile Tyr Leu Glu Lys Glu Glu Gly
           35                  40
<210> 138
   <211> 40
   <212> PRT
   <213> Artificial sequence
<220>
   <223> 111-150 HIV-2 Nef fragment
<400> 138
   Thr Tyr Arg Leu Ala Arg Asp Met Ser His Leu Ile Lys Glu Lys Gly
   1               5                   10                  15
   Gly Leu Glu Gly Leu Tyr Tyr Ser Asp Arg Arg Arg Arg Val Leu Asp
                   20                  25                  30
   Ile Tyr Leu Glu Lys Glu Glu Gly
           35                  40
<210>139
   <211> 39
   <212> PRT
   <213> Artificial sequence
<220>
   <223> 112-150 HIV-2 Nef fragment
<400> 139
   Tyr Arg Leu Ala Arg Asp Met Ser His Leu Ile Lys Glu Lys Gly Gly
   1               5                   10                  15
   Leu Glu Gly Leu Tyr Tyr Ser Asp Arg Arg Arg Arg Val Leu Asp Ile
               20                  25                  30
   Tyr Leu Glu Lys Glu Glu Gly
           35
<210> 140
   <211> 38
   <212> PRT
   <213> Artificial sequence
<220>
   <223> 113-150 HIV-2 Nef fragment
<400> 140
   Arg Leu Ala Arg Asp Met Ser His Leu Ile Lys Glu Lys Gly Gly Leu
   1               5                   10                  15
   Glu Gly Leu Tyr Tyr Ser Asp Arg Arg Arg Arg Val Leu Asp Ile Tyr
               20                  25                  30
   Leu Glu Lys Glu Glu Gly
           35
<210> 141
   <211> 37
   <212> PRT
   <213> Artificial sequence
<220>
   <223> 114-150 HIV-2 Nef fragment
<400> 141
   Leu Ala Arg Asp Met Ser His Leu Ile Lys Glu Lys Gly Gly Leu Glu
   1               5                   10                  15
   Gly Leu Tyr Tyr Ser Asp Arg Arg Arg Arg Val Leu Asp Ile Tyr Leu
               20                  25                  30
   Glu Lys Glu Glu Gly
           35
<210> 142
   <211> 36
   <212> PRT
   <213> Artificial sequence
<220>
   <223> 115-150 HIV-2 Nef fragment
<400> 142
   Ala Arg Asp Met Ser His Leu Ile Lys Glu Lys Gly Gly Leu Glu Gly
   1               5                   10                  15
   Leu Tyr Tyr Ser Asp Arg Arg Arg Arg Val Leu Asp Ile Tyr Leu Glu
               20                  25                  30
   Lys Glu Glu Gly
           35
<210> 143
   <211> 35
   <212> PRT
   <213> Artificial sequence
<220>
   <223> 116-150 HIV-2 Nef fragment
<400> 143
   Arg Asp Met Ser His Leu Ile Lys Glu Lys Gly Gly Leu Glu Gly Leu
   1               5                   10                  15
   Tyr Tyr Ser Asp Arg Arg Arg Arg Val Leu Asp Ile Tyr Leu Glu Lys
               20                  25                  30
   Glu Glu Gly
           35
<210> 144
   <211> 34
   <212> PRT
   <213> Artificial sequence
<220>
   <223> 117-150 HIV-2 Nef fragment
<400> 144
   Asp Met Ser His Leu Ile Lys Glu Lys Gly Gly Leu Glu Gly Leu Tyr
   1               5                   10                  15
   Tyr Ser Asp Arg Arg Arg Arg Val Leu Asp Ile Tyr Leu Glu Lys Glu
               20                  25                  30
   Glu Gly
<210> 145
   <211> 33
   <212> PRT
   <213> Artificial sequence
<220>
   <223> 118-150 HIV-2 Nef frgament
<400> 145
   Met Ser His Leu Ile Lys Glu Lys Gly Gly Leu Glu Gly Leu Tyr Tyr
   1               5                   10                  15
   Ser Asp Arg Arg Arg Arg Val Leu Asp Ile Tyr Leu Glu Lys Glu Glu
               20                  25                  30
   Gly
<210> 146
   <211> 32
   <212> PRT
   <213> Artificial sequence
<220>
   <223> 119-150 HIV-2 Nef fragment
<400> 146
   Ser His Leu Ile Lys Glu Lys Gly Gly Leu Glu Gly Leu Tyr Tyr Ser
   1               5                   10                  15
   Asp Arg Arg Arg Arg Val Leu Asp Ile Tyr Leu Glu Lys Glu Glu Gly
               20                  25                  30
<210> 147
   <211> 31
   <212> PRT
   <213> Artificial sequence
<220>
   <223> 120-150 HIV-2 Nef fragment
<400> 147
   His Leu Ile Lys Glu Lys Gly Gly Leu Glu Gly Leu Tyr Tyr Ser Asp
   1               5                   10                  15
   Arg Arg Arg Arg Val Leu Asp Ile Tyr Leu Glu Lys Glu Glu Gly
               20                  25                  30
<210> 148
   <211> 30
   <212> PRT
   <213> Artificial sequence
<220>
   <223> 121-150 HIV-2 Nef fragment
<400> 148
   Leu Ile Lys Glu Lys Gly Gly Leu Glu Gly Leu Tyr Tyr Ser Asp Arg
   1               5                   10                  15
   Arg Arg Arg Val Leu Asp Ile Tyr Leu Glu Lys Glu Glu Gly
               20                  25                  30
<210> 149
   <211> 46
   <212> PRT
   <213> Artificial sequence
<220>
   <223> 104-149 HIV-2 Nef fragment
<400> 149
   Arg Val Pro Leu Arg Glu Met Thr Tyr Arg Leu Ala Arg Asp Met Ser
   1               5                   10                  15
   His Leu Ile Lys Glu Lys Gly Gly Leu Glu Gly Leu Tyr Tyr Ser Asp
               20                  25                  30
   Arg Arg Arg Arg Val Leu Asp Ile Tyr Leu Glu Lys Glu Glu
           35                  40                  45
<210> 150
   <211> 45
   <212> PRT
   <213> Artificial sequence
<220>
   <223> 105-149 HIV-2 Nef fragment
<400> 150
   Val Pro Leu Arg Glu Met Thr Tyr Arg Leu Ala Arg Asp Met Ser His
   1               5                   10                  15
   Leu Ile Lys Glu Lys Gly Gly Leu Glu Gly Leu Tyr Tyr Ser Asp Arg
               20                  25                  30
   Arg Arg Arg Val Leu Asp Ile Tyr Leu Glu Lys Glu Glu
           35                  40                  45
<210> 151
   <211> 44
   <212> PRT
   <213> Artificial sequence
<220>
   <223> 106-149 HIV-2 Nef fragment
<400> 151
   Pro Leu Arg Glu Met Thr Tyr Arg Leu Ala Arg Asp Met Ser His Leu
   1               5                   10                  15
   Ile Lys Glu Lys Gly Gly Leu Glu Gly Leu Tyr Tyr Ser Asp Arg Arg
               20                  25                  30
   Arg Arg Val Leu Asp Ile Tyr Leu Glu Lys Glu Glu
           35                  40
<210> 152
   <211> 43
   <212> PRT
   <213> Artificial sequence
<220>
   <223> 107-149 HIV-2 Nef fragment
<400> 152
   Leu Arg Glu Met Thr Tyr Arg Leu Ala Arg Asp Met Ser His Leu Ile
   1               5                   10                  15
   Lys Glu Lys Gly Gly Leu Glu Gly Leu Tyr Tyr Ser Asp Arg Arg Arg
               20                  25                  30
   Arg Val Leu Asp Ile Tyr Leu Glu Lys Glu Glu
           35                  40
<210> 153
   <211> 42
   <212> PRT
   <213> Artificial sequence
<220>
   <223> 108-149 HIV-2 Nef fragment
<400> 153
   Arg Glu Met Thr Tyr Arg Leu Ala Arg Asp Met Ser His Leu Ile Lys
   1 5 10 15
   Glu Lys Gly Gly Leu Glu Gly Leu Tyr Tyr Ser Asp Arg Arg Arg Arg
   20 25 30
   Val Leu Asp Ile Tyr Leu Glu Lys Glu Glu
   35 40
<210> 154
   <211> 41
   <212> PRT
   <213> Artificial sequence
<220>
   <223> 109-149 HIV-2 Nef fragment
<400> 154
   Glu Met Thr Tyr Arg Leu Ala Arg Asp Met Ser His Leu Ile Lys Glu
   1 5 10 15
   Lys Gly Gly Leu Glu Gly Leu Tyr Tyr Ser Asp Arg Arg Arg Arg Val
   20 25 30
   Leu Asp Ile Tyr Leu Glu Lys Glu Glu
   35 40
<210> 155
   <211> 40
   <212> PRT
   <213> Artificial sequence
<220>
   <223> 110-149 HIV-2 Nef fragment
<400> 155
   Met Thr Tyr Arg Leu Ala Arg Asp Met Ser His Leu Ile Lys Glu Lys
   1 5 10 15
   Gly Gly Leu Glu Gly Leu Tyr Tyr Ser Asp Arg Arg Arg Arg Val Leu
   20 25 30
   Asp Ile Tyr Leu Glu Lys Glu Glu
   35 40
<210> 156
   <211> 39
   <212> PRT
   <213> Artificial sequence
<220>
   <223> 111-149 HIV-2 Nef fragment
<400> 156
   Thr Tyr Arg Leu Ala Arg Asp Met Ser His Leu Ile Lys Glu Lys Gly
   1               5                   10                  15
   Gly Leu Glu Gly Leu Tyr Tyr Ser Asp Arg Arg Arg Arg Val Leu Asp
               20                  25                  30
   Ile Tyr Leu Glu Lys Glu Glu
           35
<210> 157
   <211> 38
   <212> PRT
   <213> Artificial sequence
<220>
   <223> 112-149 HIV-2 Nef fragment
<400> 157
   Tyr Arg Leu Ala Arg Asp Met Ser His Leu Ile Lys Glu Lys Gly Gly
   1               5                   10                  15
   Leu Glu Gly Leu Tyr Tyr Ser Asp Arg Arg Arg Arg Val Leu Asp Ile
               20                  25                  30
   Tyr Leu Glu Lys Glu Glu
           35
<210> 158
   <211> 37
   <212> PRT
   <213> Artificial sequence
<220>
   <223> 113-149 HIV-2 Nef fragment
<400> 158
   Arg Leu Ala Arg Asp Met Ser His Leu Ile Lys Glu Lys Gly Gly Leu
   1               5                   10                  15
   Glu Gly Leu Tyr Tyr Ser Asp Arg Arg Arg Arg Val Leu Asp Ile Tyr
               20                  25                  30
   Leu Glu Lys Glu Glu
           35
<210> 159
   <211> 36
   <212> PRT
   <213> Artificial sequence
<220>
   <223> 114-149 HIV-2 Nef fragment
<400> 159
   Leu Ala Arg Asp Met Ser His Leu Ile Lys Glu Lys Gly Gly Leu Glu
   1               5                   10                  15
   Gly Leu Tyr Tyr Ser Asp Arg Arg Arg Arg Val Leu Asp Ile Tyr Leu
               20                  25                  30
   Glu Lys Glu Glu
           35
<210> 160
   <211> 35
   <212> PRT
   <213> Artificial sequence
<220>
   <223> 115-149 HIV-2 Nef fragment
<400> 160
   Ala Arg Asp Met Ser His Leu Ile Lys Glu Lys Gly Gly Leu Glu Gly
   1               5                   10                  15
   Leu Tyr Tyr Ser Asp Arg Arg Arg Arg Val Leu Asp Ile Tyr Leu Glu
               20                  25                  30
   Lys Glu Glu
           35
<210> 161
   <211> 34
   <212> PRT
   <213> Artificial sequence
<220>
   <223> 116-149 HIV-2 Nef fragment
<400> 161
   Arg Asp Met Ser His Leu Ile Lys Glu Lys Gly Gly Leu Glu Gly Leu
   1               5                   10                  15
   Tyr Tyr Ser Asp Arg Arg Arg Arg Val Leu Asp Ile Tyr Leu Glu Lys
               20                  25                  30
   Glu Glu
<210> 162
   <211> 33
   <212> PRT
   <213> Artificial sequence
<220>
   <223> 117-149 HIV-2 Nef fragment
<400> 162
   Asp Met Ser His Leu Ile Lys Glu Lys Gly Gly Leu Glu Gly Leu Tyr
   1               5                   10                  15
   Tyr Ser Asp Arg Arg Arg Arg Val Leu Asp Ile Tyr Leu Glu Lys Glu
               20                  25                  30
   Glu
<210> 163
   <211> 32
   <212> PRT
   <213> Artificial sequence
<220>
   <223> 118-149 HIV-2 Nef fragment
<400> 163
   Met Ser His Leu Ile Lys Glu Lys Gly Gly Leu Glu Gly Leu Tyr Tyr
   1               5                   10                  15
   Ser Asp Arg Arg Arg Arg Val Leu Asp Ile Tyr Leu Glu Lys Glu Glu
               20                  25                  30
<210> 164
   <211> 31
   <212> PRT
   <213> Artificial sequence
<220>
   <223> 119-149 HIV-2 Nef fragment
<400> 164
   Ser His Leu Ile Lys Glu Lys Gly Gly Leu Glu Gly Leu Tyr Tyr Ser
   1 5 10 15
   Asp Arg Arg Arg Arg Val Leu Asp Ile Tyr Leu Glu Lys Glu Glu
   20 25 30
<210> 165
   <211> 30
   <212> PRT
   <213> Artificial sequence
<220>
   <223> 120-149 HIV-2 Nef fragment
<400> 165
   His Leu Ile Lys Glu Lys Gly Gly Leu Glu Gly Leu Tyr Tyr Ser Asp
   1 5 10 15
   Arg Arg Arg Arg Val Leu Asp Ile Tyr Leu Glu Lys Glu Glu
   20 25 30
<210> 166
   <211> 29
   <212> PRT
   <213> Artificial sequence
<220>
   <223> 121-149 HIV-2 Nef fragment
<400> 166
   Leu Ile Lys Glu Lys Gly Gly Leu Glu Gly Leu Tyr Tyr Ser Asp Arg
   1 5 10 15
   Arg Arg Arg Val Leu Asp Ile Tyr Leu Glu Lys Glu Glu
   20 25
<210> 167
   <211> 45
   <212> PRT
   <213> Artificial sequence
<220>
   <223> 104-148 HIV-2 Nef fragment
<400> 167
   Arg Val Pro Leu Arg Glu Met Thr Tyr Arg Leu Ala Arg Asp Met Ser
   1 5 10 15
   His Leu Ile Lys Glu Lys Gly Gly Leu Glu Gly Leu Tyr Tyr Ser Asp
   20 25 30
   Arg Arg Arg Arg Val Leu Asp Ile Tyr Leu Glu Lys Glu
   35 40 45
<210> 168
   <211> 44
   <212> PRT
   <213> Artificial sequence
<220>
   <223> 105-148 HIV-2 Nef fragment
<400> 168
   Val Pro Leu Arg Glu Met Thr Tyr Arg Leu Ala Arg Asp Met Ser His
   1 5 10 15
   Leu Ile Lys Glu Lys Gly Gly Leu Glu Gly Leu Tyr Tyr Ser Asp Arg
   20 25 30
   Arg Arg Arg Val Leu Asp Ile Tyr Leu Glu Lys Glu
   35 40
<210> 169
   <211> 43
   <212> PRT
   <213> Artificial sequence
<220>
   <223> 106-148 HIV-2 Nef fragment
<400> 169
   Pro Leu Arg Glu Met Thr Tyr Arg Leu Ala Arg Asp Met Ser His Leu
   1 5 10 15
   Ile Lys Glu Lys Gly Gly Leu Glu Gly Leu Tyr Tyr Ser Asp Arg Arg
   20 25 30
   Arg Arg Val Leu Asp Ile Tyr Leu Glu Lys Glu
   35 40
<210> 170
   <211> 42
   <212> PRT
   <213> Artificial sequence
<220>
   <223> 107-148 HIV-2 Nef fragment
<400> 170
   Leu Arg Glu Met Thr Tyr Arg Leu Ala Arg Asp Met Ser His Leu Ile
   1               5                   10                  15
   Lys Glu Lys Gly Gly Leu Glu Gly Leu Tyr Tyr Ser Asp Arg Arg Arg
               20                  25                  30
   Arg Val Leu Asp Ile Tyr Leu Glu Lys Glu
           35                  40
<210> 171
   <211> 41
   <212> PRT
   <213> Artificial sequence
<220>
   <223> 108-148 HIV-2 Nef fragment
<400> 171
   Arg Glu Met Thr Tyr Arg Leu Ala Arg Asp Met Ser His Leu Ile Lys
   1               5                   10                  15
   Glu Lys Gly Gly Leu Glu Gly Leu Tyr Tyr Ser Asp Arg Arg Arg Arg
               20                  25                  30
   Val Leu Asp Ile Tyr Leu Glu Lys Glu
           35                  40
<210> 172
   <211> 40
   <212> PRT
   <213> Artificial sequence
<220>
   <223> 109-148 HIV-2 Nef fragment
<400> 172
   Glu Met Thr Tyr Arg Leu Ala Arg Asp Met Ser His Leu Ile Lys Glu
   1 5 10 15
   Lys Gly Gly Leu Glu Gly Leu Tyr Tyr Ser Asp Arg Arg Arg Arg Val
   20 25 30
   Leu Asp Ile Tyr Leu Glu Lys Glu
   35 40
<210> 173
   <211> 39
   <212> PRT
   <213> Artificial sequence
<220>
   <223> 110-148 HIV-2 Nef fragment
<400> 173
   Met Thr Tyr Arg Leu Ala Arg Asp Met Ser His Leu Ile Lys Glu Lys
   1 5 10 15
   Gly Gly Leu Glu Gly Leu Tyr Tyr Ser Asp Arg Arg Arg Arg Val Leu
   20 25 30
   Asp Ile Tyr Leu Glu Lys Glu
   35
<210> 174
   <211> 38
   <212> PRT
   <213> Artificial sequence
<220>
   <223> 111-148 HIV-2 Nef fragment
<400> 174
   Thr Tyr Arg Leu Ala Arg Asp Met Ser His Leu Ile Lys Glu Lys Gly
   1 5 10 15
   Gly Leu Glu Gly Leu Tyr Tyr Ser Asp Arg Arg Arg Arg Val Leu Asp
   20 25 30
   Ile Tyr Leu Glu Lys Glu
   35
<210> 175
   <211> 37
   <212> PRT
   <213> Artificial sequence
<220>
   <223> 112-148 HIV-2 Nef fragment
<400> 175
   Tyr Arg Leu Ala Arg Asp Met Ser His Leu Ile Lys Glu Lys Gly Gly
   1               5                   10                  15
   Leu Glu Gly Leu Tyr Tyr Ser Asp Arg Arg Arg Arg Val Leu Asp Ile
               20                  25                  30
   Tyr Leu Glu Lys Glu
           35
<210> 176
   <211> 36
   <212> PRT
   <213> Artificial sequence
<220>
   <223> 113-148 HIV-2 Nef fragment
<400> 176
   Arg Leu Ala Arg Asp Met Ser His Leu Ile Lys Glu Lys Gly Gly Leu
   1               5                   10                  15
   Glu Gly Leu Tyr Tyr Ser Asp Arg Arg Arg Arg Val Leu Asp Ile Tyr
               20                  25                  30
   Leu Glu Lys Glu
           35
<210> 177
   <211> 35
   <212> PRT
   <213> Artificial sequence
<220>
   <223> 114-148 HIV-2 Nef fragment
<400> 177
   Leu Ala Arg Asp Met Ser His Leu Ile Lys Glu Lys Gly Gly Leu Glu
   1               5                   10                  15
   Gly Leu Tyr Tyr Ser Asp Arg Arg Arg Arg Val Leu Asp Ile Tyr Leu
               20                  25                  30
   Glu Lys Glu
           35
<210> 178
   <211> 34
   <212> PRT
   <213> Artificial sequence
<220>
   <223> 115-148 HIV-2 Nef fragment
<400> 178
   Ala Arg Asp Met Ser His Leu Ile Lys Glu Lys Gly Gly Leu Glu Gly
   1               5                   10                  15
   Leu Tyr Tyr Ser Asp Arg Arg Arg Arg Val Leu Asp Ile Tyr Leu Glu
               20                  25                  30
   Lys Glu
<210> 179
   <211> 33
   <212> PRT
   <213> Artificial sequence
<220>
   <223> 116-148 HIV-2 Nef fragment
<400> 179
   Arg Asp Met Ser His Leu Ile Lys Glu Lys Gly Gly Leu Glu Gly Leu
   1               5                   10                  15
   Tyr Tyr Ser Asp Arg Arg Arg Arg Val Leu Asp Ile Tyr Leu Glu Lys
               20                  25                  30
   Glu
<210> 180
   <211> 32
   <212> PRT
   <213> Artificial sequence
<220>
   <223> 117-148 HIV-2 Nef fragment
<400> 180
   Asp Met Ser His Leu Ile Lys Glu Lys Gly Gly Leu Glu Gly Leu Tyr
   1               5                   10                  15
   Tyr Ser Asp Arg Arg Arg Arg Val Leu Asp Ile Tyr Leu Glu Lys Glu
               20                  25                  30
<210> 181
   <211> 31
   <212> PRT
   <213> Artificial sequence
<220>
   <223> 118-148 HIV-2 Nef fragment
<400> 181
   Met Ser His Leu Ile Lys Glu Lys Gly Gly Leu Glu Gly Leu Tyr Tyr
    1               5                   10                  15
   Ser Asp Arg Arg Arg Arg Val Leu Asp Ile Tyr Leu Glu Lys Glu
               20                  25                  30
<210> 182
   <211> 30
   <212> PRT
   <213> Artificial sequence
<220>
   <223> 119-148 HIV-2 Nef fragment
<400> 182
   Ser His Leu Ile Lys Glu Lys Gly Gly Leu Glu Gly Leu Tyr Tyr Ser
   1               5                   10                  15
   Asp Arg Arg Arg Arg Val Leu Asp Ile Tyr Leu Glu Lys Glu
               20                  25                  30
<210> 183
   <211> 29
   <212> PRT
   <213> Artificial sequence
<220>
   <223> 120-148 HIV-2 Nef fragment
<400> 183
   His Leu Ile Lys Glu Lys Gly Gly Leu Glu Gly Leu Tyr Tyr Ser Asp
   1               5                   10                  15
   Arg Arg Arg Arg Val Leu Asp Ile Tyr Leu Glu Lys Glu
               20                  25
<210> 184
   <211> 28
   <212> PRT
   <213> Artificial sequence
<220>
   <223> 121-148 HIV-2 Nef fragment
<400> 184
   Leu Ile Lys Glu Lys Gly Gly Leu Glu Gly Leu Tyr Tyr Ser Asp Arg
   1               5                   10                  15
   Arg Arg Arg Val Leu Asp Ile Tyr Leu Glu Lys Glu
               20                  25
<210> 185
   <211> 44
   <212> PRT
   <213> Artificial sequence
<220>
   <223> 104-147 HIV-2 Nef fragment
<400> 185
   Arg Val Pro Leu Arg Glu Met Thr Tyr Arg Leu Ala Arg Asp Met Ser
   1               5                   10                  15
   His Leu Ile Lys Glu Lys Gly Gly Leu Glu Gly Leu Tyr Tyr Ser Asp
               20                  25                  30
   Arg Arg Arg Arg Val Leu Asp Ile Tyr Leu Glu Lys
           35                  40
<210> 186
   <211> 43
   <212> PRT
   <213> Artificial sequence
<220>
   <223> 105-147 HIV-2 Nef fragment
<400> 186
   Val Pro Leu Arg Glu Met Thr Tyr Arg Leu Ala Arg Asp Met Ser His
   1               5                   10                  15
   Leu Ile Lys Glu Lys Gly Gly Leu Glu Gly Leu Tyr Tyr Ser Asp Arg
               20                  25                  30
   Arg Arg Arg Val Leu Asp Ile Tyr Leu Glu Lys
           35                  40
<210> 187
   <211> 42
   <212> PRT
   <213> Artificial sequence
<220>
   <223> 106-147 HIV-2 Nef fragment
<400> 187
   Pro Leu Arg Glu Met Thr Tyr Arg Leu Ala Arg Asp Met Ser His Leu
   1 5 10 15
   Ile Lys Glu Lys Gly Gly Leu Glu Gly Leu Tyr Tyr Ser Asp Arg Arg
   20 25 30
   Arg Arg Val Leu Asp Ile Tyr Leu Glu Lys
   35 40
<210> 188
   <211> 41
   <212> PRT
   <213> Artificial sequence
<220>
   <223> 107-147 HIV-2 Nef fragment
<400> 188
   Leu Arg Glu Met Thr Tyr Arg Leu Ala Arg Asp Met Ser His Leu Ile
   1 5 10 15
   Lys Glu Lys Gly Gly Leu Glu Gly Leu Tyr Tyr Ser Asp Arg Arg Arg
   20 25 30
   Arg Val Leu Asp Ile Tyr Leu Glu Lys
   35 40
<210> 189
   <211> 40
   <212> PRT
   <213> Artificial sequence
<220>
   <223> 108-147 HIV-2 Nef fragment
<400> 189
   Arg Glu Met Thr Tyr Arg Leu Ala Arg Asp Met Ser His Leu Ile Lys
   1 5 10 15
   Glu Lys Gly Gly Leu Glu Gly Leu Tyr Tyr Ser Asp Arg Arg Arg Arg
   20 25 30
   Val Leu Asp Ile Tyr Leu Glu Lys
   35 40
<210> 190
   <211> 39
   <212> PRT
   <213> Artificial sequence
<220>
   <223> 109-147 HIV-2 Nef fragment
<400> 190
   Glu Met Thr Tyr Arg Leu Ala Arg Asp Met Ser His Leu Ile Lys Glu
   1               5                   10                  15
   Lys Gly Gly Leu Glu Gly Leu Tyr Tyr Ser Asp Arg Arg Arg Arg Val
               20                  25                  30
   Leu Asp Ile Tyr Leu Glu Lys
           35
<210> 191
   <211> 38
   <212> PRT
   <213> Artificial sequence
<220>
   <223> 110-147 HIV-2 Nef fragment
<400> 191
   Met Thr Tyr Arg Leu Ala Arg Asp Met Ser His Leu Ile Lys Glu Lys
   1               5                   10                  15
   Gly Gly Leu Glu Gly Leu Tyr Tyr Ser Asp Arg Arg Arg Arg Val Leu
               20                  25                  30
   Asp Ile Tyr Leu Glu Lys
           35
<210> 192
   <211> 37
   <212> PRT
   <213> Artificial sequence
<220>
   <223> 111-147 HIV-2 Nef fragment
<400> 192
   Thr Tyr Arg Leu Ala Arg Asp Met Ser His Leu Ile Lys Glu Lys Gly
   1 5 10 15
   Gly Leu Glu Gly Leu Tyr Tyr Ser Asp Arg Arg Arg Arg Val Leu Asp
   20 25 30
   Ile Tyr Leu Glu Lys
   35
<210> 193
   <211> 36
   <212> PRT
   <213> Artificial sequence
<220>
   <223> 112-147 HIV-2 Nef fragment
<400> 193
   Tyr Arg Leu Ala Arg Asp Met Ser His Leu Ile Lys Glu Lys Gly Gly
   1 5 10 15
   Leu Glu Gly Leu Tyr Tyr Ser Asp Arg Arg Arg Arg Val Leu Asp Ile
   20 25 30
   Tyr Leu Glu Lys
   35
<210> 194
   <211> 35
   <212> PRT
   <213> Artificial sequence
<220>
   <223> 113-147 HIV-2 Nef fragment
<400> 194
   Arg Leu Ala Arg Asp Met Ser His Leu Ile Lys Glu Lys Gly Gly Leu
   1 5 10 15
   Glu Gly Leu Tyr Tyr Ser Asp Arg Arg Arg Arg Val Leu Asp Ile Tyr
   20 25 30
   Leu Glu Lys
   35
<210> 195
   <211> 34
   <212> PRT
   <213> Artificial sequence
<220>
   <223> 114-147 HIV-2 Nef fragment
<400> 195
   Leu Ala Arg Asp Met Ser His Leu Ile Lys Glu Lys Gly Gly Leu Glu
   1               5                   10                  15
   Gly Leu Tyr Tyr Ser Asp Arg Arg Arg Arg Val Leu Asp Ile Tyr Leu
               20                  25                  30
   Glu Lys
<210> 196
   <211> 33
   <212> PRT
   <213> Artificial sequence
<220>
   <223> 115-147 HIV-2 Nef fragment
<400> 196
   Ala Arg Asp Met Ser His Leu Ile Lys Glu Lys Gly Gly Leu Glu Gly
   1               5                   10                  15
   Leu Tyr Tyr Ser Asp Arg Arg Arg Arg Val Leu Asp Ile Tyr Leu Glu
               20                  25                  30
   Lys
<210> 197
   <211> 32
   <212> PRT
   <213> Artificial sequence
<220>
   <223> 116-147 HIV-2 Nef fragment
<400> 197
   Arg Asp Met Ser His Leu Ile Lys Glu Lys Gly Gly Leu Glu Gly Leu
   1               5                   10                  15
   Tyr Tyr Ser Asp Arg Arg Arg Arg Val Leu Asp Ile Tyr Leu Glu Lys
               20                  25                  30
<210> 198
   <211> 31
   <212> PRT
   <213> Artificial sequence
<220>
   <223> 117-147 HIV-2 Nef fragment
<400> 198
   Asp Met Ser His Leu Ile Lys Glu Lys Gly Gly Leu Glu Gly Leu Tyr
   1 5 10 15
   Tyr Ser Asp Arg Arg Arg Arg Val Leu Asp Ile Tyr Leu Glu Lys
   20 25 30
<210> 199
   <211> 30
   <212> PRT
   <213> Artificial sequence
<220>
   <223> 118-147 HIV-2 Nef fragment
<400> 199
   Met Ser His Leu Ile Lys Glu Lys Gly Gly Leu Glu Gly Leu Tyr Tyr
   1 5 10 15
   Ser Asp Arg Arg Arg Arg Val Leu Asp Ile Tyr Leu Glu Lys
   20 25 30
<210> 200
   <211> 29
   <212> PRT
   <213> Artificial sequence
<220>
   <223> 119-147 HIV- Nef fragment
<400> 200
   Ser His Leu Ile Lys Glu Lys Gly Gly Leu Glu Gly Leu Tyr Tyr Ser
   1 5 10 15
   Asp Arg Arg Arg Arg Val Leu Asp Ile Tyr Leu Glu Lys
   20 25
<210> 201
   <211> 28
   <212> PRT
   <213> Artificial sequence
<220>
   <223> 120-147 HIV-2 Nef fragment
<400> 201
   His Leu Ile Lys Glu Lys Gly Gly Leu Glu Gly Leu Tyr Tyr Ser Asp
   1               5                   10                  15
   Arg Arg Arg Arg Val Leu Asp Ile Tyr Leu Glu Lys
               20                  25
<210> 202
   <211> 27
   <212> PRT
   <213> Artificial sequence
<220>
   <223> 121-147 HIV-2 Nef fragment
<400> 202
   Leu Ile Lys Glu Lys Gly Gly Leu Glu Gly Leu Tyr Tyr Ser Asp Arg
   1               5                   10                  15
   Arg Arg Arg Val Leu Asp Ile Tyr Leu Glu Lys
               20                  25
<210> 203
   <211> 43
   <212> PRT
   <213> Artificial sequence
<220>
   <223> 104-146 HIV-2 Nef fragment
<400> 203
   Arg Val Pro Leu Arg Glu Met Thr Tyr Arg Leu Ala Arg Asp Met Ser
   1               5                   10                  15
   His Leu Ile Lys Glu Lys Gly Gly Leu Glu Gly Leu Tyr Tyr Ser Asp
               20                  25                  30
   Arg Arg Arg Arg Val Leu Asp Ile Tyr Leu Glu
           35                  40
<210> 204
   <211> 42
   <212> PRT
   <213> Artificial sequence
<220>
   <223> 105-146 HIV-2 Nef fragment
<400> 204
   Val Pro Leu Arg Glu Met Thr Tyr Arg Leu Ala Arg Asp Met Ser His
   1 5 10 15
   Leu Ile Lys Glu Lys Gly Gly Leu Glu Gly Leu Tyr Tyr Ser Asp Arg
   20 25 30
   Arg Arg Arg Val Leu Asp Ile Tyr Leu Glu
   35 40
<210> 205
   <211> 41
   <212> PRT
   <213> Artificial sequence
<220>
   <223> 106-146 HIV-2 Nef fragment
<400> 205
   Pro Leu Arg Glu Met Thr Tyr Arg Leu Ala Arg Asp Met Ser His Leu
   1 5 10 15
   Ile Lys Glu Lys Gly Gly Leu Glu Gly Leu Tyr Tyr Ser Asp Arg Arg
   20 25 30
   Arg Arg Val Leu Asp Ile Tyr Leu Glu
   35 40
<210> 206
   <211> 40
   <212> PRT
   <213> Artificial sequence
<220>
   <223> 107-146 HIV-2 Nef fragment
<400> 206
   Leu Arg Glu Met Thr Tyr Arg Leu Ala Arg Asp Met Ser His Leu Ile
   1 5 10 15
   Lys Glu Lys Gly Gly Leu Glu Gly Leu Tyr Tyr Ser Asp Arg Arg Arg
   20 25 30
   Arg Val Leu Asp Ile Tyr Leu Glu
   35 40
<210> 207
   <211> 39
   <212> PRT
   <213> Artificial sequence
<220>
   <223> 108-146 HIV-2 Nef fragment
<400> 207
   Arg Glu Met Thr Tyr Arg Leu Ala Arg Asp Met Ser His Leu Ile Lys
   1               5                   10                  15
   Glu Lys Gly Gly Leu Glu Gly Leu Tyr Tyr Ser Asp Arg Arg Arg Arg
               20                  25                  30
   Val Leu Asp Ile Tyr Leu Glu
           35
<210> 208
   <211> 38
   <212> PRT
   <213> Artificial sequence
<220>
   <223> 109-146 HIV-2 Nef fragment
<400> 208
   Glu Met Thr Tyr Arg Leu Ala Arg Asp Met Ser His Leu Ile Lys Glu
   1               5                   10                  15
   Lys Gly Gly Leu Glu Gly Leu Tyr Tyr Ser Asp Arg Arg Arg Arg Val
               20                  25                  30
   Leu Asp Ile Tyr Leu Glu
           35
<210> 209
   <211> 37
   <212> PRT
   <213> Artificial sequence
<220>
   <223> 110-146 HIV-2 Nef fragment
<400> 209
   Met Thr Tyr Arg Leu Ala Arg Asp Met Ser His Leu Ile Lys Glu Lys
   1               5                   10                  15
   Gly Gly Leu Glu Gly Leu Tyr Tyr Ser Asp Arg Arg Arg Arg Val Leu
               20                  25                  30
   Asp Ile Tyr Leu Glu
           35
<210> 210
   <211> 36
   <212> PRT
   <213> Artificial sequence
<220>
   <223> 111-146 HIV-2 Nef fragment
<400> 210
   Thr Tyr Arg Leu Ala Arg Asp Met Ser His Leu Ile Lys Glu Lys Gly
   1               5                   10                  15
   Gly Leu Glu Gly Leu Tyr Tyr Ser Asp Arg Arg Arg Arg Val Leu Asp
               20                  25                  30
   Ile Tyr Leu Glu
           35
<210> 211
   <211> 35
   <212> PRT
   <213> Artificial sequence
<220>
   <223> 112-146 HIV-2 Nef fragment
<400> 211
   Tyr Arg Leu Ala Arg Asp Met Ser His Leu Ile Lys Glu Lys Gly Gly
   1               5                   10                  15
   Leu Glu Gly Leu Tyr Tyr Ser Asp Arg Arg Arg Arg Val Leu Asp Ile
               20                  25                  30
   Tyr Leu Glu
           35
<210> 212
   <211> 34
   <212> PRT
   <213> Artificial sequence
<220>
   <223> 113-146 HIV-2 Nef fragment
<400> 212
   Arg Leu Ala Arg Asp Met Ser His Leu Ile Lys Glu Lys Gly Gly Leu
   1 5 10 15
   Glu Gly Leu Tyr Tyr Ser Asp Arg Arg Arg Arg Val Leu Asp Ile Tyr
   20 25 30
   Leu Glu
<210> 213
   <211> 33
   <212> PRT
   <213> Artificial sequence
<220>
   <223> 114-146 HIV-2 Nef fragment
<400> 213
   Leu Ala Arg Asp Met Ser His Leu Ile Lys Glu Lys Gly Gly Leu Glu
   1 5 10 15
   Gly Leu Tyr Tyr Ser Asp Arg Arg Arg Arg Val Leu Asp Ile Tyr Leu
   20 25 30
   Glu
<210> 214
   <211> 32
   <212> PRT
   <213> Artificial sequence
<220>
   <223> 115-146 HIV-2 Nef fragment
<400> 214
   Ala Arg Asp Met Ser His Leu Ile Lys Glu Lys Gly Gly Leu Glu Gly
   1 5 10 15
   Leu Tyr Tyr Ser Asp Arg Arg Arg Arg Val Leu Asp Ile Tyr Leu Glu
   20 25 30
<210> 215
   <211> 31
   <212> PRT
   <213> Artificial sequence
<220>
   <223> 116-146 HIV-2 Nef fragment
<400> 215
   Arg Asp Met Ser His Leu Ile Lys Glu Lys Gly Gly Leu Glu Gly Leu
   1 5 10 15
   Tyr Tyr Ser Asp Arg Arg Arg Arg Val Leu Asp Ile Tyr Leu Glu
   20 25 30
<210> 216
   <211> 30
   <212> PRT
   <213> Artificial sequence
<220>
   <223> 117-146 HIV-2 Nef fragment
<400> 216
   Asp Met Ser His Leu Ile Lys Glu Lys Gly Gly Leu Glu Gly Leu Tyr
   1 5 10 15
   Tyr Ser Asp Arg Arg Arg Arg Val Leu Asp Ile Tyr Leu Glu
   20 25 30
<210> 217
   <211> 29
   <212> PRT
   <213> Artificial sequence
<220>
   <223> 118-146 HIV-2 Nef fragment
<400> 217
   Met Ser His Leu Ile Lys Glu Lys Gly Gly Leu Glu Gly Leu Tyr Tyr
   1 5 10 15
   Ser Asp Arg Arg Arg Arg Val Leu Asp Ile Tyr Leu Glu
   20 25
<210> 218
   <211> 28
   <212> PRT
   <213> Artificial sequence
<220>
   <223> 119-146 HIV-2 Nef fragment
<400> 218
   Ser His Leu Ile Lys Glu Lys Gly Gly Leu Glu Gly Leu Tyr Tyr Ser
   1 5 10 15
   Asp Arg Arg Arg Arg Val Leu Asp Ile Tyr Leu Glu
   20 25
<210> 219
   <211> 27
   <212> PRT
   <213> Artificial sequence
<220>
   <223> 120-146 HIV-2 Nef fragment
<400> 219
   His Leu Ile Lys Glu Lys Gly Gly Leu Glu Gly Leu Tyr Tyr Ser Asp
   1 5 10 15
   Arg Arg Arg Arg Val Leu Asp Ile Tyr Leu Glu
   20 25
<210> 220
   <211> 26
   <212> PRT
   <213> Artificial sequence
<220>
   <223> 121-146 HIV-2 Nef fragment
<400> 220
   Leu Ile Lys Glu Lys Gly Gly Leu Glu Gly Leu Tyr Tyr Ser Asp Arg
   1 5 10 15
   Arg Arg Arg Val Leu Asp Ile Tyr Leu Glu
   20 25
<210> 221
   <211> 42
   <212> PRT
   <213> Artificial sequence
<220>
   <223> 104-145 HIV-2 Nef fragment
<400> 221
   Arg Val Pro Leu Arg Glu Met Thr Tyr Arg Leu Ala Arg Asp Met Ser
   1 5 10 15
   His Leu Ile Lys Glu Lys Gly Gly Leu Glu Gly Leu Tyr Tyr Ser Asp
   20 25 30
   Arg Arg Arg Arg Val Leu Asp Ile Tyr Leu
   35 40
<210> 222
   <211> 41
   <212> PRT
   <213> Artificial sequence
<220>
   <223> 105-145 HIV-2 Nef fragment
<400> 222
   Val Pro Leu Arg Glu Met Thr Tyr Arg Leu Ala Arg Asp Met Ser His
   1               5                   10                  15
   Leu Ile Lys Glu Lys Gly Gly Leu Glu Gly Leu Tyr Tyr Ser Asp Arg
               20                  25                  30
   Arg Arg Arg Val Leu Asp Ile Tyr Leu
           35                  40
<210> 223
   <211> 40
   <212> PRT
   <213> Artificial sequence
<220>
   <223> 106-145 HIV-2 Nef fragment
<400> 223
   Pro Leu Arg Glu Met Thr Tyr Arg Leu Ala Arg Asp Met Ser His Leu
   1               5                   10                  15
   Ile Lys Glu Lys Gly Gly Leu Glu Gly Leu Tyr Tyr Ser Asp Arg Arg
               20                  25                  30
   Arg Arg Val Leu Asp Ile Tyr Leu
           35                  40
<210> 224
   <211> 39
   <212> PRT
   <213> Artificial sequence
<220>
   <223> 107-145 HIV-2 Nef fragment
<400> 224
   Leu Arg Glu Met Thr Tyr Arg Leu Ala Arg Asp Met Ser His Leu Ile
   1 5 10 15
   Lys Glu Lys Gly Gly Leu Glu Gly Leu Tyr Tyr Ser Asp Arg Arg Arg
   20 25 30
   Arg Val Leu Asp Ile Tyr Leu
   35
<210> 225
   <211> 38
   <212> PRT
   <213> Artificial sequence
<220>
   <223> 108-145 HIV-2 nef fragment
<400> 225
   Arg Glu Met Thr Tyr Arg Leu Ala Arg Asp Met Ser His Leu Ile Lys
   1 5 10 15
   Glu Lys Gly Gly Leu Glu Gly Leu Tyr Tyr Ser Asp Arg Arg Arg Arg
   20 25 30
   Val Leu Asp Ile Tyr Leu
   35
<210> 226
   <211> 37
   <212> PRT
   <213> Artificial sequence
<220>
   <223> 109-145 HIV-2 Nef fragment
<400> 226
   Glu Met Thr Tyr Arg Leu Ala Arg Asp Met Ser His Leu Ile Lys Glu
   1 5 10 15
   Lys Gly Gly Leu Glu Gly Leu Tyr Tyr Ser Asp Arg Arg Arg Arg Val
   20 25 30
   Leu Asp Ile Tyr Leu
   35
<210> 227
   <211> 36
   <212> PRT
   <213> Artificial sequence
<220>
   <223> 110-145 HIV-2 Nef fragment
<400> 227
   Met Thr Tyr Arg Leu Ala Arg Asp Met Ser His Leu Ile Lys Glu Lys
   1               5                   10                  15
   Gly Gly Leu Glu Gly Leu Tyr Tyr Ser Asp Arg Arg Arg Arg Val Leu
               20                  25                  30
   Asp Ile Tyr Leu
           35
<210> 228
   <211> 35
   <212> PRT
   <213> Artificial sequence
<220>
   <223> 111-145 HIV-2 Nef fragment
<400> 228
   Thr Tyr Arg Leu Ala Arg Asp Met Ser His Leu Ile Lys Glu Lys Gly
   1               5                   10                  15
   Gly Leu Glu Gly Leu Tyr Tyr Ser Asp Arg Arg Arg Arg Val Leu Asp
               20                  25                  30
   Ile Tyr Leu
           35
<210> 229
   <211> 34
   <212> PRT
   <213> Artificial sequence
<220>
   <223> 112-145 HIV-2 Nef fragment
<400> 229
   Tyr Arg Leu Ala Arg Asp Met Ser His Leu Ile Lys Glu Lys Gly Gly
   1               5                   10                  15
   Leu Glu Gly Leu Tyr Tyr Ser Asp Arg Arg Arg Arg Val Leu Asp Ile
               20                  25                  30
   Tyr Leu
<210> 230
   <211> 33
   <212> PRT
   <213> Artificial sequence
<220>
   <223> 113-145 HIV-2 Nef fragment
<400> 230
   Arg Leu Ala Arg Asp Met Ser His Leu Ile Lys Glu Lys Gly Gly Leu
   1 5 10 15
   Glu Gly Leu Tyr Tyr Ser Asp Arg Arg Arg Arg Val Leu Asp Ile Tyr
   20 25 30
   Leu
<210> 231
   <211> 32
   <212> PRT
   <213> Artificial sequence
<220>
   <223> 114-145 HIV-2 Nef fragment
<400> 231
   Leu Ala Arg Asp Met Ser His Leu Ile Lys Glu Lys Gly Gly Leu Glu
   1 5 10 15
   Gly Leu Tyr Tyr Ser Asp Arg Arg Arg Arg Val Leu Asp Ile Tyr Leu
   20 25 30
<211> 31
   <212> PRT
   <213> Artificial sequence
<220>
   <223> 115-145 HIV-2 Nef fragment
<400> 232
<210> 233
   <211> 30
   <212> PRT
   <213> Artificial sequence
<220>
   <223> 116-145 HIV-2 Nef fragment
<400> 233
   Arg Asp Met Ser His Leu Ile Lys Glu Lys Gly Gly Leu Glu Gly Leu
   1               5                   10                  15
   Tyr Tyr Ser Asp Arg Arg Arg Arg Val Leu Asp Ile Tyr Leu
               20                  25                  30
<210> 234
   <211> 29
   <212> PRT
   <213> Artificial sequence
<220>
   <223> 117-145 HIV-2 Nef fragment
<400> 234
   Asp Met Ser His Leu Ile Lys Glu Lys Gly Gly Leu Glu Gly Leu Tyr
   1               5                   10                  15
   Tyr Ser Asp Arg Arg Arg Arg Val Leu Asp Ile Tyr Leu
               20                  25
<210> 235
   <211> 28
   <212> PRT
   <213> Artificial sequence
<220>
   <223> 118-145 HIV-2 Nef fragment
<400> 235
   Met Ser His Leu Ile Lys Glu Lys Gly Gly Leu Glu Gly Leu Tyr Tyr
   1               5                   10                  15
   Ser Asp Arg Arg Arg Arg Val Leu Asp Ile Tyr Leu
               20                  25
<210> 236
   <211> 27
   <212> PRT
   <213> Artificial sequence
<220>
   <223> 119-145 HIV-2 Nef fragment
<400> 236
   Ser His Leu Ile Lys Glu Lys Gly Gly Leu Glu Gly Leu Tyr Tyr Ser
   1               5                   10                  15
   Asp Arg Arg Arg Arg Val Leu Asp Ile Tyr Leu
               20                  25
<210> 237
   <211> 26
   <212> PRT
   <213> Artificial sequence
<220>
   <223> 120-145 HIV-2 Nef fragment
<400> 237
   His Leu Ile Lys Glu Lys Gly Gly Leu Glu Gly Leu Tyr Tyr Ser Asp
   1               5                   10                  15
   Arg Arg Arg Arg Val Leu Asp Ile Tyr Leu
               20                  25
<210> 238
   <211> 25
   <212> PRT
   <213> Artificial sequence
<220>
   <223> 121-145 HIV-2 Nef fragment
<400> 238
   Leu Ile Lys Glu Lys Gly Gly Leu Glu Gly Leu Tyr Tyr Ser Asp Arg
   1               5                   10                  15
   Arg Arg Arg Val Leu Asp Ile Tyr Leu
               20                  25
<210> 239
   <211> 41
   <212> PRT
   <213> Artificial sequence
<220>
   <223> 104-144 HIV-2 Nef fragment
<400> 239
   Arg Val Pro Leu Arg Glu Met Thr Tyr Arg Leu Ala Arg Asp Met Ser
   1 5 10 15
   His Leu Ile Lys Glu Lys Gly Gly Leu Glu Gly Leu Tyr Tyr Ser Asp
   20 25 30
   Arg Arg Arg Arg Val Leu Asp Ile Tyr
   35 40
<210> 240
   <211> 40
   <212> PRT
   <213> Artificial sequence
<220>
   <223> 105-144 HIV-2 Nef fragment
<400> 240
   Val Pro Leu Arg Glu Met Thr Tyr Arg Leu Ala Arg Asp Met Ser His
   1 5 10 15
   Leu Ile Lys Glu Lys Gly Gly Leu Glu Gly Leu Tyr Tyr Ser Asp Arg
   20 25 30
   Arg Arg Arg Val Leu Asp Ile Tyr
   35 40
<210> 241
   <211> 39
   <212> PRT
   <213> Artificial sequence
<220>
   <223> 106-144 HIV-2 Nef fragment
<400> 241
   Pro Leu Arg Glu Met Thr Tyr Arg Leu Ala Arg Asp Met Ser His Leu
   1 5 10 15
   Ile Lys Glu Lys Gly Gly Leu Glu Gly Leu Tyr Tyr Ser Asp Arg Arg
   20 25 30
   Arg Arg Val Leu Asp Ile Tyr
   35
<210> 242
   <211> 38
   <212> PRT
   <213> Artificial sequence
<220>
   <223> 107-144 HIV-2 Nef fragment
<400> 242
   Leu Arg Glu Met Thr Tyr Arg Leu Ala Arg Asp Met Ser His Leu Ile
   1               5                   10                  15
   Lys Glu Lys Gly Gly Leu Glu Gly Leu Tyr Tyr Ser Asp Arg Arg Arg
               20                  25                  30
   Arg Val Leu Asp Ile Tyr
           35
<210> 243
   <211> 37
   <212> PRT
   <213> Artificial sequence
<220>
   <223> 108-144 HIV-2 Nef fragment
<400> 243
   Arg Glu Met Thr Tyr Arg Leu Ala Arg Asp Met Ser His Leu Ile Lys
   1               5                   10                  15
   Glu Lys Gly Gly Leu Glu Gly Leu Tyr Tyr Ser Asp Arg Arg Arg Arg
               20                  25                  30
   Val Leu Asp Ile Tyr
           35
<210> 244
   <211> 36
   <212> PRT
   <213> Artificial sequence
<220>
   <223> 109-144 HIV-2 Nef fragment
<400> 244
   Glu Met Thr Tyr Arg Leu Ala Arg Asp Met Ser His Leu Ile Lys Glu
   1               5                   10                  15
   Lys Gly Gly Leu Glu Gly Leu Tyr Tyr Ser Asp Arg Arg Arg Arg Val
               20                  25                  30
   Leu Asp Ile Tyr
           35
<210> 245
   <211> 35
   <212> PRT
   <213> Artificial sequence
<220>
   <223> 110-144 HIV-2 Nef fragment
<400> 245
   Met Thr Tyr Arg Leu Ala Arg Asp Met Ser His Leu Ile Lys Glu Lys
   1               5                   10                  15
   Gly Gly Leu Glu Gly Leu Tyr Tyr Ser Asp Arg Arg Arg Arg Val Leu
               20                  25                  30
   Asp Ile Tyr
           35
<210> 246
   <211> 34
   <212> PRT
   <213> Artificial sequence
<220>
   <223> 111-144 HIV-2 Nef fragment
<400> 246
   Thr Tyr Arg Leu Ala Arg Asp Met Ser His Leu Ile Lys Glu Lys Gly
   1               5                   10                  15
   Gly Leu Glu Gly Leu Tyr Tyr Ser Asp Arg Arg Arg Arg Val Leu Asp
               20                  25                  30
   Ile Tyr
<210> 247
   <211> 33
   <212> PRT
   <213> Artificial sequence
<220>
   <223> 112-144 HIV-2 Nef fragment
<400> 247
   Tyr Arg Leu Ala Arg Asp Met Ser His Leu Ile Lys Glu Lys Gly Gly
   1               5                   10                  15
   Leu Glu Gly Leu Tyr Tyr Ser Asp Arg Arg Arg Arg Val Leu Asp Ile
               20                  25                  30
   Tyr
<210> 248
   <211> 32
   <212> PRT
   <213> Artificial sequence
<220>
   <223> 113-144 HIV-2 Nef fragment
<400> 248
   Arg Leu Ala Arg Asp Met Ser His Leu Ile Lys Glu Lys Gly Gly Leu
   1               5                   10                  15
   Glu Gly Leu Tyr Tyr Ser Asp Arg Arg Arg Arg Val Leu Asp Ile Tyr
               20                  25                  30
<210> 249
   <211> 31
   <212> PRT
   <213> Artificial sequence
<220>
   <223> 114-144 HIV-2 Nef fragment
<400> 249
   Leu Ala Arg Asp Met Ser His Leu Ile Lys Glu Lys Gly Gly Leu Glu
   1               5                   10                  15
   Gly Leu Tyr Tyr Ser Asp Arg Arg Arg Arg Val Leu Asp Ile Tyr
               20                  25                  30
<210> 250
   <211> 30
   <212> PRT
   <213> Artificial sequence
<220>
   <223> 115-144 HIV-2 Nef fragment
<400> 250
   Ala Arg Asp Met Ser His Leu Ile Lys Glu Lys Gly Gly Leu Glu Gly
   1 5 10 15
   Leu Tyr Tyr Ser Asp Arg Arg Arg Arg Val Leu Asp Ile Tyr
   20 25 30
<210> 251
   <211> 29
   <212> PRT
   <213> Artificial sequence
<220>
   <223> 116-144 HIV-2 Nef fragment
<400> 251
   Arg Asp Met Ser His Leu Ile Lys Glu Lys Gly Gly Leu Glu Gly Leu
   1 5 10 15
   Tyr Tyr Ser Asp Arg Arg Arg Arg Val Leu Asp Ile Tyr
   20 25
<210> 252
   <211> 28
   <212> PRT
   <213> Artificial sequence
<220>
   <223> 117-144 HIV-2 Nef fragment
<400> 252
   Asp Met Ser His Leu Ile Lys Glu Lys Gly Gly Leu Glu Gly Leu Tyr
   1 5 10 15
   Tyr Ser Asp Arg Arg Arg Arg Val Leu Asp Ile Tyr
   20 25
<210> 253
   <211> 27
   <212> PRT
   <213> Artificial sequence
<220>
   <223> 118-144 HIV-2 Nef fragment
<400> 253
   Met Ser His Leu Ile Lys Glu Lys Gly Gly Leu Glu Gly Leu Tyr Tyr
   1 5 10 15
   Ser Asp Arg Arg Arg Arg Val Leu Asp Ile Tyr
   20 25
<210> 254
   <211> 26
   <212> PRT
   <213> Artificial sequence
<220>
   <223> 119-144 HIV-2 Nef fragment
<400> 254
   Ser His Leu Ile Lys Glu Lys Gly Gly Leu Glu Gly Leu Tyr Tyr Ser
   1               5                   10                  15
   Asp Arg Arg Arg Arg Val Leu Asp Ile Tyr
               20                  25
<210> 255
   <211> 25
   <212> PRT
   <213> Artificial sequence
<220>
   <223> 120-144 HIV-2 Nef fragment
<400> 255
   His Leu Ile Lys Glu Lys Gly Gly Leu Glu Gly Leu Tyr Tyr Ser Asp
   1               5                   10                  15
   Arg Arg Arg Arg Val Leu Asp Ile Tyr
               20                  25
<210> 256
   <211> 24
   <212> PRT
   <213> Artificial sequence
<220>
   <223> 121-144 HIV-2 Nef fragment
<400> 256
   Leu Ile Lys Glu Lys Gly Gly Leu Glu Gly Leu Tyr Tyr Ser Asp Arg
   1               5                   10                  15
   Arg Arg Arg Val Leu Asp Ile Tyr
               20
<210> 257
   <211> 40
   <212> PRT
   <213> Artificial sequence
<220>
   <223> 104-143 HIV-2 Nef fragment
<400> 257
   Arg Val Pro Leu Arg Glu Met Thr Tyr Arg Leu Ala Arg Asp Met Ser
   1               5                   10                  15
   His Leu Ile Lys Glu Lys Gly Gly Leu Glu Gly Leu Tyr Tyr Ser Asp
               20                  25                  30
   Arg Arg Arg Arg Val Leu Asp Ile
           35                  40
<210> 258
   <211> 39
   <212> PRT
   <213> Artificial sequence
<220>
   <223> 105-143 HIV-2 Nef fragment
<400> 258
   Val Pro Leu Arg Glu Met Thr Tyr Arg Leu Ala Arg Asp Met Ser His
   1               5                   10                  15
   Leu Ile Lys Glu Lys Gly Gly Leu Glu Gly Leu Tyr Tyr Ser Asp Arg
               20                  25                  30
   Arg Arg Arg Val Leu Asp Ile
           35
<210> 259
   <211> 38
   <212> PRT
   <213> Artificial sequence
<220>
   <223> 106-143 HIV-2 Nef fragment
<400> 259
   Pro Leu Arg Glu Met Thr Tyr Arg Leu Ala Arg Asp Met Ser His Leu
   1               5                   10                  15
   Ile Lys Glu Lys Gly Gly Leu Glu Gly Leu Tyr Tyr Ser Asp Arg Arg
               20                  25                  30
   Arg Arg Val Leu Asp Ile
           35
<210> 260
   <211> 37
   <212> PRT
   <213> Artificial sequence
<220>
   <223> 107-143 HIV-2 Nef fragment
<400> 260
   Leu Arg Glu Met Thr Tyr Arg Leu Ala Arg Asp Met Ser His Leu Ile
   1               5                   10                  15
   Lys Glu Lys Gly Gly Leu Glu Gly Leu Tyr Tyr Ser Asp Arg Arg Arg
               20                  25                  30
   Arg Val Leu Asp Ile
           35
<210> 261
   <211> 36
   <212> PRT
   <213> Artificial sequence
<220>
   <223> 108-143 HIV-2 Nef fragment
<400> 261
   Arg Glu Met Thr Tyr Arg Leu Ala Arg Asp Met Ser His Leu Ile Lys
   1               5                   10                  15
   Glu Lys Gly Gly Leu Glu Gly Leu Tyr Tyr Ser Asp Arg Arg Arg Arg
               20                  25                  30
   Val Leu Asp Ile
           35
<210> 262
   <211> 35
   <212> PRT
   <213> Artificial sequence
<220>
   <223> 109-143 HIV-2 Nef fragment
<400> 262
   Glu Met Thr Tyr Arg Leu Ala Arg Asp Met Ser His Leu Ile Lys Glu
   1               5                   10                  15
   Lys Gly Gly Leu Glu Gly Leu Tyr Tyr Ser Asp Arg Arg Arg Arg Val
               20                  25                  30
   Leu Asp Ile
           35
<210> 263
   <211> 34
   <212> PRT
   <213> Artificial sequence
<220>
   <223> 110-143 HIV-2 Nef fragment
<400> 263
   Met Thr Tyr Arg Leu Ala Arg Asp Met Ser His Leu Ile Lys Glu Lys
   1               5                   10                  15
   Gly Gly Leu Glu Gly Leu Tyr Tyr Ser Asp Arg Arg Arg Arg Val Leu
               20                  25                  30
   Asp Ile
<210> 264
   <211> 33
   <212> PRT
   <213> Artificial sequence
<220>
   <223> 111-143 HIV-2 Nef fragment
<400> 264
   Thr Tyr Arg Leu Ala Arg Asp Met Ser His Leu Ile Lys Glu Lys Gly
   1               5                   10                  15
   Gly Leu Glu Gly Leu Tyr Tyr Ser Asp Arg Arg Arg Arg Val Leu Asp
               20                  25                  30
   Ile
<210> 265
   <211> 32
   <212> PRT
   <213> Artificial sequence
<220>
   <223> 112-143 HIV-2 Nef fragment
<400> 265
   Tyr Arg Leu Ala Arg Asp Met Ser His Leu Ile Lys Glu Lys Gly Gly
   1               5                   10                  15
   Leu Glu Gly Leu Tyr Tyr Ser Asp Arg Arg Arg Arg Val Leu Asp Ile
               20                  25                  30
<210> 266
   <211> 31
   <212> PRT
   <213> Artificial sequence
<220>
   <223> 113-143 HIV-2 Nef fragment
<400> 266
   Arg Leu Ala Arg Asp Met Ser His Leu Ile Lys Glu Lys Gly Gly Leu
   1               5                   10                  15
   Glu Gly Leu Tyr Tyr Ser Asp Arg Arg Arg Arg Val Leu Asp Ile
               20                  25                  30
<210> 267
   <211> 30
   <212> PRT
   <213> Artificial sequence
<220>
   <223> 114-143 HIV-2 Nef fragment
<400> 267
   Leu Ala Arg Asp Met Ser His Leu Ile Lys Glu Lys Gly Gly Leu Glu
   1               5                   10                  15
   Gly Leu Tyr Tyr Ser Asp Arg Arg Arg Arg Val Leu Asp Ile
               20                  25                  30
<210> 268
   <211> 29
   <212> PRT
   <213> Artificial sequence
<220>
   <223> 115-143 HIV-2 Nef fragment
<400> 268
   Ala Arg Asp Met Ser His Leu Ile Lys Glu Lys Gly Gly Leu Glu Gly
   1 5 10 15
   Leu Tyr Tyr Ser Asp Arg Arg Arg Arg Val Leu Asp Ile
   20 25
<210> 269
   <211> 28
   <212> PRT
   <213> Artificial sequence
<220>
   <223> 116-143 HIV-2 Nef fragment
<400> 269
   Arg Asp Met Ser His Leu Ile Lys Glu Lys Gly Gly Leu Glu Gly Leu
   1 5 10 15
   Tyr Tyr Ser Asp Arg Arg Arg Arg Val Leu Asp Ile
   20 25
<210> 270
   <211> 27
   <212> PRT
   <213> Artificial sequence
<220>
   <223> 117-143 HIV-2 Nef fragment
<400> 270
   Asp Met Ser His Leu Ile Lys Glu Lys Gly Gly Leu Glu Gly Leu Tyr
   1 5 10 15
   Tyr Ser Asp Arg Arg Arg Arg Val Leu Asp Ile
   20 25
<210> 271
   <211> 26
   <212> PRT
   <213> Artificial sequence
<220>
   <223> 118-143 HIV-2 Nef fragment
<400> 271
   Met Ser His Leu Ile Lys Glu Lys Gly Gly Leu Glu Gly Leu Tyr Tyr
                    5                   10                  15
   Ser Asp Arg Arg Arg Arg Val Leu Asp Ile
               20                  25
<210> 272
   <211> 25
   <212> PRT
   <213> Artificial sequence
<220>
   <223> 119-143 HIV-2 Nef fragment
<400> 272
   Ser His Leu Ile Lys Glu Lys Gly Gly Leu Glu Gly Leu Tyr Tyr Ser
   1 5 10 15
   Asp Arg Arg Arg Arg Val Leu Asp Ile
   20 25
<210> 273
   <211> 24
   <212> PRT
   <213> Artificial sequence
<220>
   <223> 120-143 HIV-2 Nef fragment
<400> 273
   His Leu Ile Lys Glu Lys Gly Gly Leu Glu Gly Leu Tyr Tyr Ser Asp
   1 5 10 15
   Arg Arg Arg Arg Val Leu Asp Ile
   20
<210> 274
   <211> 23
   <212> PRT
   <213> Artificial sequence
<220>
   <223> 121-143 HIV-2 Nef fragment
<400> 274
   Leu Ile Lys Glu Lys Gly Gly Leu Glu Gly Leu Tyr Tyr Ser Asp Arg
   1 5 10 15
   Arg Arg Arg Val Leu Asp Ile
   20